(19) Europäisches Patentamt
European Patent Office
Office européen des brevets

(11) Veröffentlichungsnummer: **0 257 400**
**A2**

(12) # EUROPÄISCHE PATENTANMELDUNG

(21) Anmeldenummer: **87111382.5**

(51) Int. Cl.4: **A61K 47/00**, A61K 9/10

(22) Anmeldetag: **06.08.87**

Die Bezeichnung der Erfindung wurde geändert (Richtlinien für die Prüfung im EPA, A-III, 7.3).

(30) Priorität: **07.08.86 DE 3626700**

(43) Veröffentlichungstag der Anmeldung:
**02.03.88 Patentblatt 88/09**

(84) Benannte Vertragsstaaten:
**AT BE CH DE ES FR GB GR IT LI LU NL SE**

(71) Anmelder: **MEDICE Chem.-Pharm. Fabrik Pütter GmbH & Co. KG**
**Kuhloweg 37-39**
**D-5860 Iserlohn/Westfalen(DE)**

(72) Erfinder: **Paradies, Henrich, Prof.Dr.med.Dr.rer.nat.**
**Kuhloweg 38**
**D-5860 Iserlohn(DE)**

(74) Vertreter: **Reinhard, Skuhra, Weise**
**Leopoldstrasse 51**
**D-8000 München 40(DE)**

(54) **Kationische Tenside enthaltende Arzneimittel.**

(57) Es wird eine pharmazeutische Zubereitung offenbart, die aufgebaut ist aus einer Micelle, bestehend aus einem kationischen Tensid mit einem einwertigen Anion und einem hydrophoben pharmazeutischen Wirkstoff, dispergiert in einem Lösungsmittel, dessen pH-Wert kleiner ≤ 7 ist, wobei die kritische Micellbildungskonzentration (KMK) im Bereich von 1,0 . $10^{-7}$ bis 1,5 . $10^{-4}$ Mol/Liter liegt. Außerdem wird eine Anzahl neuer kationischer Tenside (Heterozyklen) offenbart. Die offenbarten Zubereitungen haben insbesondere den Vorteil, daß durch die Erhöhung der Hydrophobizität der Alkyl-bzw. Aryl-Kette bzw. Restes am $N^+$-Tensid die Membran-Permeabilität erhöht wird, so daß die pharmazeutischen Wirkstoffe quantitativ passiv in das Zytosol übertreten können.

EP 0 257 400 A2

## Pharmazeutische Zubereitungen

Die vorliegende Erfindung betrifft pharmazeutische Zubereitungen, bekannte kationische Tenside als Bestandteile der pharmazeutischen Zubereitung, neue chemische Verbindungen (kationische Tenside), die insbesondere als Bestandteil der pharmazeutischen Zubereitungen verwendet werden, Verfahren zur Herstellung der pharmazeutischen Zubereitung und Verfahren zur Herstellung der bekannten und neuen chemischen Verbindungen (kationischen Tensiden).

### Stand der Technik und dessen Nachteile:

Micellen in wässriger Lösung, sowohl nichtionische, kationische und anionische sind in der Literatur in zahlreichen Veröffentlichungen beschrieben worden. (Mittal, K.L. (1977) Micellization, Solubilization and microemulsions, Plenum Press, New York. - Mittal, K.L. (1979), Solution Chemistry of Surfactants, Plenum Press, New York. - Menger, F.M. (1977). In Bioorganic Chemistry III. Macro-and Multicomponent Systems (E.E.Van Tanelen, Ed.), Academic Press, New York. - Menger, F.M. (1979a) Acc. Chem. Res. 12, 111-117. On the Structures of Micelles. - J.H.Fendler, E.J. Fendler (1975) Catalysis in micellar and macromolecular Systems, Academic Press.) Ihr Aufbau und ihre galenische, medizinische und technische Verwendung ist Gegenstand zahlreicher Untersuchungen. So ist die antiseptische Wirkung von Cetylpyridiniumchlorid, Benzethoniumchlorid und Benzalkoniumchlorid oder deren Bromide bekannt. Auch, daß sie in geringen Konzentrationen bakterizide Wirkung in vitro gegenüber einer großen Anzahl von grampositiven und gramnegativen Bakterien zeigen, wobei die gramnegativen wesentlich empfindlicher als die grampositiven reagieren. Auch sind bestimmte gramnegative Bakterien resistent gegenüber diesen quartären Ammoniumbasen, z.B. Pseud. cepalia, Mycobakt. tuberculosis.

Normalerweise haben kationische Micellen in wäßriger Phase zusätzlich in ihrem hydrophoben Kern, der weitgehend durch die aliphatische Kette und ihre Länge bestimmt wird, eine hydrophobe-hydrophile Grenzschicht (Sternlayer), die hydratisiert ist und z.T. die Gegenionen beherbergt. Die Größe dieser Grenzschicht liegt im allgemeinen zwischen 7-10 Å. Außerdem sind sie noch mit der Guy-Chapman-Layer von 10-20 Å umgeben, die nicht elektrostatisch gebundene Gegenionen, z.B. $Cl^-$, $Br^-$, $HSO_4^-$ und unstrukturiertes Wasser enthalten. Nur die Konzentrationen der Gegenionen als auch andere Ionen bewirken eine Senkung der kritischen Micellenbildungs-Konzentration (KMK) bei konstanter Temperatur, Druck und chemischen Potentials, wobei die Natur der Gegenionen die Form und Größe der Micellen in wässriger Phase bestimmen können. Dies bewirken allerdings nur die Fraktion von Gegenionen, welche im Stern-layer in der Nähe des quartären Stickstoffs sich befinden.

Die reinen, bislang bekannten kationischen quartären Ammoniumbasen - offiziell auch als Invertseifen bezeichnet - haben nur eine begrenzte und nicht spezifische antimikrobielle Wirkung (siehe z.B. W. Forth, D. Henschler, W. Rummel, Allgemeine und spezielle Pharmakologie und Toxikologie, 4. Auflage. B.I. Wissenschaftsverlag, 1983, S. 616). Daher sind ihre Einsetzbarkeit z.B. als Konservierungsmittel oder als Desinfiziens in den operativen Fächern der Medizin oder auf Infektionsstationen (Antiseptika) begrenzt, trotz ihrer geringen Toxizität. Domagk erkannte 1935 (siehe Wallhäußer, K.H.: Sterilisation, Desinfektion, Konservierung, Keimidentifizierung, Betriebshygiene. 2. Aufl., Thieme, Stuttgart, 1978), daß die quartären Ammoniumbasen nur dann bakterizid wirksam sind, wenn mindestens einer der Substituenten am Stickstoff aus einer linearen Alkylkette mit 8-18 Kohlenstoffatomen besteht, wobei die optimale Kettenlänge bei $C_{12}$-$C_{16}$ liegt. Die bekanntesten Vertreter dieser Stoffklasse sind die Benzalkonium-Salze (Chloride und Bromide). Darüber hinaus sind Hexadecylpyridinium-chlorid und Benzethonium-chlorid bekannt und haben medizinische und pharmazeutische Bedeutung erlangt. Die Wirkung dieser Invertseifen ist bekanntlich stark milieuabhängig. Durch Seifen z.B. wird die Wirkung weitgehend aufgehoben, wie auch im sauren pH-Bereich. Auch Blut, Eiter, Stuhl sowie Schmutz führen gleichfalls zur Inaktivierung. Außerdem haben sie eine Eiweiß fällende Wirkung, die schon bei geringen Konzentrationen der $N^+$-Tenside einsetzt, d.h. im Bereich von 1-2 Gew.% von wäßrigen Lösungen. Bei Konzentration dieser bekannten Tenside, welche nur das 2-3-fache der kritischen KMK betragen, erfolgt zwar keine eiweißfällende Wirkung (Denaturierung), doch eine reversible Inaktivierung von Enzymsystemen und Stützproteinen durch Entfaltung der aktiven dreidimensionalen Struktur. ("loss of activity through unfolding")

Bekannt ist auch die antibakterielle und unspezifische Wirkung von quartären Ammoniumverbindungen und ihre oberflächenaktive Wirkung, von Dequalinium-acetat, Cetyldimethyl-ammonium-bromid (CTAB) und Hexadecyl-pyridiniumchlorid (CPCl), (siehe z.B. Goodman and Gilman's, The Pharmacological Basis of Therapeutics, EDS. A.G. Goodman, L.S. Goodman, Th.W. Rall, F. Murad, 1985, 7th Edition, Collier,

MacMillan Publishing Company, N. Y., S. 971,; Merck Index, 1985). Die micellaren Eigenschaften dieser Verbindungen sind mit ihrer Oberflächenaktivität und antimikrobiellen Eigenschaften in Beziehung gesetzt worden (siehe Attwood, D, and Florence, A.T., Surfactant Systems, Chapman and Hall, London u. New York, 1983). Jedoch kann die unspezifische Oberflächenaktivität dieser quartären aliphatischen und aromatischen Ammoniumbasen nicht a priori als Voraussetzung für die antibakterielle, antifungale uund keratolytische Wirkung angesehen werden, da nichtionische Detergentien, z.B. Brij, Triton X 100, Lubrol etc. nicht reaktiv werden.

Organische quartäre Ammoniumbasen des Typs ($R_n$, $R_1$, $R_2$, $R_m$, $N^+$)$Y^-$, (HET=$N^+$-$(CH_2)_x$ -$CH_3$)$Y^-$ und $[H_3C)_3.C-CH_2-C(CH_3)_2-X_1-[O-(CH_2)_2]_2-N^+(CH_3)_2-CH_2-X_2]Y^-$ sind nur zum Teil bekannt, z.B. Hexadecyltrimethylammoniumchlorid und Bromid (Cetyltrimethylammonium-), Hexadecylpyridiniumchlorid bzw. Bromid (Cetylpyridiniumchlorid) und N,N'-Dimethyl-N-2 2-4-(1,1,3,3-tetrymethylbutyl)phenoxyethylphenyl methaniumchlorid (Benzethoniumchlorid, Methylbenzethoniumchlorid) und die Benzalkoniumchloride mit Alkylresten von $C_8H_{17}$ bis $C_{18}H_{37}$. Diese genannten $N^+$-Tenside haben alle eine kleine kritische Micellbildungskonstante (KMK) im Bereiche von $10^{-4}$-$10^{-5}$ Mol, je nach Milieubedingungen, wie z.b. Ionenstärke, Temperatur, Druck und Zusatz von organischen Lösungsmitteln bestimmter Dielektrizitätskonstanten. Der Einfluß eines Anions, $Y^-$, wie fraktionierte Bindungen, Zahl der Anionen an der Micelloberfläche (Sternlayer) und ihr Einfluß auf die geometrische Form der gesamten kationischen Micelle der oben genannten quartären organischen Ammoniumbasen ist bisher wenig untersucht. So auch die Form dieser o.g. Tenside in Gegenwart von Potenzierungsgemischen, wie Glyzerol, Dimethylsulfoxid, Ethanol, Propanol und ihre Stabilität gegnüber Temperatur und Aufnahmefähigkeit von hydrophoben (lipophilen) pharmazeutischen Wirkstoffen. Hier liegen keine quantifizierbare Untersuchungen auch der o.g. $N^+$-Tenside vor.

Tenside der allgemeinen Form (HET=$N^+$-$(CH_2)_x$-$CH_3$)$Y^-$, wobei der Heterozyklus ein Benzimidazol-, Pyrimidin-, Imidazol-,Thiazol-, Benz-thiazol oder Purinrest ist sind bislang nicht beschrieben worden, sowie ihr micellares Verhalten in wäßrigen Lösungen in Gegenwart und Abwesenheit von Potenzierungsgemischen. Dies gilt ebenso für substituierte Pyridiniumverbindungen, welche darüber hinaus - wie später gezeigt werden wird - in wäßriger Lösung Vesikel bestimmter Größe und Form bilden können.

Der relativ breite und undifferenzierte Wirkungsmechanismus der bereits bekannten quartären organischen Ammoniumbasen und das damit bedingte Anwendungsgebiet als Antiseptika und ihre toxische Wirkung bei höheren therapeutischen Dosen, hat die Anwendung dieser organischen quartären Ammoniumbasen pharmazeutisch beschränkt. Auch ist für 1 Gew.%-ige oder höher wäßrige Lösungen, Cremen und Salben hypersensitive, allergische und topische Irritationen beobachtet worden, so daß ein gezielter therapeutischer Einsatz nur bedingt möglich ist.

Bekannt ist die bakterizide Wirkung von Chlorhexidin bei grampositiven und gramnegativen Bakterien, jedoch resistent gegen gramnegative Bazillen.

Pharmazeutische Präparationen, welche eine gezieltere Therapie mit micellar eingeschlossenen pharmazeutischen Wirkstoffen, z.B. antiviraler, antifungaler, antineoplastischer Natur in therapeutisch wirksamen Dosen und einer geeigneten pharmazeutischen Zubereitung (Galenik) liegen nicht vor.

Ein großer Nachteil der bisher bekannten pharmazeutischen Zubereitungen von quartären organischen Ammoniumbasen, auch in Gegenwart von Potenzierungsgemischen, liegt in der Polydispersität der kolloidalen micellaren Lösungen. Je nach pharmazeuttischer Zubereitungsform, pH-Wert, Ionenstärke, Gegenanion $Y^-$ und Temperatur liegen in einer pharmazeutischen Zubereitung bislang Micellen verschiedener Form und Größe sowie Stabilität und Aufnahmekapazität von pharmazeutischen Wirkstoffen vor.

Im weitesten Sinne versteht man unter Micellen durch Assoziation gebildete Aggregate von gelösten Molekülen. Im engeren, heute hauptsächlich verwendeten Sinn bezeichnet man als Micellen diejenigen Aggregate, die sich aus Tensid-Molekülen in wäßrigen Lösungen oberhalb einer bestimmten Temperatur (Krafft-Punkt) bzw. einer charakteristischen Konzentration bilden. Diese Konzentration nennt man kritische Micellbildungskonzentration (KMK; englisch: critical micellization concentration, cmc). Beim Überschreiten der KMK bleibt die Monomerenkonzentration praktisch konstant und die überschüssigen Tensid-Moleküle bilden Micellen. Diese können in verschiedener Gestalt (Kugeln, Stäbchen, Scheibchen) auftreten, abhängig von der chemischen Konstitution des Tensids sowohl von Temperatur, Konzentration oder Ionenstärke der Lösung. Die Micellen haben charakteristische Aggregationszahlen mit einer meist nur geringen Verteilungsbreite. Das Erreichen der KMK gibt sich durch sprunghafte Änderungen der Oberflächenspannung, (was man zur Messung der KMK ausnützt) des osmotischen Drucks, der elektrischen Leitfähigkeit und Viskosität zu erkennen.

Bei den Micellen handelt es sich um thermodynamische stabile Assoziationskolloide grenzflächenaktiver Stoffe, bei denen die hydrophoben Reste der Monomeren im Inneren der Aggregate liegen und durch hydrophobe Wechselwirkung (van-der-Waals-Kräfte) zusammengehalten werden; die hydrophilen Gruppen sind dem Wasser zugewandt und vermitteln durch Solvatation die Löslichkeit des Kolloids.

Weitere Informationen über Micellen finden sich in Römpps Chemielexikon, 8. Auflage, Franckh'sche Verlagsbuchhandlung Stuttgart, 1985, Seite 2600ff.

Eine Aufgabe der vorliegenden Erfindung ist es, eine pharmazeutische Zubereitung zu schaffen, die den Wirkstoff in möglichst stabiler Form enthält und bei welcher der Wirkstoff am Ort des pathologischen Geschehens möglichst schnell und vollständig freigesetzt wird.

Diese Aufgabe wird erfindungsgemäß durch eine pharmazeutische Zubereitung gelöst, die dadurch gekennzeichnet ist, daß sie aufgebaut ist aus einer Micelle, bestehend aus einem kationischen Tensid mit einem einwertigen Anion und einem hydrophoben pharmazeutischen Wirkstoff, dispergiert in einem Lösungsmittel, dessen pH-Wert kleiner ≤ 7 ist, wobei die kritische Micellbildungskonzentration (KMK) im Bereich von $1{,}0 \cdot 10^{-7}$ bis $1{,}5 \cdot 10^{-4}$ Mol/Liter liegt.

Vorzugsweise ist diese pharmazeutische Zubereitung aufgebaut aus einer Micelle, bestehend aus einem kationischen Tensid mit einem einwertigen Anion in einer Menge von 0,01 bis 0,1 Gew.% bezogen auf die gesamte pharmazeutische Zubereitung, und einem hydrophoben pharmazeutischen Wirkstoff in einer Menge von 0,001 bis 0,5 Gew.% bezogen auf die gesamte pharmazeutische Zubereitung, dispergiert in einem Lösungsmittel, dessen pH-Wert ≤ 7,0 ist, in einer Menge von 99,40 bis 99,989 Gew.% bezogen auf die gesamte pharmazeutische Zubereitung, wobei die kritische Micellbildungskonzentration (KMK) im Bereich von $1{,}0 \cdot 10^{-7}$ bis $1{,}5 \cdot 10^{-4}$ Mol/Liter liegt.

Diese hier beschriebenen Micellen in wäßriger Phase haben bei einer hydrophoben Kettenlänge von 15-($CH_2$)-Gruppen einschließlich ihres quartären Stickstoffs im aromatischen Gebilde einen Durchmesser von ~50-100 Å, je nach Natur der Gegenionen.

<u>Beschreibung und Herstellung der quartären Ammoniumbasen:</u>

Das erfindungsgemäße kationische Tensid ist vorzugsweise eine Verbindung der allgemeinen Formel

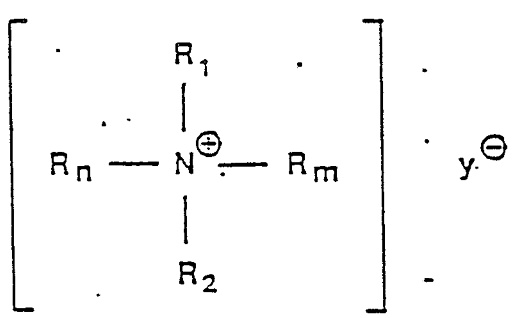

wobei vorzugsweise

$R_1$ = ein Alkylrest mit 1 - 12 C-Atomen oder ein Aralkylrest,

$R_2$ = ein Alkylrest mit 1 - 12 C-Atomen oder ein Aralkylrest,

$R_n$ = ein geradkettiger oder verzweigter Alkylrest, substituiert oder nicht substituiert, mit 1 - 22 C-Atomen, vorzugsweise 10 - 20 C-Atomen oder ein Alkenylrest mit 8 - 20 C-Atomen, vorzugsweise 8 - 10 C-Atomen oder ein 5-oder 6-gliedriger aromatischer Heterozyklus mit einem oder 2 Stickstoffatomen, und wahlweise einem Schwefelatom oder einem Sauerstoffatom und

$R_m$ = ein geradkettiger oder verzweigter Alkylrest, substituiert oder nicht substituiert, mit 1 - 22 C-Atomen, vorzugsweise 10 - 20 C-Atomen oder ein Alkenylrest mit 8 - 20 C-Atomen, vorzugsweise 8 - 10 C-Atomen oder ein 5-oder 6-gliedriger aromatischer Heterozyklus mit einem oder 2 Stickstoffatomen, und wahlweise einem Schwefelatom oder einem Sauerstoffatom oder ein Chinoliniumrest und

$y^-$ = ein einwertiges Anion ist.

Weitere Vorzugsweise Ausführungsformen sind:

Geradkettiges oder verzweigtes Alkyl $R_n$ mit $C_6$-$C_{22}$, insbesondere aber $C_{12}$-$C_{20}$, Kohlenstoffatom, ist beispielsweise n-Heptyl, 2-Methylhexyl-, 3-Methylhexyl, 3-Ethylpentyl, 2,2-, 2,3-, 2,4-, oder 3,3-Dimethylpentyl, n-Octyl, 4-Methylheptyl, 2,2,2-, 2,2,4-, 2,3,3-, 2,3,4-Trimethylpentyl, n-Nonyl, n-Decyl, n-Undecyl, n-Dodecyl, n-Tridecyl, n-Tetradecyl, n-Pentadecyl, n-Hexadecyl (Cetyl), n-Heptadecyl, n-Octadecyl, n-Nonadecyl oder n-Eicosyl (Arachinyl).

Bevorzugt wird ein geradkettiges Alkyl mit einer geraden Anzahl von 10-20 Kohlenstoffatomen, z.B. n-Dodecyl, n-Tetradecyl, n-Hexadecyl (Cetyl), n-Octadecyl oder n-Eicosyl. Sie haben alle die gleiche Bindungs-und Aufnahmekapazität von anorganischen und organischen (hydrophoben) Wirkstoffen, beispielsweise $Hg(CN)_2$, ZnEDTA, ZnO, und $K_{18}(KW_{21}Sb_9O_{86})_{17}$ als anorganische antivirale Wirkstoffe, und Azathioprin, Nystatin, Amphotericin, Idoxuridin, Cytarabin und Trifluorthymidin als organische Wirkstoffe.

Bevorzugt ist ein Alkenyl mit 12-20 Kohlenstoffatomen für $R_n$, wenn $R_m$ ein Methyl-, Ethyl bis zum Hexyl-Rest ist, speziell ein Alkenyl mit einer Doppelbindung, z.B. 9-cis-Dodecenyl, 9-cis-Tetradecenyl, 9-cis-Hexadecenyl, 6-cis-Octadecenyl, 6-trans-Octadecenyl und 9-cis-Octadecenyl.

$R_1$, $R_2$ und $R_m$ ist vorzugsweise Methyl-, Ethyl oder auch Hexyl.

Ein aromatischer Heterozyklus für $R_n$ der Formel (I) ist ein 5-oder 6-gliedriger Heterozyklus, mit einem oder zwei Stickstoffatomen und wahlweise einem Stickstoff-und einem Schwefelatom z.B. ein Pyridin-, ein Pyrimidin-, ein Pyrazin-(1,4-Diazin), ein Pyrazol-, ein Imidazol-, ein Thiazol-und Purinrest (7N-Imidazolium-[4,5-d]pyrimidin) oder ein benzokondensierter Thiazol-und Imidazolrest z.B. $N_3$-Benzimidazol oder Benzthiazol.

Substituenten dieses Heterozyklus sind am Stickstoffatom sowie gegebenenfalls an einem Kohlenstoffatom ein Niederalkyl, z.B. Methyl oder Aethyl, oder ein Hydroxyniederalkyl, z.B. Hydroxymethyl oder 2-Hydroxyäthyl, Oxo, Hydroxy oder Halogen, z.B. Chlor oder Brom.

Ein Heterocyclus ist vorzugsweise 2-oder 4-Niederalkylpyridinium z.B. 2-oder 4-Methyl oder 2-oder 4-Aethylpyridinium, Diniederalkylpyridinium z.B. 2,6-Dimethyl-, 2-Methyl-3-äthyl-, 2-Methyl-4-äthyl-, 2-Methyl-5-äthyl-oder 2-Methyl-6-äthylpyridinium, 2-, 3-oder 4-Halogenpyridinium z.B. 2-, 3-oder 4-Chlorpyridinium oder 2-, 3-oder 4-Brompyridinium, 2-Niederalkylimidazolinium, -oxazolinium oder -thiazolinium z.B. 2-Methyl-oder 2-Aethylimidazolinium, -oxazolinium oder -thiazolinium oder 2-Niederalkyl-8-halogenchinolinium z.B. 2-Methyl-8-chlorchinolinium.

$Y^{\ominus}$ ist ein Anion, vorzugsweise Chlorid, Bromid, Jodid oder Ethylsulfat, ein Niederalkonat, wie Formiat, Acetat, Propionat, Hydrogensulfat ($HSO_4^-$), Malat oder Fumarat, Salizylat, Alginat oder Glukonat.

Ein kationisches Tensid der allgemeinen Formel (I) ist vorzugsweise N-Benzyl-N,N-dimethyl-N-2-[2-(4-(1,1,3,3-tetramethylbutyl)-phenoxy)-äthoxy]-äthylammoniumchlorid, N-Benzyl-N,N-dimethyl-N-2[2-(3-methyl-4-(1,1,3,3-tetramethylbutyl)-phenoxy)-äthoxy]-äthylammoniumchlorid (Methylbenzethoniumchlorid), n-Dodecyltrimethylammoniumchlorid oder - bromid, Trimethyl-n-tetradecylammoniumchlorid oder -bromid, n-Hexadecyltrimethylammoniumchlorid oder -bromid (Cetyltrimethylammoniumchlorid oder -bromid), Trimethyl-n-octadecylammoniumchlorid oder -bromid, Aethyl-n-dodecyldimethylammoniumchlorid oder -bromid, Aethyldimethyl-n-tetradecylammoniumchlorid oder -bromid, Aethyl-n-hexadecyldimethylammoniumchlorid oder -bromid, Aethyldimethyl-n-octadecylammoniumchlorid oder -bromid, n-Alkyl-benzyldimethylammoniumchlorid oder -bromid (Benzalkoniumchlorid oder -bromid), z.B. Benzyl-ndodecyldimethylammoniumchlorid oder -bromid, Benzyldimethyl-n-tetradecalammoniumchlorid oder -bromid, Benzyl-n-hexadecyldimethylammoniumchlorid oder -bromid oder Benzyldimethyl-n-octadecylammoniumchlorid oder -bromid, N-(n-Decyl)-pyridiniumchlorid oder -bromid, N-(n-Dodecyl)-Pyridiniumchlorid oder -bromid, N-(n-Tetradeyl)-pyridiniumchlorid oder -bromid, N-(n-Hexadecyl)-pyridiniumchlorid oder -bromid (Cetylpyridiniumchlorid) oder N-(n-Octadecyl)-pyridiniumchlorid oder -bromid oder eine Mischung von diesen Tensiden.

Ein kationisches Tensid der allgemeinen Formel (I) $R_nN^{\oplus}(R_1,R_2)R_mY^{\ominus}$ ist vorzugsweise mit $R_n = R_1 = R_2$ z.B. $R_nN^{\oplus}(CH_3)_3Y^{\ominus}$ 3-Methyl-Hexyl-trimethyl-Ammoniumchlorid, n-Nonyl-Trimethyl-Ammoniumbromid, n-Undecyl-trimethyl-Ammoniumchlorid, n-Hexadecyl-trimethyl-Ammoniumchlorid, n-Octadecyl oder n-Eicosyl-trimethyl-Ammoniumbromid mit einer geraden Anzahl von 12-20 Kohlenstoffatomen.

Auf der Grundlage einer Mikro-Emulsion und/oder Salbe z.B. in Gegenwart bis zu 10 % ($^g/g$) DMSO haben diese N-Tenside gleiche antifungale, antibakterielle und keratolytische Eigenschaften wie die nicht kovalent gebundenen pharmazeutischen Wirkstoffe.

Die Herstellung der Tenside der allgemeinen Formel $R_n$ $N^{\oplus}(R_1,R_2)R_mY^{\ominus}$ sind analog dem in dem Standardwerk "Cationic Surfactants" von E. Jungermann, Dekker, N.Y., 1970 beschrieben, herzustellen, siehe auch das jährlich neu erscheinende Handbuch "Mc Cutcheon's Emulcifiers and Detergents" Manufacturing Cofectioner Publishing Co.. Andere Alkyl-Pyridinium Halogenide können durch Reaktion von stöchiometrischen Mengen des Pyridinderivates mit langkettigen Alkylhalogeniden in guter Ausbeute erhalten werden. Andere Verfahren gehen von den entsprechenden, ultra-zyklischen N-Verbindungen und 1,3-Propanmethan aus, wie z.B. bei F.J. Fendler et al, J.Chem.Soc., Perkin Trans III., 1097 (1977) beschrieben. Andere Verfahren, welche zu ähnlich guter Ausbeute führen, sind z.B. bei Attwood, D., Elwarthy, P.H., and Kaye, S.B., J.Phys.Chem. 74 , 3529 (1970) beschrieben, sie können analog für die Synthese der Substanzen der Formel II angewendet werden. Die pharmazeutischen Wirkstoffe sind im Handel erhältlich.

Die Synthese der Verbindungen der allgemeinen Formel $R_n$, $R_m$, $R_1$, $R_2N^{\oplus}$ $Y^{\ominus}$ oder $R_n$, $R_m$ $N^{\oplus}(CH_3)_2$ $Y^{\ominus}$ im speziellen werden nach folgender Vorschrift dargestellt:

a) Das entsprechende Alkyljodid oder Bromid wird mit einem Überschuß von Trimethylamin (Halogenid: Amin = 1:1,45) für 24 Stunden bei 20°C zur Herstellung der entsprechenden quartären Ammoniumbase im Autoklaven stehen gelassen. Kein anderes Lösungsmittel als Methanol, welches mit dem Trimethylamin oder $R_1$, $R_2$-Alkylamin gesättigt worden ist, wurde verwendet. Das Reaktionsgemisch wird in ein 5-faches Volumen von Ether eingerührt und am Rückfluß für 20 min erhitzt. Der feste Rückstand, der sich nach Abkühlung in Ether bildet, wird abfiltriert. Umkristallisiert wird aus Chloroform. Die Kristalle werden wiederholte male mit wasserfreiem Ether gewaschen. Die Rekristallisationen bis zum konstanten Schmelzpunkt wurden aus Ethanol/Ether (1:1, % $^{g}$/g) in Gegenwart von aktivierter Holzkohle vorgenommen. Die Kristalle wurden bei 80 °C über Kalziumchlorid unter Vakuum bei 1 mm/Hg über Nacht getrocknet.

b) Zur Herstellung von $R_n$, $R_m$, $R_1$, $R_2N^{\oplus}$ $Y^{\ominus}$ werden die entsprechenden Amine $R_1$, $R_2$-$N^{\oplus}$-Amine mit den stöchiometrischen Mengen der $R_n$, $R_m$-Jodide in abolutem Ethanol-Hexan(1:2 % $^{g}$/g) für 48 Stunden am Rückfluß gekocht. Danach wird die Reaktion abgekühlt und in einen 5-fachen Überschuß von Ether gegossen und abfiltriert. Die Rekristallisation erfolgte wie unter a) angegeben.

c) Um die quartären Ammoniumhalogenide in die entsprechenden Bromide, Chloride oder auch Jodide umzuwandeln, bietet sich folgendes Verfahren an:
300 g Amberlite IRA-400 (4 mequiv/g) als Chloridform vorliegend, werden in einer Säule (45×5 cm) gefüllt und mit 1 Liter einer 20%igen wässrigen Lösung Kaliumchlorid oder Kaliumbromid oder Kaliumjodid oder $KY^{\ominus}$ bei sehr langsamer Durchflußzeit gewaschen. Die Matrix wurde danach mit deionisiertem Wasser gewaschen bis keine Reaktion auf Chlorid oder Bromid oder Jodid mehr eintrat. Anschließend wurde die Säulenmatrix mit einer 10%igen wässrigen Lösung eines quartären Ammoniumbromides beladen. Die nachfolgende Elution erfolgte mit Wasser bei einer Flußrate von 1 ml/min. Das entsprechende quartäre Ammoniumbromid bzw. -halogenid wurde durch Konzentrieren des Eluates am Rotationsverdampfer erhalten. Die Rekristallisation erfolgte wie unter a) beschrieben. Die nachfolgende Tabelle 4 zeigt einige kationische Tenside der Form $R_n$ $N^{\oplus}(CH_3)_3$ $Y^{\ominus}$, welche nach diesem Verfahren hergestellt worden sind.

Eine Unterklasse der Verbindungen der allgemeinen Formel (I) ist die Verbindung der allgemeinen Formel

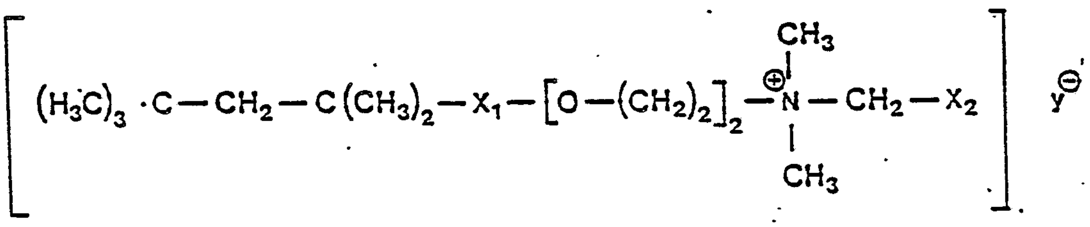

Es handelt sich um Abkömmlinge der Benzethoniumhalogenide. Durch Substitution der Reste $X_1$ und $X_2$ wobei $X_1 = X_2$ sein kann, können analog hergestellt werden wie sie bereits im US-Patent 2,115,250 von (1938) oder auch nach US-Patent 2,170,111 von (1939) und 2,229,024 von (1941) beschrieben sind. Diese speziellen N-Tenside sind besonders stabil auch in Gegenwart eines Potenzierungsgemisches und haben überraschenderweise eine hohe Aufnahmekapazität zum micellaren Einschluß von pharmazeutischen Wirkstoffen. Außerdem sind sie bei verfahrensgemäßer Herstellung milieuunabhängig. $Y^{\ominus}$ ist ein Anion z.B. Chlorid, Bromid und auch Jodid, ein Niederalkonat, wie Formiat, Acetat, Propionat, Malat oder Fumarat, Salizylat, Alginat oder Glukonat.

Tabelle 4:

Preparation und Schmelzpunkt, sowie Elementaranalyse der quartären Ammonium-Verbindungen des Typus $RN^{+}(CH_3)_3Y^{\ominus}$ aus $R_n$, $R_m$, $R_1$, $R_2$ $N^{\ominus}$ $Y^{\ominus}$ mit $R_1$=$R_2$ und $R_n$=$R_m$.

| Nr. | R | $Y^{\ominus}$ | KMK Mol | Fp. °C | Analyse gefunden C | H | N | Y |
|---|---|---|---|---|---|---|---|---|
| 1 | Methyl | Br | $1,5x10^{-5}$ | | | | | |
| 2 | Ethyl | I | $2,0x10^{-5}$ | >300[c] | 27,90 | 6,56 | 6,49 | |
| | | | | >300[d] | 27,92 | 6,56 | 6,51 | |
| 3 | n-Propyl | I | $2,0x10^{-5}$ | 190 | 31,51 | 7,05 | 6,09 | |
| | | | | 189 | 31,46 | 7,04 | 6,11 | |
| 4 | Isopropyl | I | $3,5x10^{-5}$ | >300 | 31,50 | 7,08 | 6,09 | |
| | | | | 316 | 31,46 | 7,04 | 6,11 | |
| 5 | n-Butyl | I | $4,1x10^{-5}$ | 231 | 34,69 | 7,48 | 5,72 | |
| | | | | 226 | 34,58 | 7,46 | 5,76 | |
| 6 | t-Butyl | I | $6,0x10^{-6}$ | 256 | 34,66 | 7,47 | 5,72 | |
| | | | | 260 | 34,58 | 7,46 | 5,76 | |
| 7 | n-Pentyl | I | $7,0x10^{-5}$ | 224 | 37,28 | 7,86 | 5,41 | |
| | | | | | 37,37 | 7,84 | 5,45 | |
| 8 | 1-Methylbutyl | I | $1,0x10^{-6}$ | 224 | 37,48 | 7,87 | 5,43 | 49,17 |
| | | | | | 37,37 | 7,84 | 5,45 | 49,34 |
| 9 | n-Hexyl | I | $7,9x10^{-6}$ | 160 | 39,68 | 8,19 | 5,11 | |
| | | | | 166 | 39,86 | 8,18 | 5,16 | |
| 10 | Cyclopentyl | I | $6,0x10^{-6}$ | 271 | 37,78 | 7,13 | 5,41 | 49,63 |
| | | | | | 37,66 | 7,11 | 5,47 | 49,74 |
| 11 | Cyclohexyl | I | $7,1x10^{-6}$ | 271 | 40,25 | 7,48 | 5,18 | |
| | | | | | 40,16 | 7,49 | 5,20 | |
| 12 | Allyl | I | $1,5x10^{-7}$ | 104 | 31,81 | 6,22 | 6,15 | 55,76 |
| | | | | 102 | 31,73 | 6,21 | 6,17 | 55,89 |
| 13 | 2-Propynyl | I | $6,0x10^{-5}$ | 181 | 32,09 | 5,40 | 6,19 | 56,29 |
| | | | | | 32,01 | 5,37 | 6,22 | 56,39 |
| 14 | 3-Butenyl | I | $3,5x10^{-5}$ | 236 | 34,93 | 6,70 | 5,78 | 52,56 |
| | | | | | 34,87 | 6,69 | 5,81 | 52,63 |

| Nr. | R | $Y^{\ominus}$ | K·K Mol | Fp. °C | Analyse gefunden C | H | N | Y |
|---|---|---|---|---|---|---|---|---|
| 15 | Phenyl | I | $7{,}0\times10^{-5}$ | 227 | 41,12 | 5,38 | 5,31 | 48,15 |
| | | | | 227 | 41,08 | 5,36 | 5,32 | 48,23 |
| 16 | Benzyl | I | $7{,}3\times10^{-5}$ | 179 | 43,33 | 5,82 | 5,00 | |
| | | | | 179 | 43,33 | 5,82 | 5,05 | |
| 17 | 4-Chlorbutyl | I | $5{,}1\times10^{-6}$ | 182 | 29,42 | 5,97 | 5,01 | |
| | | | | | 30,28 | 6,17 | 5,05 | |
| 18 | 4-Brombutyl | I | $7{,}0\times10^{-6}$ | 131 | 25,30 | 5,40 | 4,62 | |
| | | | | | 26,10 | 5,32 | 4,35 | |
| 19 | 4-Jodbutyl | I | $1{,}5\times10^{-7}$ | 160 | 23,43 | 4,75 | 4,00 | 67,80 |
| | | | | | 22,78 | 4,64 | 3,79 | 68,79 |
| 20 | 2-Ethoxyethyl | Br | $2{,}0\times10^{-7}$ | 174 | 39,07 | 8,44 | 6,49 | 38,48 |
| | | | | | 39,63 | 8,55 | 6,60 | 37,67 |
| 21 | 2-Phenoxyethyl | Br | $1{,}5\times10^{-7}$ | 162 | 50,74 | 6,98 | 5,34 | 30,79 |
| | | | | | 50,78 | 6,97 | 5,38 | 30,71 |
| 22 | p-Methylbenzyl | Br | $2{,}0\times10^{-7}$ | 197 | 53,97 | 7,78 | 5,66 | 32,49 |
| | | | | | 54,10 | 7,43 | 5,74 | 32,72 |
| 23 | p-Fluorbenzyl | Br | $2{,}5\times10^{-7}$ | 237 | 48,32 | 6,10 | 5,61 | |
| | | | | | 48,40 | 6,09 | 5,65 | |
| 24 | p-Chlorbenzyl | Br | $3{,}0\times10^{-5}$ | 207 | 45,39 | 5,71 | 5,29 | |
| | | | | | 45,39 | 5,75 | 5,26 | |
| 25 | p-Brombenzyl | Br | $4{,}0\times10^{-5}$ | 220 | 38,93 | 4,92 | 4,52 | 51,59 |
| | | | | | 38,86 | 4,89 | 4,53 | 51,71 |

Das erfindungsgemäße kationische Tensid ist vorzugsweise eine Verbindung der allgemeinen Formel
$[HET \equiv N^{+}\text{-}(CH_2)_x\text{-}CH_3]\ y^{-}$
wobei
$HET \equiv N^{+}$ -ein substituierter oder nichtsubstituieter Pyridiniumrest oder
ein substituierter oder nicht substituierter Pyrimidiniumrest oder
ein substituierter Pyrazin-(1,4-Diazinium)rest oder
ein Imidazoliumrest (4,5-d)pyrmidin Rest substituiert oder nicht substituiert , oder
ein substituierter oder nicht substituierter Imidazolium-Rest oder
ein substituierter oder nicht substituierter Pyrazoliumrest, oder
ein substituierter oder nicht substituierter Thiazoliumrest, oder
ein substituierter oder nicht substituierter Benz-thiazoliumrest, oder
ein substituierter oder nicht substituierter Benz-imidazoliumrest,
x = 8 bis 20 und
$y^{-}$ = Chlorid, Bromid, Jodid, Formiat, Acetat, Propionat, Hydrogensulfat, Malat, Fumarat, Salicylat, Alginat, Glukonat oder Ethylsulfat
bedeuten.

Vorzugsweise Ausführungsformen dieses kationischen Tensids sind folgende Verbindungen:
In den folgenden Ausführungsformen, in denen $y^-$ vorkommt, bedeutet dieses $y^-$ jeweils eines der vorstehenden dreizehn Anionen.

N-Alkyl-pyridinium der Formel

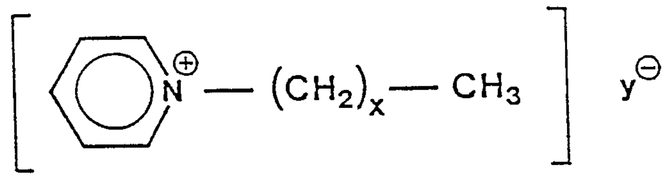

Hexadecylpyridinium der Formel

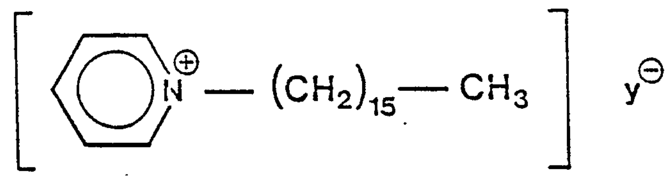

N-Alkyl-4-hydroxypyridinium der Formel

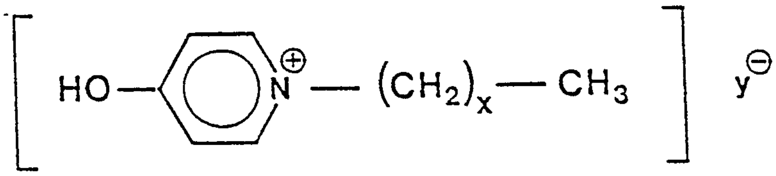

Hexadecyl-4-hydroxypyridinium der Formel

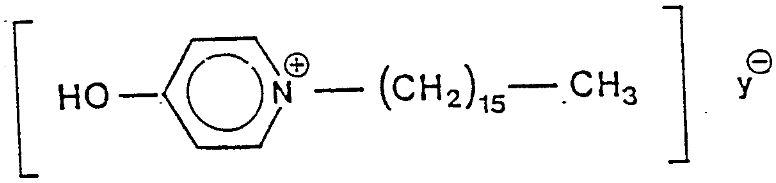

2,5,6 substituierte $N_1$-Alkyl-pyrimidinium-Verbindungen der Formel

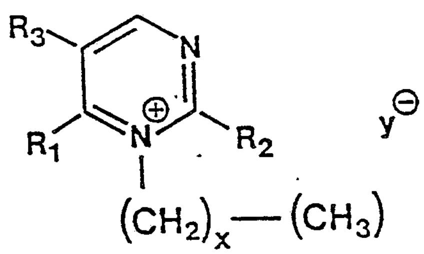

$R_1 = R_2 = R_3 = H$

$R_1 = NH_2$; $R_2 = OH$; $R_3 = H$

$R_1 = NH_2$; $R_2 = OH$; $R_3 =$

$R_1 = OH$; $R_2 = OH$; $R_3 = CH_3$

$R_1 = OH$; $R_2 = OH$; $R_3 = H$

$R_1 = F$; $R_2 = OH$; $R_3 = H$

$R_1 = OH$; $R_2 = OH$; $R_3 = F$

2,5,6 substituiertes $N_1$-Hexadecylpyrimidinium der Formel

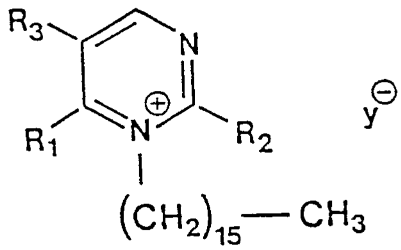

$R_1 = R_2 = R_3 = H$

$R_1 = NH_2$ ; $R_2 = OH$ ; $R_3 = H$

$R_1 = NH_2$ ; $R_2 = OH$ ; $R_3 =$

$R_1 = OH$ ; $R_2 = OH$ ; $R_3 = CH_3$

$R_1 = OH$ ; $R_2 = OH$ ; $R_3 = H$

$R_1 = F$ ; $R_2 = OH$ ; $R_3 = H$

$R_1 = OH$ ; $R_2 = OH$ ; $R_3 = F$

4-n-Alkyl-pyrazinium-2-carboxamid der Formel

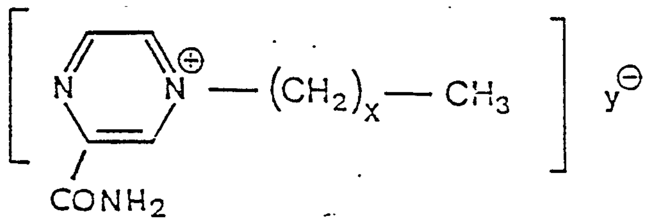

4-Hexadecylpyrazinium-2-carboxamid der Formel

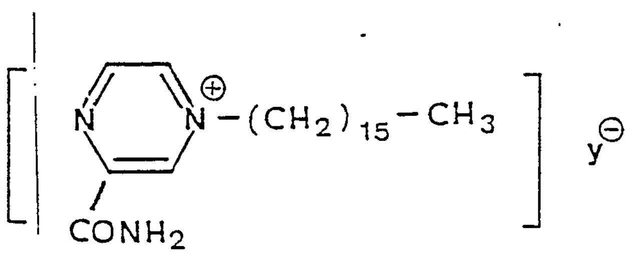

7-n-Alkyl-imidazolium [4,5-d]-pyrimidin der Formel

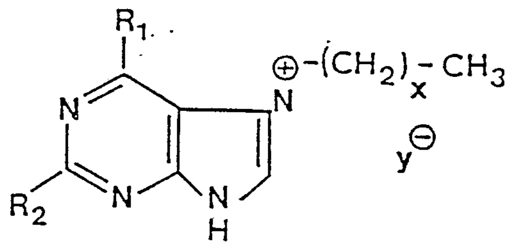

$R_1 = OH$ ; $R_2 = OH$
$R_1 = H$ ; $R_2 = H$
$R_1 = F$ ; $R_2 = NH_2$
$R_1 = F$ ; $R_2 = OH$
$R_1 = NH_2$ ; $R_2 = H$
$R_1 = NH_2$ ; $R_2 = NH_2$

7-Hexadecylimidazolium [4,5-d]pyrimidin der Formel

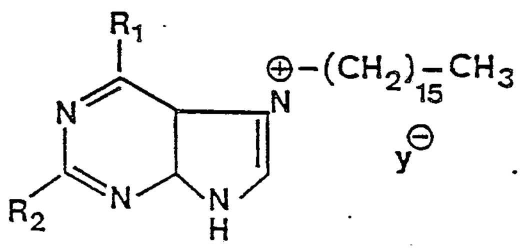

$R_1 = OH$ ; $R_2 = OH$
$R_1 = H$ ; $R_2 = H$
$R_1 = F$ ; $R_2 = NH_2$
$R_1 = F$ ; $R_2 = OH$
$R_1 = NH_2$ ; $R_2 = H$
$R_1 = NH_2$ ; $R_2 = NH_2$

3-n-Alkyl-5,6-substituierte Benzimidazolium-Verbindungen der Formel

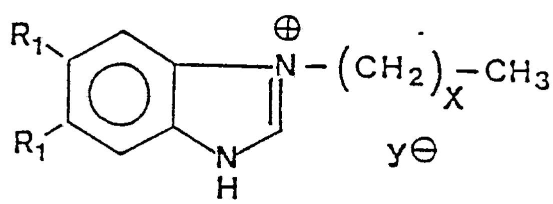

$R_1 = OH$

*4-substituierte 2-Hexadecylpyrazolium-Verbindungen der Formel*

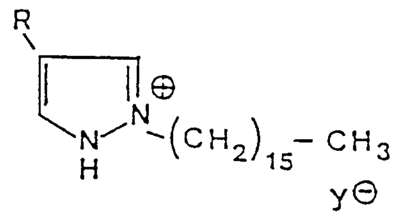

$R = H;\ CH_3;\ OH$

*1-n-Alkyl-4-substituierte Imidazolium-Verbindungen*

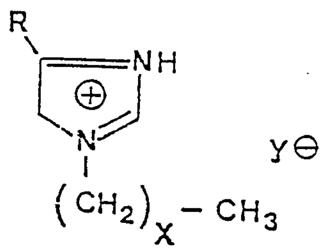

$R = H;\ CH_3;$

*1-Hexadecyl-4-substituierte Imidazolium-Verbindungen der Formel*

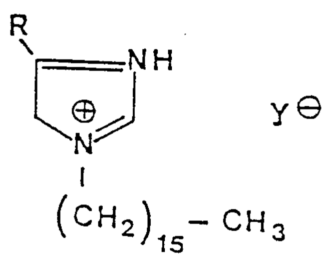

$R = H;\ CH_3;$

*3-n-Alkyl-5,6-substituierte Thiazolium-Verbindungen der Formel*

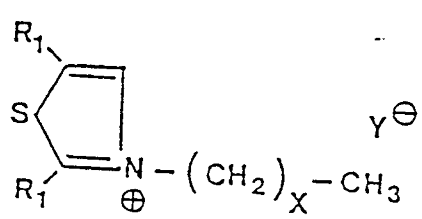

$R_1 = H;$

$R_1 = CH_3;$

*3-n-Hexadecyl-5,6-substituierte Thiazolium-Verbindungen der Formel*

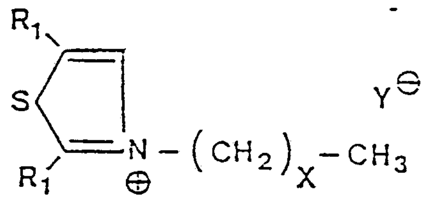

$R_1 = H$;

$R_1 = CH_3$;

*3-n-Alkyl-5,6-substituierte Benzthiazolium-Verbindungen der Formel*

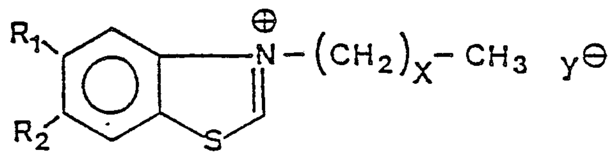

$R_1 = R_2 = H$

$R_1 = CH_3$

$R_1 = R_2 = OH$

$R_1 = R_2 = CH_3$

*4-[1,1bis n-Alkyl ·(Niederalkyl)] N-Hexadecylpyridinium-Verbindungen der Formel*

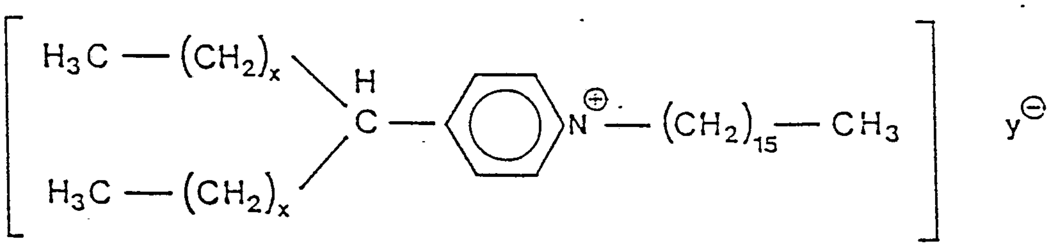

*3,5 bis [(n-Alkyloxy)carbonyl-] N-Hexadecylpyridinium-Verbindungen der Formel*

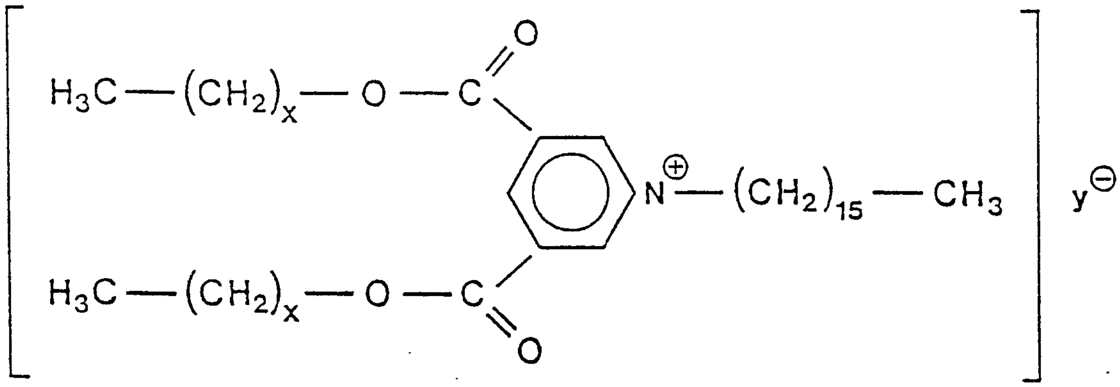

*4-(17-tritriacontyl)-n-methyl-pyridiniumchlorid der Formel*

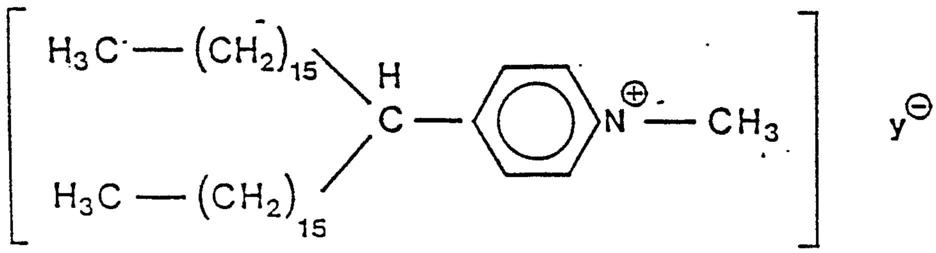

3,5bis[(n-hexadecyloxy)carbonyl)]-N-methyl-pyridinium-chlorid

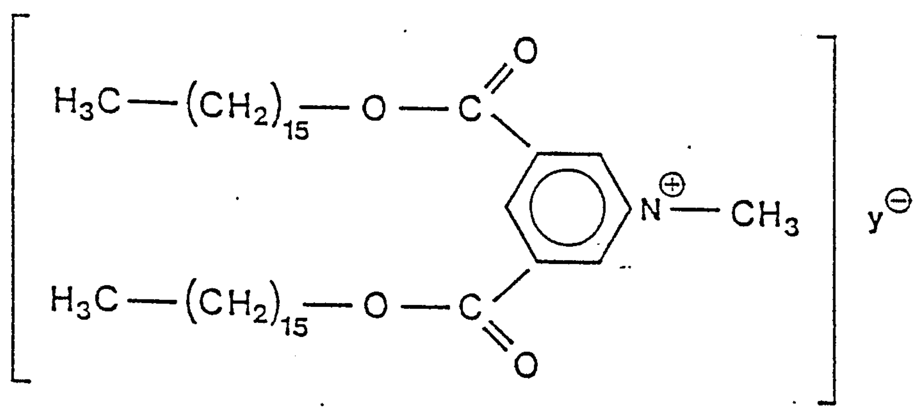

Kationische Tenside der allgemeinen Formel

$$\left[ (H_3C)_3 \cdot C - CH_2 - C(CH_3)_2 - X_1 - \left[ O - (CH_2)_2 \right]_2 - \overset{\oplus}{\underset{CH_3}{\overset{CH_3}{N}}} - CH_2 - X_2 \right] y^{\ominus}$$

wobei

$x_1$ = ein nichtsubstituierter oder in der 4-Stellung oder in der 3,5-Stellung oder in der 1,2,4,5-Stellung substituierter Phenylrest,

$x_2$ = ein nichtsubstituierter oder in der 4-Stellung oder in der 3,5-Stellung oder in der 1,2,4,5-Stellung substituierter Phenylrest und

y. = die Anionen gemäß dem Patentanspruch 78

bedeuten.

Generelles zur Herstellung der ($HET \equiv N^+$-$(CH_2)_x$-$CH_3$) $Y^-$-Verbindungen II:

Die erfindungsgemäßen kationischen Tenside der allgemeinen Formel II, außer Hexadecylpyridiniumhalogenid, sind neu.

In dem kationischen Tensid der allgemeinen Formel II ist HET $\equiv N^+$ vorzugsweise ein substituierter oder nichtsubstituierter Pyridiniumrest oder ein substituierter oder nicht substituierter Pyridiniumrest oder ein substituierter Pyrazin-(1,4-Diazinium)rest oder ein Imidazoliumrest (4,5-d)pyrimidin Rest substituiert oder nicht substituiert, oder ein substituierter oder nicht substituierter Imidazolium-Rest oder ein substituierter oder nicht substituierter Pyrazoliumrest, oder ein substituierter oder nicht substituierter Thiazoliumrest oder ein substituierter oder nicht substituierter Benz-thiazoliumrest, oder ein substituierter oder nicht substituierter Benz-imidazoliumrest.

Diese kationischen Tenside sind dadurch charakterisiert, daß sie eine sehr kleine Micellbildungskonstante (KMK) von ungefähr $1{,}5 \times 10^{-7}$Mol haben, sehr stark antimikrobiell, antifungal wirksam sind, keine Polydispersität in Gegenwart von anorganischen Anionen bzw. Potenzierungsgemischen zeigen, und z.T. selbst mikrobielle Stoffwechselprodukte (Antimetabolite) sind, die nicht toxisch für die Wirtzelle sind.

Die Ausbildung der salzartigen Struktur dieser Klasse von kationischen Tensiden der Form(HET$\equiv N^{\oplus}$-$(CH_2)_x$-$CH_2$) Y$\ominus$ ist u.a. in der Elektronendichte-Verteilung der heteroaromatischen Kerne bzw. in ihrer Basizität, einschließlich des Einflußes der Substituenten, begründet. Eine notwendige Bedingung, welche zur Ausbildung von quartären Salzen dieser fünf-und sechsgliedrigen heteroaromatischen Klasse führt, besteht darin, daß die Elektronendichte am Stickstoff, welcher quartärniert wird, nach MO-SCF-Rechnungen einen Betrag von -0,08 (z.B. Pyrazin-$N_4$) bis -0,159 (z.B. Imidazol-$N_1$, Purin-$N_7$) haben muß. Die Stabilität der einzelnen hier beschriebenen heterozyklischen kationischen Tenside wird außerdem noch durch ihre Symmetrie und Kettenlänge der Alkylkette am quartären Stickstoff bestimmt:

Im Falle des Imidazols, Benzimidazols z.B. wird durch die Ausbildung des Salzes am quartären Stickstoff $N_1$ und das freie Elektronenpaar am $N_3$ und der dadurch bedingten hohen Symmetrie stabilisiert. Ähnliches gilt für das $H_9$-Tautomere des Purins und seiner symmetrisch angeordneten Substituenten, welche die negativen Ladungen am $N_1$ (-0,124), $N_3$ (-0,108) und $N_9$ (-0,149) dergestalt beeinflussen, daß die Quartärnisation am $N_9$ bevorzugt wird, indem sich die o.g. Reihe $N_1 \rightarrow N_3 \rightarrow N_9$ umkkehrt. Durch die Wahl von geeigneten Lösungsmitteln kann man die Ausbeuten erhöhen. Während für Pyridin-, Pyrimidin-und Imidazolreste symmetrische Effekte am Kern eine wesentliche Rolle spielen, ist z.B. bei Pyrazin der elektronische Effekt in der 2-Stellung bedeutend, jedoch gibt es auch sehr starke induktive Effekte (z.B. 2-Amino-Gruppe), weniger als Mesomere. Dies gilt auch für das Pyrazol.

Die Länge der Alkylkette am quartären Stickstoffatom bestimmt nicht nur Schmelzpunkt und Hyrophobizität der später in wässrigen Lösungen gebildeten kationischen Micellen, sondern auch die Ausbeuten nehmen mit zunehmender Kettenlänge ab, während die Reaktionszeiten z.B. in Nitrobenzol oder 2-Ethoxyethanol zunehmen.

Stabile und leicht kristallierbare Verbindungen werden für $C_{12}$-$C_{18}$ erhalten, wobei das Gegenion Y$\ominus$ ausnahmslos Bromid und Chlorid ist. Die anderen Verbindungen können leicht aus Aceton oder Chloroform umkristallisiert werden. Die entsprechenden Jodverbindungen sind temperatur-und lichtempfindlich.

<u>Spezielle Herstellung der $(HET{\equiv}N^{\oplus}-(CH_2)_x-CH_3)\,Y^{\ominus}$-Verbindungen.</u>

a) Die entsprechenden Verbindungen des Pyridins oder substituierten Pyridins als sechsgliedriger Heterozyklus lassen sich aus der entsprechenden Alkylbromiden oder Jodiden in Methanol bei 35 °C und Pyridin bzw. substituierten Pyridinen mit einer Ausbeute von 70 % herstellen. Die entsprechenden molaren Mengen des Alkylbromides, die fast alle im Handel erhältlich sind, aber durch Hochdruckflüssigkeitschromatographie (HPLC) präparativ nachgereinigt werden müssen, werden zunächst in Methanol (10facher Volumenüberschuss gemessen am Pyridin) gelöst, und unter Stickstoff die stöchiometrische Menge Pyridin, das ebenfalls in Methanol gelöst ist, unter Rühren zugetropft. Es wird über 6 Stunden unter Rühren am Rückfluß bei 70°C erhitzt, so daß die Reaktionsausbeute fast quantitativ ist. So ist z.B. die Ausbeute von Hexadecyl-4-hydroxy-pyridiniumchlorid oder Bromid in Methanol als Lösungsmittel 95 %, mit Ethanol 80 % und in Ether/Ethanol nur 40 %. Dodecylpyridinium-chlorid wird mit einer Ausbeute von fast 70 % erhalten. 3,5-Dihydroxy-dodecylpyridinium-bromid bildet sich quantitativ nach der vorhergehenden Vorschrift aus Dodecyl-bromid und 3,5-Dihydroxypyridin in siedendem Chloroform nach 4 Stunden (Schmelzpunkt 180°C).

Reinigung der entsprechenden Pyridiniumverbindungen. -Durch wiederholtes Umkristallisieren aus Gemischen von Methanol/Ether, beginnend mit $^{40}/_{60}$($^{v}/_{v}$); $^{50}/_{50}$($^{v}/_{v}$) und schließlich $^{90}/_{10}$($^{v}/_{v}$) erhält man die gewünschten Produkte mit konstantem Schmelzpunkt, einheitlich Molekulargewicht und spezifischer oberflächenaktiven Eigenschaften (gemessen durch die Konzentrationsabhängigkeit der Oberflächenspannung). Außerdem zeigen diese Verbindungen die vorne geschilderten typischen $^{1}$H-NMR-Signale. Die zahlreichen $CH_2$-Gruppen und die $CH_3$-Gruppe erzeugen eine deutlich sichtbare Absorptionsschwingung im IR-Spektrum bei 2930 $cm^{-1}$ und 2850 $cm^{-1}$ (Methylengruppe) eine mittelschwache Bande bei 2960 $cm^{-1}$ und eine schwache bei 2870 $cm^{-1}$, welche der Methylgruppe zugeordnet werden kann.

Eine schnelle und quantitative Trennung der n-Alkyl-pyridiniumhalogenide von nicht umgesetzten n-Alkylbromiden und Pyridin wird durch präparative Hochdruckflüssigkeitschromatographie auf einer RP18-Säule mit Hilfe des Elutionsgemisches bestehend aus 60 %($^{v}/_{v}$)Methanol (Ethanol) und Acetonitril 40%($^{v}/_{v}$) isokratische bei 9,52 atm Säulendruck erreicht (UV-Detektion bei 260 nm).

b) Pyrimidin-Verbindungen

1.) Hexadecylpyrimidinium -bromid.-0,01 Mol 5-Aminopyrimidin ( 0,95 g) und Hexadecylbromid, 0,01 Mol ( 3,05 g) werden in 20 ml Methanol unter Rühren und Stickstoff bei 20°C 24 Stunden in Gegenwart von katalytischen Mengen (0,5 mg) Natriumamid umgesetzt. Das entstandene $N_1$-Hexadecyl-5-aminopyrimidinium-bromid wird in Aceton bei 76°C gelöst, und nach dem Abkühlen auf Zimmertemperatur kristallisiert das $N_1$-Hexadecyl-5-aminopyridinium-bromid mit Schmelzpunkt 122°C. Ausbeute 35 %.

0,01 Mol von diesem $N_1$-Hexadecyl-5-aminopyrimidinium-bromid ( 3,20 g) werden im Methanol/Wasser $^{50}/_{50}$ ($^{v}/_{v}$) bei 0°C im Eisbad mit 1 g $NaNO_2$ und 0,1 ml konzentrierter Bromwasserstoffsäure unter Stickstoff 6 Stunden gerührt. Danach wird das Gemisch auf Raumtemperatur gebracht und anschließend bei 80°C am Rückfluß für 2 Stunden unter Stickstoff und Rühren erhitzt. Das entstandene Hexadecylpyrimidinium-bromid wird mit 2-Ethoxyethanol extrahiert und bei 10°C zum Auskristallisieren gebracht. Ausbeute 30 %, Schmelzpunkt 105°C(Bromid)189°C (Chlorid).

Präparative Trennung von nichtumgesetzten Produkten kann auch durch Hochdruckflüssigkeitschromatographie erreicht werden, wie bei den Pyridiniumabkömmlingen beschrieben.

2.) In 2,5,6-Stellung substituierte Pyrimidiniumverbindungen werden durch Umsetzung in 2-Ethoxyethanol unter Druck im Autoklaven bei 100°C bei einer Reaktionsdauer von 8 Stunden aus den entsprechenden n-Alkylbromiden bzw. Jodiden und den substituierten Pyrimidinverbindungen mit Ausbeuten zwischen 30 und 40 % erhalten. Die Umkristallisationen werden für alle substituierten Pyrimidiniumverbindungen aus Chloroform vorgenommen.

Präparative Trennung von nicht umgesetzten Produkten kann wie vorne beschrieben durch Hochdruckflüssigkeitschromatographie erreicht werden.

3.) $N_1$-n-Alkyl-Verbindungen des Pyrimidins können durch Umsatz von n-Alkyl-Mgx(x = Br,Cl) in guten Ausbeuten mit Pyrimidin oder 2,6,5,6-substituierten Pyrimidinen in Gegenwart von 1,2-Dimethoxyethan und/oder n-Heptan erhalten werden. Es findet kein Hetarin oder Additions-Eliminations-oder Eliminations-Additions-Mechanismus statt.

0,01 Mol ( 1,0 g) 5-Fluor-pyrimidin werden in 1,2-Dimethoxymethan (100 ml) unter Rühren im Dreihalskolben und unter Stickstoff gelöst. Aus einem Tropftrichter läßt man 0,08 Mol (gleiche Größenordnung wie oben) n-Decylmagnesiumchlorid (oder 0,09 Mol ≙ 29,6 g n-Hexadecylmagnesiumbromid), gelöst in 20 ml

Heptan bei 20°C langsam zutropfen. Diese Lösung wird auf 40°C gebracht, für 12 Stunden gerührt und nach abgeschlossener Reaktion werden aus einem Tropftrichter 20 ml 50 Gew.% Bromwasserstoffsäure bei konstanter Temperatur zugetropft. Nach 1 Stunde ist das überschüssige Grignard-Reagenz umgesetzt. Es wird auf 0°C abgekühlt und der evtl.noch bestehende Überschuß von Grignard-Reagenz durch Zusatz von Methanol ver nichtet und die quartären $N_1$-Pyrimidiniumbasen durch 2-Ethoxyethanol extrahiert. Die erste Umkristallisation wird aus Chloroform/Methanol bei 0°C durchgeführt, die weiteren Umkristallisationen bei Raumtemperatur.
Schmelzpunkt: 5-Fluor-$N_1$-decylpyrimidiniumbromid 199°C (Zers.)
Schmelzpunkt: 5-Fluor-hexadecylpyrimidiniumbromid 175°C (Zers.)

c) Herstellung der 7-n-Alkyl-imidazolium 4,5-d pyrimidinderivate (Purin), z.B. 7-Hexadecyl-imidazolium-2,6-dihydroxy[4,5-d] pyrimidinbromid

1,5 g 2,6-Dihydroxy-purin ( 0,01Mol) werden in 100 ml Aceton im Vierhalskolben bei 35°C gelöst. Aus zwei Tropftrichtern werden unter Rühren und Stickstoff einmal Triethyl-oxoniumborfluorid ($Et_3O^{\oplus}BF_4$) in dreifachem Überschuß ( 5,7 g = 0,03 Mol) gegenüber n-Hexadecylbromid ( 3,3 g, 0,01 Mol), das sich in dem zweiten Tropftrichter befindet, gleichzeitig mit n-Hexadecyl-Br zugetropft. Die Reaktion wird unter stetem Rühren über 6 Stunden bei 40°C gehalten und anschließend 10 Stunden bei 65°C am Rückfluß gekocht. Nach Abschluß der Reaktion setzt man 100 ml Ethanol zu, filtriert die gebildete quartäre Ammoniumbase über einen Glassintertiegel (1G4) ab und kristallisiert aus einem Gemisch bestehend aus 2-Ethoxyethanol/Chloroform,1:1 um . Ausbeute: 0,5 g, Schmelzpunkt: 122°C.
Die Verbindung ist hygroskopisch und bildet ein kristallines Adukt mit zwei Teilen Chloroform.
Die UV-Spektren zeigen die typischen Absorptionseigenschaften der Purinderivate. Desgleichen die $^1$H-NMR -Spektren, gemessen in $d_6$-$ME_2SO_4$.

d) Die entsprechenden Benzothiazole-und Benzimidazol-n-Alkyl-Verbindungen, vor allem wenn sie in er 2-Stellung halogeniert sind, bilden sich nach diesem Verfahren mit einer Ausbeute von 50 % und sind sehr leicht aus Chloroform umzukristallisieren.

e) Die entsprechenden quartären Salze des Pyrazols lassen sich ebenfalls nach dem Verfahren c) herstellen. Auch kann nach Verfahren b3) durch Einsatz von n-Hexylmagnesiumbromid bzw. n-Alkylmagnesiumchlorid arbeiten, da weder ein Additions-Eliminations-noch ein Eliminations-Additions-Mechanismus abläuft. Die 4-H-Pyrazoliumsalze mit $R = CH_3$, OH, H bilden sich mit hoher Ausbeute von 60 %.
Da der n-Alkylrest sowohl am $N_1$ wie auch am $N_2$ oder beides lokalisiert sein kann, ist es notwendig, das Reaktionsprodukt durch Hochdruckflüssigkeitschromatographie auf eine RP-18-Säule in einem Aceton/Acetonitril-Elutionsgemisch zu trennen wie vorne beschrieben. Dies ist auch notwendig, wenn das entsprechende n-Alkylbromid im Bombenrohr oder Autoklaven mit einem Pyrazolabkömmling bei 100°C in Gegenwart von Piperidin zur Reaktion gebracht werden. Das Verhältnis von Di-N-substituierten zu Mono-$N_2$-substituierten Pyrazoliumabkömmlingen verhält sich wie 1,5:1.

f) Die Imidazolium-Verbindungen, die $N_1$-substituierten wie auch die $N_1$, $N_2$-disubstituierten lassen sich wie die entsprechenden Pyridiniumverbindungen herstellen.
Zur Herstellung der $N_1$-substituierten Imidazolium-Verbindungen verfährt man wie unter b3) beschrieben. Die Ausbeuten liegen bei 30 %. Als geeignetes Reaktionsmedium empfiehlt sich Aceton.

g) Die Quartärnisation des Pyrazins am $N_4$, wenn in 2-Stellung substituiert ist, erfolgt mit 50%iger Ausbeute, Wenn in 2-Stellung z.B. ein Chlor oder eine Carboxamid (Carbamoyl-) Gruppe angesiedelt ist. Wenn gemäß Vorschrift b1) verfahren wird, erhält man Ausbeuten von 20-30 % je nach Größe des Alkylrestes. Verfährt man nach der bekannten Herstellung von Pyridiniumverbindungen (a) erhöhen sich die Ausbeuten auf 50 %.
Wie gewöhnlich und vorne ausgeführt bestimmt die $(CH_2)_x$-Kette mit X = 10-20 die Größe und die KMK in wässrigen Lösungen. Die gebildete Größe, Form und Molekulargewichtsverteilung der Micelle in wässriger Lösung bei pH <7,0 wird nach der Natur des Gegenions $Y^{\ominus}$ bestimmt.
Die kovalent gebundenen pharmazeutischen Wirkstoffe können z.B. auf 9-$\beta$-Arabino-1,4-adenin, 5-Fluorcytosin, Acaturidin, 6-Mercaptopurin oder Thioguanin ausgedehnt werden. Hierzu gehören auch die Nukleoside bzw. Nukleotide der Thymidin-Reihe, die das Wachstum von neoplastischen Tumoren hemmen, u.a. durch Inhibierung der DNS-Synthese. Auch die antiviralen Stoffe der 1,3,5-Triazine, z.B. das 2-Acetamido-4-morphino-1,3,5-Triazin, welches virustatische Eigenschaften gegen Herpes zoster besitzt, sind zu nennen.

Tabelle 2

Charakteristische Eigenschaften der $N^{\oplus}$-Tenside der allgemeinen Formel HET $N^{\oplus} \equiv (CH_2)_x-CH_3\ Y^{\ominus}$

| Nr. | HET $N^{\oplus} \equiv (CH_2)_x-CH_3$ | $Y^{\ominus}$ | Fp °C | Analyse (%) gefunden C | H | N | Y | KMK $\times 10^{-6}$ M |
|---|---|---|---|---|---|---|---|---|
| 1. | Hexadecyl-4-Hydroxy-pyridinium | Br · 1/2 $H_2O$ | 85 | 59,86 | 10,94 | 4,37 | 24,83 | 0,95 |
| 2. | Dodecyl-pyridinium | Cl · $H_2O$ | 73 | 71,46 | 11,20 | 4,90 | | 1,52 |
| 3. | 2-Hydroxy,6-amino-hexadecyl-pyrimidinium | Cl | 155 | 61,45 | 18,69 | 10,76 | 9,10 | 2,00 |
| 4. | Hexadecyl-pyrimidinium | Br | 105 | 62,02 | 10,09 | 7,25 | | 2,50 |
| 5. | 2,6-Dihydroxy,5-Fluor, hexadecyl-pyrimidinium | Cl · 2 $H_2O$ | 172 | 61,13 | 22,68 | 7,14 | 9,05 | 0,85 |
| 6. | 2-Hydroxy,5-methyl,6-amino-hexadecyl-pyrimidinium | Br | 192 | 56,18 | 16,67 | 9,36 | | 1,00 |
| 7. | Dodecyl-pyrimidinium | Cl | 85 | 64,79 | 13,78 | 9,45 | | 1,20 |
| 8. | 2,6-Dihydroxy-dodecyl-pyrimidinium | Br | 70 | 53,07 | 17,12 | 7,75 | 22,06 | 1,90 |
| 9. | 2-Carboxamid-4-hexadecyl-1,4-diazinium | Cl | 195 (Zers.) | 71,30 | 6,77 | 11,89 | | 0,30 |
| 10. | 7-Hexadecylimidazolium-2,6-dihydroxy[4,5-d]pyrimidin | Cl · 1 $H_2O$ | 112 | 60,80 | 17,13 | 13,51 | | 0,50 |

Fortsetzung Tabelle 2

| | | | | | | | | |
|---|---|---|---|---|---|---|---|---|
| 11. | 7-Hexadecylimidazolium-2,6-diamino-[4,5-d]pyrimidin | Br · 1/2 $H_2O$ | 170 (Zers.) | 55,17 | 8,93 | 18,41 | 17,49 | 1,30 |
| 12. | 3-Hexadecylbenzimidazolium | Cl · $H_2O$ | 100 | 72,53 | 10,80 | 7,35 | | 6,70 |
| 13. | 4-Methyl- -2-hexadecyl-pyrazolium | Cl | 172 | 69,67 | 11,89 | 8,14 | | 0,70 |
| 14. | 5-Methyl-1-hexadecylimidazolium | Cl | 142 | 69,69 | 11,89 | 8,12 | | 3,90 |
| 15. | 3-Hexadecylthiazolium | Br · 2 $H_2O$ | 155 | 58,20 | 17,83 | 3,59 | | 0,91 |
| 16. | 2,5-Dimethyl-3-hexadecyl-thiazolium | Br · 1 $H_2O$ | 170 (Zers.) | 57,15 | 20,50 | 3,34 | 19,01 | 15,00 |
| 17. | 3-Hexadecyl-6-methyl-benzimid-azolium | Cl · 2 $H_2O$ | 119 (Zers.) | 69,81 | 14,13 | 7,09 | | 17,00 |
| 18. | 3-Dodecyl-6-methyl-benzimid-azolium | Br · 1 $H_2O$ | 98 | 59,40 | 12,52 | 7,29 | | 7,30 |
| 19. | 3-Hexadecyl-5,6-dihydroxy-benzthiazolium | Cl · 2 $H_2O$ | 70 | 60,60 | 28,54 | 3,07 | 7,79 | 7,90 |
| 20. | 3-Dodecyl-benzthiazolium | Br · 1 $H_2O$ | 90 | 70,20 | 14,57 | 4,31 | | 10,90 |

## Tabelle 3

Ausbeuten und hydrodynamischer Radius von N-Tensiden der Formel HET $\equiv_{\ominus}N-(CH_2)_x-CH_3$ und Benzethonium Abkömmlinge in Abhängigkeit von $Y^{\ominus}$

| Nr. | Tensid | Gegenion $Y^{\ominus}$ | $<R_H>$ (Å) | Ausbeute (%) |
|---|---|---|---|---|
| 1 | N-Cetyl-4-methyl-imidazolinium | $Br^{\ominus}$ | 140,0 | |
| | | $Cl^{\ominus}$ | 70,0 | 60 |
| | | $NO_3^-$ | 20,0 | 70 |
| 2 | N-Hexadecyl-4-cetyl-imidazolinium | $Cl^{\ominus}$ | 100 | 40 |
| | | $HSO_4^-$ | 150 | 30 |
| 3 | N-Hexadecyl-5-carboxamid-pyridinium | $Br^{\ominus}$ | 120,0 | 60 |
| | | $Cl^{\ominus}$ | 55,0 | 60 |
| | | Fumarat | 70,0 | 70 |
| | | Maleat | 120,0 | 30 |
| 4 | 8-Ketohexadecylpyridinium | $Cl^{\ominus}$ | 50,5 | 00 |
| | | $Br^{\ominus}$ | 140,0 | 80 |
| | | $NO_3^-$ | 170,0 | 100 |
| 5 | Methyl-3-stearyloxy-propyl-pyridinium | $Cl^{\ominus}$ | 140,0 | 60 |
| | | Salizylat | 1000,0 | 60-80 (20,25°C) |
| 6 | Cetyl-2,3-dihydroxy-propyl-hexadecyl-pyridinium | $Cl^{\ominus}$ | 150,0 | 20 |
| | | $Br^{\ominus}$ | 180,4 | 25 |
| | | $OH^{\ominus}$ | 210,4 | 30 |
| | | Maleat | 120,0 | 41 |
| 7 | 3,5-bis [(n-hexadecyloxy-carbonyl]-N-methyl-pyridinium | Salizylat | 1000 | 60 |
| | | Fumarat | 2500 | 70 |
| | | $Cl^{\ominus}$ | 350 | 50 |
| 8 | a) 2,-4-Dihydroxy-5-methyl-hexadecyl-pyridinium | $Cl^{\ominus}$ | 1000 | 30 |
| | | $Br^{\ominus}$ | 1500 | 30 |
| | b) 2,-4-Dihydroxy-5-Fluor-hexadecyl-pyridinium | $Br^{\ominus}$ | 210 | 30 |
| | | $Cl^{\ominus}$ | 150 | 30 |
| 9 | a) 2-Carboxamid-3-hexadecyl-1,4-pyridinium | $Cl^{\ominus}$ | 220 | 30 |
| | | $NO_3^-$ | 440 | 30 |
| | b) 2-carboxamid-3-dodecyl-1,4-pyridinium | $NO_3^-$ | 366 | 30 |
| | | Fumarat | 750 | 30 |
| 10 | 3-[[(Dimethylamino)-carboxyl] oxyl]-1-hexadecyl-pyridinium | $Cl^{\ominus}$ | 450 | 30 |
| | | Fumarat | 700 | 60 |
| | | $Br^{\ominus}$ | 1000 | 40 |
| 11 | 3-hexadecyl-benzimidazo-linium | $Cl^{\ominus}$ | 300 | 50 |
| | | Maleat | 1500 | 40 |
| | | Fumarat | 250 | 30 |
| | | $NO_3^-$ | 500 | 70 |
| | | $SO_4^{2}$ | 350 | 70 |
| 12 | Benzyldimethyl[2-[2-(p-1,1,3,3,tetramethylbutyl-p,p'-dimethyl-phenoxy)ethoxy]ethyl]ammonium | $Cl^{\ominus}$ | 150 | 30 |
| | | $Br^{\ominus}$ | 3000 | 40 |
| | | $NO_3^{\ominus}$ | 150 | 10 |
| | | Maleat | 3000 | 20 |
| | | Fumarat | 2500 | 25 |
| | | Salizylat | 3000 | 20 |

Die folgende Abbildung 6 zeigt die Varianz des hydrodynamischen Radius von Benzethonium-Chlorid und N-Hexadecyl-4-cetyl-imidazolium-salicylat in Abhängigkeit des hydrodynamischen Radius nach verschiedenen ultraschallbehandelten Zeiten in Minuten, gemessen durch inelastische Laser-Lichtstreuung.

Weitere vorzugsweise Ausführungsformen der Erfindung:

Während der Gesamtbereich der kritischen Micellbildungskonzentration (KMK) im Bereich von 1,0 . $10^{-7}$ bis 1,5 $10^{-4}$ Mol/Liter liegt, liegt die KMK vorzugsweise im Bereich von 1,0 bis 8,5 . $10^{-7}$/Liter.

Vorzugsweise ist das kationische Tensid mit dem einwertigen Anion in einer Menge von 0,05 bis 0,1 Gew.%, bezogen auf die gesamte pharmazeutische Zubereitung, enthalten.

Besonders gute Ergebnisse werden erzielt, wenn das kationische Tensid mit dem einwertigen Anion in einer Menge von 0,08 - 0,1 Gew.%, bezogen auf die gesamte pharmazeutische Zubereitung, enthalten ist.

Vorzugsweise ist der hydrophobe pharmazeutische Wirkstoff in einer Menge von 0,06 - 0,05 Gew.%, bezogen auf die gesamte pharmazeutische Zubereitung, enthalten.

Besonders gute Ergebnisse werden erzielt, wenn der hydrophobe pharmazeutische Wirkstoff in einer Menge von 0,001 - 0,005 Gew.%, bezogen auf die gesamte pharmazeutische Zubereitung, enthalten ist.

Vorzugsweise Lösungsmittel sind Wasser oder Wasser + Glycerin oder Wasser + Glycerin + Ethanol.

Vorzugsweise ist das einwertige Anion ein monobasischer oder dibasischer Fettsäurerest.

Vorzugsweise ist das einwertige Anion Acetat, Propionat, Fumarat, Maleat, Succinat, Aspartat oder Glutamat.

Vorzugsweise ist das einwertige Anion ein Zuckerrest.

Vorzugsweise ist das einwertige Anion Glukonat, Galacturonat oder Alginat.

Vorzugsweise ist das einwertige Anion Chlorid, Bromid, Jodid oder Hydrogensulfat.

Vorzugsweise ist der hydrophobe pharmazeutische Wirkstoff ein antimikrobieller Wirkstoff oder ein antifungaler Wirkstoff oder ein antiproliferativer Wirkstoff oder ein antiviraler Wirkstoff.

Vorzugsweise ist der hydrophobe pharmazeutische Wirkstoff eine anorganische Verbindung der Elemente Zink oder Quecksilber oder Wolfram und/oder Antimon. Vorzugsweise ist dabei die anorganische Verbindung $Z_nSO_4$ oder $Z_nO$ oder $Hg(CN)_2$ oder $(NH_4)_{18}$ $(NaW_{21}$ $Sb_9O_{86})_{17}$ oder auch ein Alkali-oder Erdalkalisalz der Phosphonsäure $ROP(O)Me_2$ oder auch ein N-Phosphonoacetyl-1-aspartat.

Vorzugsweise ist der hydrophobe pharmazeutische Wirkstoff ein Antibiotikum oder ein antiviraler Wirkstoff oder ein antifungaler Wirkstoff oder ein antineoplastischer Wirkstoff.

Vorzugsweise ist das Lösungsmittel Wasser und/oder Ethanol und/oder Glycerin. Vorzugsweise ist das Lösungsmittel Wasser und/oder Ethanol und/oder Dimethylsulfoxid.

Während der pH-Wert des Lösungsmittels jedenfalls $\leq 7$ sein muß, ist der vorzugsweise pH-Wert des Lösungsmittels = 5 bzw. in der Nähe von 5.

Die pharmazeutische Zubereitung kann erfindungsgemäß im wesentlichen dadurch hergestellt werden, daß zunächst in einem Reaktionsgefäß das Lösungsmittel vorgelegt wird, dann das kationische Tensid unter Rühren bei Zimmertemperatur zugegeben wird, dann zur entstandenen isotropen micellaren Lösung der hydrophobe pharmazeutische Wirkstoff unter Rühren bei Zimmertemperatur zugegeben wird und zu dessen vollständiger Lösung weitergerührt wird.

Besonders günstige Ergebniss werden mit den kationischen Tensiden der allgemeinen Formel II erzielt, wenn x = 14 ist, d.h., wenn die Alkylkette 15 C-Atome aufweist.

Diese geradkettigen $C_{15}$-Abkömmlinge der N-Tenside zeichnen sich insbesondere aus durch eine einfache chemische Herstellung. Außerdem haben sie überraschenderweise die niedrigste KMK (diese liegt ca. bei 2,5 . $10^{-7}$ Mol/Liter). Sie sind weiterhin durch $Y^-$ sehr leicht zu steuern (Form, Molekulargewichtsverteilung, Polydispersität). Außerdem sind sie variabel aufgrund ihrer Größe der Alkylkette und daher bezüglich Aufnahme der pharmazeutischen Wirkstoffe. Schließlich zeichnen sie sich durch leichte Kristallisierbarkeit aus.

Wie bereits erwähnt, ist der Rest Hexadecylpyridinium an sich (als reine chemische Verbindung) bekannt. Nicht bekannt ist der erfindungsgemäße Einfluß des zugehörigen Anions ($Y^-$) auf die Micellengröße und die Form der Micelle. Im Hinblick auf den anmeldungsgemäß beanspruchten unabhängigen Stoffschutz für alle offenbarten neuen Verbindungen wird deshalb im folgenden ein Oberbegriff offenbart, der vorzugsweise erfindungsgemäße neue Verbindungen umfaßt. Dieser Oberbegriff lautet "isoelektronische heterozyklische Stickstoffbasen mit 5-oder 6-Ringen, enthaltend entweder 2 N-Atome in 1,2-Stellung bzw. 1,3-Stellung bzw. 1,4-Stellung oder ein S-Atom in 1-Stellung mit einem N-Atom in 3-Stellung".

Herstellungsverfahren für die pharmazeutische Zubereitung:

Generelles zur Herstellung der wässrigen Phase:

Um vorzugsweise eine monodisperse, homogene und isotrope wäßrige Lösung der $N^+$-Tenside sowohl in Hinsicht auf Form (sphärisch, oval, langgestreckt) und Größe als auch auf Molekulargewichtsverteilung zu erreichen, müssen die angeführten Lösungen einschließlich ihrer eingeschlossenen hydrophoben pharmazeutischen Wirkstoffe

a. ultraschallbehandelt werden, z.B. bei 100 Watt, eine Minute, eventuell anschließend dann durch b.

b. durch Säulenchromatographie, z.B. auf einer Agarose A 0,5 m, Sepharose 2 B, Sephadex G 200, DEAE-Sepharose C1-6B bei pH 6,0 oder auf einem Ultragel AcA44 (pH 6.0 - 6.5) oder BiO-Gel 1,5 m bei pH ≤7,0 nachgereinigt; oder

c. auf einem linearen Dichtegradienten, z.B. von 1 -30 Gew.% Subrose, in einer präparativen Ultrazentrifuge in einem SW-27-Rotor bei 25.000 UpM für 12 Stunden zentrifugiert werden. Bei Anwendung einer Zonal-Zentrifugation bei gleichem Gradienten (20°C) können bei 10,000 UpM große Mengen von homogenen Populationen von Micellen und Vesikeln zentrifugiert werden.

d. DEAE-Zellulose-Säulenchromatographie bei pH 5,0 -6,5 (pH≤7), z.B. durch einen Phosphatgradienten (linear von 0,01M $KH_2PO_4$/0,01M $K_2HPO_4$, pH 6,5 bis zu 0,05M $KH_2PO_4$/0,05M $K_2HPO_4$ im totalen Elutions-Volumen von 1000 ml) gereinigt werden, bis die entsprechende, gewünschte Population von Micellen bzw. Vesikeln erhalten worden ist.

So ist es möglich, die gewünschten homogenen Populationen von Micellen oder Visikeln einschließlich ihrer eingeschlossenen pharmazeutischen Wirkstoffe, in Form von wiederholbaren konstanten Molekulargewichten und geometrischen Formen zu erhalten. Dies ermöglicht Monomere der Tenside von den Micellen als auch von nicht eingeschlossenen pharmazeutischen Wirkstoffen quantitativ zu trennen.

Herstellung der homogenen, micellaren Lösung in wäßriger Phase:

Die wäßrige Phase kann reines Wasser sein. In der Regel wird jedoch eine wäßrige Lösung eines Elektrolyten gewählt. Z.B. kann eine wäßrige Lösung aus NaCl oder $CaCl_2$ ($MgCl_2$) verwendet werden. Zusätzlich können aktive pharmazeutische Wirkstoffe von genannter Art eingeführt werden, die dann micellar gelöst werden auch eventuell unter Beschallung.

Die meisten Verfahren sind auf eine Einkapselung hydrophiler Wirkstoffe beschränkt. Mit der vorliegenden Erfindung ist es möglich, hydrophobe z.B. lipophile anorganische $(Hg)CN)_2)$ und organische Wirkstoffe (Amphotericin B) micellar einzuschließen. Auch können hydrophile Anionen, die pharmazeutische Bedeutung haben, z.B. Salizylat, je nach Natur des N-Tensides (insbesondere der Formel II) an der externen Oberfläche der Micelle eingeschlossen werden.

Die Erfindung kann dazu verwendet werden, um entweder hydrophile oder lipophile Substanzen oder auch beide Substanzen einzuschließen. Im Falle von hydrophoben Wirkstoffen werden diese dann zusammen mit dem N-Tensid der Formel I und II in einem Glycerin/Ethanol-Gemisch, bestehend aus 15 Gew.% Glycerin, 15 Gew.% Ethanol und 70 Gew.% Wasser oder 50 Gew.% Ethanol und 50 Gew.% Wasser gelöst, eventuell geschüttelt bzw. ultraschall behandelt und anschließend auf die wäßrige Phase mit einem Gehalt von Glycerin/Ethanol von maximal 15 g Glycerin, 5 g Ethanol in 100 g Wasser verdünnt. Anschließende Gel-Permeationschromotographie oder präparative HPLC-können ungewünschtes Material entfernen und eine homogene, isotrope Lösung liefern. Während hydrophobe Substanzen vornehmlich über eine organische Phase (50 %) und anschließende Verdünnung (Wasser) hergestellt werden, werden hydrophile pharmazeutische Wirksubstanzen vorzugsweise in der wäßrigen Flüssigkeit eingesetzt, die zur Dispergierung der micellaren Lösung benutzt werden. Im Bedarfsfalle können jegliche nicht aufgenommene, aktive Wirkstoffe aus der Dispersion unter Anwendung bekannter Techniken, wie z.B. Dialysieren, Zentrifugieren, Gel-Permeationschromotographie entfernt werden.

Die Form und Größe, als auch der Hydratationsgrad der micellaren Lösungen der N-Tenside ist u.a. abhängig von $y^-$, weniger von der Struktur des Heterozyklus, wohl aber von der hydrophoben Kettenlänge $(CH_2)_x$ . So können z.B. in Gegenwart von $Br^-$ oder Salizylat$^-$ große stäbchenförmige Micellen von Hexadecylpyridinium erhalten werden von einer Größenordnung von L = 10.000 Å und einem Durchmesser von 100 - 500 Å, während man in Gegenwart von Chlorid Micellen der Größenordnung von 50 - 100 Å in wäßriger Lösung erhält. In diesem Falle gibt die Form und Größe der Micelle die Konzentration des zu verkapselnden (mi cellar) Wirkstoffes an und gestaltet sich somit umgekehrt wie bei Liposomen.

Der Vorteil der Erfindung gegenüber der Verkapselung bei Liposomen liegt

1. in der Dichtigkeit dieser N-Tenside, welche den micellar gebundenen pharmazeutischen Wirkstoff aufgrund der vorne aufgeführten Kräfte nicht freilassen kann und

2. der Steuerung der Form und Größe der Micellen durch $Y^-$, damit Steuerung der Aufnahmekapazität an hydrophoben bzw. hydrophilen Wirkstoffen, ohne großen, tiefgreifenden Einfluß des Heterozyklus auf die KMK.

Die erfolgte Bildung der kleinen und großen Micellen der N-Tenside in wäßriger Phase lassen sich anhand von physikalischen Meßmethoden nachweisen, z.B. mit gefriergetrockneten Proben ("freeze fracture") im Elektronenmikroskop oder durch Röntgenkleinwinkel-Streuung, durch dynamische Lichtstreuung, durch Kernresonanzspektroskopie ($^1H$, $^{13}C$ und $^{31}P$), als auch durch Transmissionselektronenmikroskopie. Abb. 2 und 3 zeigen z.B. elektronenmikroskopische Aufnahmen von micellar eingeschlossenem $Hg(CN)_2$ in Hexadecylpyridinium-und Benzethoniumchlorid.

Im Kernresonanzspektrum ergeben sich scharfe Signale mit schwacher Linienbreite, welche einen Hinweis auf die Bildung von Micellen mit einem Durchmesser kleiner als 600 Å liefert. Scharfe Signale bei $\delta$ ca. 0,89 ppm ($-CH_3$), $\delta$ ca. 1,28 ppm ($-CH_2-$) und $\delta$ ca. 3,23 ppm ($-N-(CH_3)_2$ sind z.B. für die Micellen der N-Tenside der allgemeinen Formel I und II charakteristisch. Für eingeschlossene Wirkstoffe in diesen Micellen der N-Tenside ist ein Methylsignal bei $\delta$ ca. 0,87 - 0,89 ppm charakteristisch, das jedoch in einem Triplett aufgespalten ist und eine wesentlich geringere Linienbreite hat als das Methylsignal, das als Singlett vorkommt bei $\delta$ = 0,89 ppm, welches allerdings nur von der Micelle herrührt.

Diese wäßrigen Phasen, welche die erfindungsgemäß erhaltenen Micellen mit eingeschlossenen Wirkstoffen enthalten, sind Verabreichungssysteme, die sich gegebenenfalls nach Konzentrierung, z.B. durch Ultrafiltration, Ultrazentrifugation oder Lyophilisieren mit anschließendem Auflösen in einer wäßrigen Phase, für die orale (p.o.) oder topische Verabreichung eignen.

Bei oraler Verabreichung können die micellar gebundenen pharmazeutischen Wirkstoffe der N-Tenside der wäßrigen Phase mit pharmazeutisch unbedenklichen Verdünnungsmitteln oder Trägern oder mit üblichen Zusätzen, z.B. Farbstoffen oder Geschmackstoffen, vermischt und als Sirup oder in Form von Kaseln verabreicht werden.

So besteht eine homogene isotrope micellare wäßrige Lösung vorzugsweise aus einem N-Tensid der Formel II und I mit einem antiviralen Wirkstoff, insbesondere mit $Hg(CN)_2$, oder $ZnSO_4$, ZnEDTA, Idoxuridin, 5-Ethyl-2'-desoxyuridin oder Trifluorthymidin, Amantadin, Rimantadin ($\alpha$-Methyladamantan) und Viderabin (9-$\beta$-Arabino <1,4>-adenin) und Ribavirin (1-$\beta$-D-Ribofuranosyl-1,2,4-triazole-3-carboxamid) als auch mit 2,6-Di-amini-kuban 1,1':3,3'-Bis-cyclobutan oder einfach substituierte 2,6-di-amino-Verbindungen ($CF_3$,Cl,$OCH_3$) in Gegenwart oder Abwesenheit von Glycerin/Ethanol dispergiert (20°C; Ionenstärke <0,2 M).

Eine homogene, isotrope micellare wäßrige Lösung besteht aus einem N-Tensid der Formel II und/oder Formel I vorzugsweise mit einem antifungalen Wirkstoff, vorzugsweise mt 5-Fluorcytosin, Clotrimazol, Econazol, Miconazol oder Oxyconazol (Z-Form) als auch mit Amphotericin B, Nystatin und ZnO.EDTA als anorganischer antifungaler Wirkstoff, sowie $Hg_2(CN)_4$ ($Hg(CN)_2$ liegt hier als Polymer vor, wobei das Dimere das zugrundeliegende Bauprinzip ist (in wäßriger Lösung) dispergiert.

Eine homogene, isotrope wäßrige Lösung besteht aus einem N-Tensid der Formel I und/oder der Formel II vorzugsweise mit einem antineoplastischen Wirkstoff, insbesondere 5-Fluorcyanid, $Hg(CN)_2.4$ (Ascorbat oder Acetylacetonat), Azauridin, Cytarabin, Azaribin, 6-Merkaptopurin, Desoxycoformycin, Azathioprin, Thioguanin, Vinblastin, Vincristin, Daunorubicin, Doxorubicin in Gegenwart oder Abwesenheit von Glycerin/Ethanol dispergiert.

Eine homogene, isotrope wäßrige Lösung besteht aus einem N-Tenside vornehmlich der Formel II oder der Formel I vorzugsweise mit Aminoglykoside wie z.B. Canamycin, Gentamycin, Neomycin etc. oder Tetrazyklinen, Chloramphenicol oder Erythromycin als bakteriostatische (grampositive) oder Clindamycin (gegen nicht sporenbildende anaerobe Bakterien) oder Rifampicin als bakteriziden, als auch Bacitracin, Tyrotricin und Polymycine in Gegenwart oder Abwesenheit von Glycerol/Ethanol dispergiert.

Die homogene Mischung kann auch anschließend in Gelen auf der Basis von Alginat, Hydrogelstrukturen wie z.B. Sephadex Agarose, Propyl-zellulose, Propyl-hydroxy-zellulose, in Gegenwart von DMSO, Glycerol dispergiert werden, wobei die pharmazeutischen Wirkstoffe micellar bei den gewünschten Konzentrationen enthalten sind.

Man dispergiert z.B. durch Schütteln, Rühren der wäßrigen Phase oder durch Ultraschallbehandlung, welche die zuvor hergestellte homogene isotrope Mischung enthält. Die Bildung der micellaren Strukturen mit den eingeschlossenen Wirkstoffen, pH $\leq$ 7,0, 20°C, findet spontan, d.h. ohne große zusätzliche Energiezufuhr von außen und mit großer Geschwindigkeit statt. Die Konzentration an N-Tensid der Formel I und II und Einschlußverbindung kann erhöht werden, wenn die KMK um mindestens das Zehnfache überschritten wird in der wäßrigen Phase bei konstantem chemischen Potential und Temperatur.

Die KMK ist eine variable Größe für die Menge der Monomeren der N-Tenside, welche man in einem bestimmten Volumen Wasser unter Verwendung von pH-Schwankungen ≤ 7,0 lösen kann. Die KMK, die erfindungsgemäß nicht sehr abhängig ist von der Natur des Gegenions, welches nur die Form bestimmt, da weit oberhalb der KMK gearbeitet wird, kann durch elektrochemische Verfahrenn (Leitfähigkeit, Potentiometrie) durch Messung der Überführungszellen im Zusammenhang mit den Gegenionen, der Oberflächenspannung, Dampfdruckerniedrigung, Gefrierpunkterniedrigung und osmotischer Druck, Messung der Dichte, des Brechungsindex, der elastischen und unelastischen Lichtstreuung (Diffusionskoeffizienten, Stokes' Radius) und der Viskosität, sowie durch Gelfiltration und Röntgenkleinwinkel-Streuungsmessungen bestimmt werden. Nanosekunden Fluoreszenz als auch die Messung der Fluoreszenspolarisation lassen zusätzlich Bestimmungen der Lage der eingeschlossenen pharmazeutischen Wirkstoffe durch die N-Tenside de Formel I und II zu, z.B. durch ZnEDTA oder $Hg(CN)_2$ als Quencher und Amphotericin B als Verstärker. Positronium-Vernichtungs-Messungen an diesen beschriebenen micellaren Lösungen mit den eingeschlossenen Wirkstoffen lassen ebenfalls Aussagen über die Menge (Konzentration) des eingeschlossenen pharmazeutischen Wirkstoffes in Abhängigkeit von der Natur und Konzentration von $y^-$ zu.

Wäßrige Phasen mit einem pH-Wert >7,0 werden nach der Dispersion zentrifugiert. Die Neutralisation auf pH ≤ 7,0 ist notwendig, um eine Zerstörung des Hetreozyklus in Formel II als auch des Wirkstoffes und/oder der Micellen unter basischen Bedingungen zu verhindern. Physiologisch gängige und verträgliche Säuren sind z.B. verdünnte wäßrige Mineralsäuren und Salzsäure, Schwefelsäure oder Phosphatsäure oder organische Säure, z.B. Niederalkansäuren wie Essigsäure oder Propionsäure. Meistens reagieren die wäßrigen Phasen der kationischen N-Tenside der Formel I und II sauer bis neutral, können aber auch durch Sörensen-Puffer oder organische Inertpuffer, wie z.B. HEPES, MOPS oder MES auf pH-Werte zwischen 3 und 7,0 genau eingestellt werden.

Herstellung der homogenen, micellaren Lösung in nichtwässrigen Phasen:

Die Auswahl der betreffenden Lösungsmittel ist von der Löslichkeit des betreffenden pharmazeutischen Wirkstoffes darin abhängig. Geeignete Lösungsmittel sind z.B. Methylenchlorid, Chloroform, Alkohole, z.B. Methanol, Ethanol und Propanol, Niederalkancarbonsäureester, (Essigsäure-Ethylester), Ether oder Mischungen dieser Lösungsmittel. Nach Herstellen der micellaren Lösung und Zugabe des pharmazeutischen Wirkstoffes, gelöst im organischen Lösungsmittel, wird das organische Lösungsmittel entweder durch die vorne erwähnten Verfahren a. - d. oder durch Abblasen mit Inertgas, z.B. Helium oder Stickstoff, entfernt.

Beispiel 1:

Man löst 10 mg Hexadecylpyridinium-chlorid in 100 ml einer Wasser/Ethanol-Mischung (85:15; w/w) bei 25°C unter Rühren und addiert 10 ml Glycerol. Der pH-Wert sollte um 6,5 sein, kann jedoch mit HCl auf diesen oder einen anderen pH-Wert (=7,0) eingestellt werden. Diese Lösung wird dann auf 20±0,01°C abgekühlt, anschließend ultraschall-behandelt (Bronson-Sonifier, Mass.,U.S.A.) für zwei Minuten bei 10 Watt. Die Bildung der Micellen wird durch Messung des Diffusionskoeffizienten mittels inelastischer Lichtstreuung bestimmt und dann nach der Beziehung

$$(1) \quad D^0_{20,W} = \frac{k_B \cdot T}{6\pi \eta_0 \cdot R_H}$$

$T$ = t°+273
$\eta_0$ = Viskosität des Lösungsmittels
$k_B$ = Boltzmann-Konstante
$D^0_{20,W}$ = Diffusionskonstante

der Stokes, Radius ($R_H$) berechnet. In Gegenwart von Cl⊖ als Y⊖ sollte er nicht größer als 50 Å, von Br⊖ nicht größer als 1000 Å sein. Zur Bildung von Mikroemulsionen von Micellen bestimmter Größe dispergiert man bei Raumtemperatur (20°C) einen filmartigen Rückstand, den man durch Eindampfen der oben genannten Lösung im Rotationsverdampfer erhält, in 1/10 des ursprünglichen Volumens durch 10-minütiges Schütteln. Man erhält eine leicht opalisierende, wässrige Lösung. Zum Einschluß eines pharmzeutischen Wirkstoffes, z.B. 5-Fluorurazil, Cytarabin oder Idoxuridin können, diese in Wasser schwerlöslichen Substanzen direkt, d. h. in fester Form oder als wässrige Suspension eingegeben werden. So gibt man z. B. Trifluoridin, 1.0 - 3.0 mg, bei 20°C unter Umrühren entweder als Mikroemulsion (Suspension) oder zur

wässrigen micellaren Lösung der quartären Ammoniumbase direkt zu. - Eine quantitative Dosierung dieser genannten Nukleosid-und Adeninnukleosid-Verbindungen kann auch durch Dialyse erreicht werden:

Die micellare Lösung der vorne genannten Konzentration (gepuffert, ungepuffert, pH ≅ 6,0, Ionenstärke variabel, T = 293°K) wird in ein Dialyseschlauch (Fa.Servant oder Pharmacia) gefüllt, verschlossen und unter stetem Rühren bei Raumtemperatur gegen eine eingestellte Lösung von pH ≤7,0 die das vorne genannte Pyridin-oder/und Adenin-Nukleosid bestimmter Konzentration enthält, für 2 Std.dialysiert. Die Abnahme der Extinktion bei 260 nm mit der Zeit der Dialyse erlaubt die Kontrolle des micellaren Einbaues der vorne genannten Wirkstoffe in den hydrophoben Kern des Hexadecylpyridiniumchlorides (Tab.1).

Tabelle 1: (20°C, pH 5,5)

| Experiment | $R_H$ (Å) (±5,0Å) | Konzentration Trifluoruridine mg/100 ml | Idoxuridin mg/100 ml | Ausbeute (%) | |
|---|---|---|---|---|---|
| 1 | 45,0 | 5 | 7,5 | 95 | 95 |
| 2 | 45,0 | 7,5 | 10,5 | 95 | 98 |
| 3 | 50,5 | 10,0 | 12,5 | 94 | 98 |
| 4 | 60,0 | 12,0 | 15,0 | 96 | 98 |
| 5 | 60,0 | 15,0 | 17,0 | 96 | 97 |
| 6 | 65,0 | 17,0 | 20,0 | 96 | 96 |
| 7 | 71,5 | 20,0 | 21,5 | 100 | 98 |
| 8 | 75,0 | 25,0 | 23,0 | 100 | 100 |
| 9 | 75,0 | 30,0 | 24,0 | 100 | 100 |
| 10 | 78,0 | 50,0 | 30,0 | 100 | 100 |

Die erfolgte Bildung von kleinen micellaren Gebilden in der vorne genannten Lösung ist im NMR-Spektrum durch die Signale $\delta$ = 1,25 (Methylen), $\delta$ = 0,86 (Methyl) erkennbar. Durch Inkorporation der vorne genannten pharmazeutischen Wirkstoffe findet je nach Absättigung im hydrophoben Kern eine Verschiebung von $\delta$=1,25(Methylen) statt, jedoch nicht von $\delta$ = 0,86 (Methyl).

Die Größe der Micellen kann durch inelastische Lichtstreuung nach Formel (1) leicht bestimmt werden (Tabelle 1). Die Größe, einschließlich der Form und zur Erhaltung einer homogenen und monodispersen Lösung kann auch durch HPLC-Chromatographie, Gelpermeation-und Agarose-Chromatographie erreicht werden. (Abb. 7)

Eine Konzentration der so hergestellten Micellen kann durch Druckdialyse erreicht werden mittels Fiberglas-Patronen definierter Porengröße. So ist es möglich, nicht nur eine definierte Konzentration an pharmazeutischem Wirkstoff vorzunehmen, sonder auch die Micellengröße, Aggregationsrate, Hydratation (Solvatation) konstant zu halten, da keine Fusion der Micellen ("intermicellar growth") eintritt. D.h. die Zahl der Micellen pro Volumeneinheit mit ihrem eingeschlossenen pharmazeutischen Wirkstoff nimmt zu (Konzentration hydrodynamischer Partikeln mit gleichem Molekulargewicht), nicht die Aggregationsrate, noch die Zahl der Eventuell vorliegenden Monomeren, die durch Ultrafiltration abgetrennt werden.



BAD ORIGINAL

Beispiel 2:

Analog Beispiel 1 löst man pro Versuch 15 mg Benzethoniumchlorid in 150 g Wasser/Ethanol (85/15; w/w) bei 25°C unter Rühren und addiert 0,5 ml Glycerin. Der pH-Wert ist normalerweise zwischen 4,8 und 5,5. Um eine klare, nicht opalisierende Lösung zu erhalten, wird diese Lösung bei 25°C für zwei Minuten bei 20 Watt ultraschallbehandelt. Die Bildung der Micellen definierter Größe ist nach Abkühlen auf 20°C nach fünf Minuten abgeschlossen. Zur Inkorporation der vorne genannten antiviralen Wirkstoffe, z.B. Trifluoruridin, Idoxuridin, kann wie unter Beispiel 1 verfahren werden.

Zum Einschluß von Miconazol (Z-Form) dispergiert man die so hergestellte micellare Lösung in Gegenwart von Miconazol bestimmter Konzentration ultraschallbehandelt (2 Minuten), dann über Agarose chromatographiert, wobei die Micellen mit dem hydrophob eingeschlossenen Z-Miconazol als einheitlicher, monodisperser Peak eluiert werden kann. Die Größe und Konzentration an Wirkstoff kann durch inelastische Lichtstreuung und UV-spektroskopisch bestimmt werden. (Abb. 8)

Analog zum Beispiel 1 kann man 10 mg Benzethoniumchlorid und eine gewünschte Konzentration Z-Miconazol in je 5 ml einer Chloroform Methanol-(3:1)-Mischung lösen, dann konzentrieren durch "hollow fiber pressure dialysis" und anschließend in Wasser oder gewünschtem Puffer dispergieren. Man erhält eine klare wässrige Lösung, welche an Micellen der Größenordnung von $R_H$ = 60-80 Å in Gegenwart von $Cl^{\ominus}$ oder $R_H$ = 100-1000 Å in Gegenwart von Salizylat mit eingeschlossenem Wirkstoff besteht.

Durch Zugabe von 1 % (g/g) Alginat und/oder 5 % (g/g) Dimethylsulfoxid können auch tixotrope Gele mit den vorne genannten eingeschlossenen Wirkstoffen hergestellt werden. Durch Erhöhung der Benzethoniumchlorid-Konzentration, einschließlich der eingeschlossenen Wirkstoffe, bis zu 2 % (g/g) können auch wirksame Öle hergestellt werden.

Beispiel 3:

Analog zu Beispiel 1 und 2 können die Gegenionen $Y^{\ominus}$ = $Cl^{\ominus}$, $Br^{\ominus}$ etc. nach verfahrensgemäßer Herstellung durch Ionenaustauscher Chromatographie an DEAE-Sephadex A 50 oder DEAE-Sepharose oder durch umdialysieren gegen das betreffende bzw. gewünschte Gegenion $Y^{\ominus}$ ausgetauscht werden.

a) eine nach Beispiel 1 und 2 hergestellte wässrige micellare Lösung wird auf pH = 7,0 mit 0,01 N NaOH gebracht (20°C). Dies kann entweder durch Titration oder Dialyse gegen 0,01 N NaOH über 10 Std. geschehen. Anschließend erfolgt eine Dialyse gegen 1N Fumarat oder Maleat-Lösung, wobei hier die Na-Salze der Fumar-bzw. Maleinsäure eingesetzt werden können. Die Dialyse ist nach 12 Std. beendet. Ein Verlust an antiviralen Wirkstoffen, die vorne genannt sind, tritt nicht auf.

b) eine nach Beispiel 1 und 2 hergestellte wässrige micellare Lösung, pH 6,0, wird auf einer DEAE-Sephadex A 50 (1,0×100 cm)-Säule, die zuvor mit einer gepufferten (0,01 M $K_2HPO_4$-Puffer) 0,1 N Salizylatlösung beladen wurde, mit einer Fließgeschwindigkeit von 10 ml/30 min eluiert (20°C). Das überschüssige Salizylat wird durch Dialyse gegen einen großen Überschuß Wasser/Ethanol/Glycerol (90/5/5; g/g) von dem Säuleneluat beseitigt. Die DEAE-Sephadex A 50 Chromatographie kann auch unter Druck im Gegenstromverfahren mit dem gleichen Lösungsmittelsystem durchgeführt werden. Es resultiert bei der Austauscher-Chromatographie (DEAE-Sephadex A 50, DEAE-Sepharose 2B, 5B, DEAE-Cellulose, hügelig) ein homogener Peak, der nach den Kriterien, die in Beispiel 1 und 2 aufgezeigt worden sind, analysiert werden können. DEAE-Sephadex und DEAE-Sepharose haben den Vorteil, daß erhebliche Mengen an micellaren quartären Ammoniumbasen sowohl gereinigt als auch auf mono-Dispersität geprüft werden können.

Beispiel 4:

Analog zu Beispiel 1 wird eine micellare Lösung von Hexadecylpyridinium-chlorid mit folgenden pharmazeutischen Wirkstoffen hergestellt:

100 g Lösung enthalten:

| | |
|---|---|
| Hexadecylpyridinium-chlorid | 0,10 g |
| Atropin-Hydrochlorid (±) | 0,002 g |
| Zink-II-chlorid | 0,004 g |

Glycerol 10,0 g
Ethanol 4,894 g
Wasser 85,0 g
pH 6,2

Diese Präparation hat einen hydrodynamischen Radius von 35,0±5,0 Å und eine Aggregationsrate von N = 35, bei einem Molekulargewicht des Monomeren von Hexadecylpyridinium-chlorid von 393,0. Jede Micelle dieses Durchmessers enthält im Durchschnitt 100 µg Zink und/oder 50 µg Atropin (-).

Die Abbildung 9 zeigt die Varianz im hydrodynamischen Radius, R $_H$, dieser Präparation. Außerdem zeigt es die vorne beschriebene erfindungsgemäße Auftrennung des Racemats Atropin in die optische Antipoden z.B. Hyocyamin (-). Die micellare Größenverteilung wird durch ZnII-chlorid nicht verändert.

Die Abbildung 10 zeigt die Varianz im hydrodynamischen Radius,$R_H$, der N-Hexadecyl-4-methyl-pyridinium-chlorid und N-Hexadecyl-4-methyl-pyridinium-chlorid + Atropin-HCL.

Beispiel 5:

Man löst 5 mg 4-(17-Tritriacontyl)-N-methyl-pyridinium-chlorid, 1-2,0 mg Amphotericin B in 10 ml einer Chloroform/Methanol-Mischung (2:1) unter Stickstoff bei 25°C auf und dampft diese Lösung im Rotationsverdampfer ein. Der filmartige Rückstand wird in 5 ml destilliertem Wasser fünf bis zehn Minuten geschüttelt. Diese Lösung wird anschließend für drei Minuten ultraschallbehandelt bis sie nicht mehr opaleszierend ist. Man kann diese, je nach Bedarf, anschließend durch Zugabe von 0,5 ml eines fünffachen Konzentrates von Phosphat-gepufferter, isotonische Kochsalzlösung auf den pH-Wert von 5,5-6,5, bringen.

Diese so hergestellte Lösung wird in eine gerührte Ultrafiltrationscelle (z.B. Amicon $^R$) eingefüllt, welche anstatt des Ultrafilters mit einem geradporigen Filter mit einem Porendurchmesser von 0,05 µm versehen ist, in Abwesenheit von $Me^{2+}$-Ionen ($Me^{2+} = Ca^{2+}, Mg^{2+}$,) so filtriert, daß das Volumen in der Zelle nicht unter 30 ml absinkt. Hierdurch werden Vesikel einheitlicher Größe von < 50.000 Å hergestellt.

Form, Größe und Molekulargewichtsverteilung können wie im Beispiel 1 und 2 ermittelt werden. Das Pyridinium-Amphiphile wird aus den entsprechenden Jodiden mit Silberchlorid in 10 % ($^v$/v) Ethanol-Wasser hergestellt. Die farblosen Kristalle haben einen $F_p$ = 64°C (aus Aceton umkristallisiert) und kristallisieren mit einem Molekül Wasser.

$^1$H-NMR ($CDCl_3/Me_4Si$): δ 0,93, (6H,t,J~4Hz), 1,28 (60 H,m), 2,8 (1H,q,J<2Hz, nicht aufgelöst), 4,75 (3H,s), 7,7-9,5 (4H,m). Charakteristisch ist ein $H_2O$-abhängiges Signal bei δ 4,4.

Anal: Calc. für $C_{39}H_{74}NCl \bullet H_2O$ (MW 610,50) C, 76,72; H, 12,55; Cl, 5,81; gefunden: C 76,53, H, 12, 42; Cl, 5,78.

Beispiel 6:

Analog zu Beispiel 5 werden 10 mg 3,5-bis [(n-hexadecyloxy) carbonyl] -N-methyl-pyridiniumchlorid ($F_p$=102-102,5°) mit 2,0 mg Amantidin oder Rimantadin in 10 ml einer Ethanol/Wasser-Mischung (1:2) unter Stickstoff bei 20°C gelöst. Nach Ultraschallbehandlung (5 min, 20°C, 10 Watt) können die gebildeten Vesikel mit ihren eingeschlossenen Wirkstoffen Amantidin oder Rimantadin auf einer Sepharose 2B nach Größe und Molekulargewicht getrennt werden, um eine homodisperse Lösung von Vesikeln mit geringer Molekular-Polydispersität zu erhalten. Im $^1$H-NMR-spektrum sieht man die deutlichen Signale der Methylen (δ=1,28) und Methyl-Protonen (δ=0,86).

Diese in Beispiel 5 und 6 gebildeten unilamellaren Vesikeln können im Elektronenmikroskop sichtbar gemacht werden. Dazu wird die Vesikel-Dispersion zunächst der "freeze-fracture"-Methode unterzogen. Auch kann durch "negative staining" mittels der zwei Tropfen-Methode auf Formvar oder Kohlegrids durchgeführt werden. Durch diese beiden Techniken ist es zusätzlich möglich, eventuelle Populationen von Vesikeln sichtbar zu machen.

Auch die unter Beispiel 1 und 2 angewendete Methode der inelastischen Lichtstreuung gestattet es, Form und Größe dieser Vesikel und ihrer eingeschlossenen pharmazeutischen Wirkstoff zu bestimmen (Abb. 11).

3,5-Bis [(n-hexadecyloxy)carbonyl]-N-methylpyridiniumchlorid, F $_p$=102,0-102,5° (Aceton). $^1$H-NMR ($CDCl_3/Me_4Si$): δ 0,85 (6H,t,J 5Hz), 1,30 (56H,m), 4,40(4H,t,J<7Hz), 5,03 (3H,s) 9,20(1H,t,J<2Hz), 10,00-(2H,d,J<2Hz).

Analyt. Calc.: $C_{40}H_{72}NO_4Cl$(MW 666,47):C72,10, H10,88, C15,32; gef. C 71,44, H 10,84, Cl 5,23.

Beispiel 7:

Man löst 3 ml Gentamycin analog zu Beispiel 1 und 2 oder in eines in der Tabelle 3 genannten Tensides der quartären Ammoniumbasen in 1 ml einer Chloroform/Methanol-Mischung (3:1) auf und dampft diese Lösung bis zu einem dünnen Film ein. Dieser wird dann in 10 ml Wasser dispergiert. Anschließend kann man die Lösung auf den gewünschten pH > 3 < 6,5 mit Puffer einstellen. Man erhält eine klare Lösung.

Diese klare Lösung enthält je nach Verwendung des Tensides gemäß Tabelle 3 eine monodisperse Verteilung von mit Gentamycin beladenen Micellen in der gewünschten Größenordnung und Ausbeute (Abb. 12).

Beispiel 8:

Eine micellare Lösung von Hexadecylpyridinium-chlorid (Cetylpyridinium) wird analog zu Beispiel 1 (20°C) bereitet und enthält die folgenden Wirkstoffe:

100 g Lösung enthalten:

Cetylpyridiniumchlorid 0,10 g
Atropin-Hydrochlorid (I) 0,004 g
Quecksilber II-cyanid 0,004 g
Glycerin 10,892 g
Ethanol 5,0 g
Wasser 84,0 g
pH, T = 293°K 5,7

Diese Präparation hat erfindungsgemäß einen hydrodynamischen Radius von 35,0±10,0 Å und eine Aggregationszahl, n, von 35 bei einem Molekulargewicht des Monomeren von Cetylpyridiniumchlorid von 393,0. Jede Micelle von diesem Durchmesser enthält im Durchschnitt 5 µg $Hg(CN)_2$ und/oder ~ 5,0 µg Atropin (-) (Abb. 14).

Diese Präparation ist eine homogene Lösung, die Micellen der Größenordnung von 30-50 Å ($R_H$) enthält. Sie inhibiert das Wachstum von Influenza A Virus wie die nachfolgende Tabelle 6 zeigt (Abb. 13).

Tabelle 6

| Inhibitor[a)] | Titration der Infektion[b)], Plaque forming units | Inhibition[c)] |
|---|---|---|
| 1-Adamantanamin-HCl | $2x10^5$ | -1,11 |
| wässrige $Hg(CN)_2$-Lösung | $1x10^6$ | -1,30 |
| Cetylpyridinium-chlorid | $1,5x10^3$ | -0,11 |
| Präparation nach Beispiel 8 | $2x10^5$ | -1,45 |
| Kontrolle | $2x10^8$ | - |

Beispiel 8 - Blatt 2 -

a) Inhibitor-Konzentrationen werden in den in vitro Zellkulturen von 100 µM zugegeben.

b) Plaque-Assay wurde nach K. Tobita, A. Suginire, C. Enamote und M. Fusiyama, Med. Microbiol. Immunol., 162, 9 (1975) an Nierenepithelien (Hund,MDCK) in Gegenwart von Trypsin durchgeführt.



BAD ORIGINAL

c) Die Inhibition ist angegeben als der negative dekadische Logerithmus des Quotienten der "Plaque forming units" in Gegenwart des Inhibitors zu der ohne Inhibitor: $^{10}$log (pfu/ml des Inhibitors / (pfu/ml Kontrolle).

Die Abbildung 7 zeigt die Varianz im hydrodynamischen Radius, $R_H$, dieser Präparation. Außerdem zeigt es die vorne erfindungsgemäß beschriebene Auftrennung des Atropins in seine optimale Antipoden in Gegenwart von $Hg(CN)_2$.

Beispiel 9:

Man löst 5 mg einer in der Tabelle 3 (vornehmlich Nr. 1,2 oder 4) angegebenen N-quartären Ammoniumbase und 2,0 mg 5-Fluorurazil oder 1,5 mg 5-Fluordesoxyuridin in 10 ml einer Chloroform/Methanol/Ether (Mischung (3/1/1) und dispergiert diese Mikro-Emulsion durch zweistündiges, heftiges Schütteln bei 25°C. Zur Weiterverarbeitung ergeben sich zwei Wege:

a) Die Suspension wird zu einem dünnen Film eingedampft (unter $N_2$ und UV-Schutz). Der filmartige Rückstand wird dann in Wasser oder Puffer, z.B. 0,01 M bis 0,001 M $KH_2PO_4$ auf pH 4,5-6,5 eingestellt bzw. dispergiert. Anschließend trennt man die klare, micellare Lösung, nachdem man vorher zur Erhöhung der Ausbeute dieser z.T. opaleszierenden Lösung ultraschallbehandelt hat (10 Watt, 2 min), auf einer Bonderpak I-250 oder einer RP 18-Säule durch Hochdruck-Flüssigkeitschromatographie (HPLC) von eventuell vorhandenen Monomeren und nicht eingeschlossenen pharmazeutischen Wirkstoffen. Es wird in einer gerührten Ultrafiltrationszelle (Amicon$^R$) mit einem Filter aus Polycarbonat mit einem Porendurchmesser von 0,015 µm konzentriert.

b) In dieser Suspension werden bei 25°C 10 % ($^g$/g) Dimethylsulfoxid (DMSO) und 2,5 % ($^g$/g) Alginat eingerührt. Das gebildete Gel bildet sich spontan. Im Röntgenkleinwinkeldiagramm wird ein Einheitsabstand von d = 125 Å gefunden, der sehr verschieden ist von Alginatgelen (d = 25,45 Å). Das Gel hat tixotrophe Eigenschaften und wird bei 45°C flüssig. Die Rückbildung des Gels erfolgt bei 42°C und erreicht nach 2 Std. seine konstanten rheologischen Parameter bei 20°C bzw. 37°C.

Die endgültigen Konzentrationen pro 100 g pharmazeutischer Zubereitung ergeben sich wie folgt:

a) 100 g Lösung enthalten:

$N^+$-Tensid (Tabelle 3, Nr. 4) 0,01 g
5-Fluordesoxyuridin 0,10 g
Glycerin 11,89 g
Wasser 88,00 g
T = 293°K, pH=5,5

b) $N^+$-Tensid (Tabelle 3, Nr. 2) 0,05 g
5-Fluordesoxyuridin 0,05 g
Dimethylsulfoxid 10,00 g
Alginat 2,50 g
Wasser 86,50 g
T = 293°K, pH = 5,5

Beispiel 10:

Man löst 15 mg (0,02 mMol) Benzethoniumchlorid, 2 mg 2-Acetamido-4-morpholino-1,3,5-Triacin in 30 ml einer Wasser/Ethanol-Mischung (80:20 oder 90:10) bei 20°C unter Ultraschallbehandlung in 0,01 M $K_2HPO_4$, pH 6,5 unter $N_2$-Strom auf. Man erhält eine opalisierende, wässrige Phase. Durch Abtrennen der "umgekehrten Micellen" (reversed micelle) von den Micellen in wässriger Phase auf einer Sepharose 2B-Säule (1,5×100 cm) erhält man eine einheitliche, monodisperse Micellen-Formation mit einem durchschnittlichen hydrodynamischen Radius von 50 Å. Die chromatographierte Lösung kann wie in Beispiel 9 durch eine Ultrafiltration konzentriert werden. Die Lösung wird stabilisiert durch Einsatz von 5 % ($^w$/w) Glycerin oder durch Zusatz von 2 % ($^w$/w) Salicylat. Diese so hergestellten Lösungen verändern ihren hydrodynamischen Radius, ihr partiell spezifisches Volumen und Molekulargewichtsverteilung im Temperaturbereich von 15-45°C nicht.

100 g Lösung enthalten:
Benzethoniumchlorid 0,15 g
2-Acetamido-4-morpholino-1,3,5-triazin 0,006 g
Salicylsäure 0,05 g
Glycerin 5,00 g
Wasser 94,894 g
T = 239°K, pH = 5,5

Beispiel 11:

Man löst 30 mg (0,020 mMol) 3,5-bis[(n-Hexadecyloxy)carbonyl]-N-methyl-pyridiniumchlorid und 1,0 mg (~0,005 mMol) Polyoxin A in 10 ml 0,01 M $KH_2PO_4$, pH 6,5, bei 20°C, das 1 ml einer Mischung von tert. Butanol/Methanol/Ethanol (2:1:1) enthält. Die Lösung wird ultraschallbehandelt (20 Watt, 5 min) im Eisbad bei 0°C und anschließend auf 20 ml mit Phosphatpuffer, pH 7,0, aufgefüllt. Die klare, nicht opaleszierende Lösung wird auf einer Sepharose 2B-Säule bei pH 7,0 in Gegenwart von Phosphat bei Raumtemperatur chromatographiert. Die mit dem pharmazeutischen Wirkstoff dotierten Vesikel werden in einer Ultrafiltrationszelle (Amicon$^R$) mit einem Porendurchmesser von 0,05 µm unter geringem Überdruck konzentriert. Nach Durchtritt von 0,3-0,5 ml Filtrat sind alle Vesikeln mit Durchmesser 350 Å abgetrennt und die überstehende Dispersion kann ampulliert und für Behandlungsversuche eingesetzt werden. Abb. 15 zeigt die Inhibierung der Chitinsynthetase bei Digitonin-behandelten Zellen (Sacchamyces cerivisiae und Candida albicans) nach Zusatz dieser Präparation in Abhängigkeit von der Polyoxin A-Konzentration Abb.16: Bild des "Freeze-Etch".

Beispiel 12:

Analog zu Beispiel 2 löst man 10 mg $Hg(CN)_2$ und 40 mg Na-Ascorbat bei pH 7,0 in 10 ml Phosphatpuffer auf. Die Suspension wird bei 0°C 5 min ultraschallbehandelt, langsam auf 20°C erwärmt und auf einen 10 %igen ($^w/w$) linearen Glycerolgradienten in einer präparativen Ultrazentrifuge bei 1000$\times$g über 6 Std. zentrifugiert (20°C, Polyolomer-Röhren). Nach dem Austropfen werden die UV-aktiven Frakturen zusammengefaßt in einer Amicon-Flowzelle konzentriert und anschliessend auf Hg, Ascorbat analysiert (HPLC; Fließmittel $CH_3OH/H_2O$ (50/50) Hg-Detektion mit Na-diethylthiocarbamat, Hexadecyl-pyridinium-Cl, z.B. durch UV-Detektion bei 251 nm; N Ascorbat durch UV-Detektion bei R = 245 nm bei pH 2,0 und R = 265 nm bei pH 7,0). Diese micellar eingeschlossenen $Hg(CN)_2$-Ascorbat Komplexe (MW≈1.500) haben gemäß Tabelle 5 folgende representativen Inhibitor-Konzentrationen gegenüber B. subtilis DNA-Polymerase III.

Tabelle 5

| Nr. | Tensid | $Hg(CN)_2$ $K_i$, µM | $Hg(CN)_2$· Ascorbat $K_i$, µM | kompetitiv mit: |
|---|---|---|---|---|
| 1 | Hexadecyl-pyridinium-$Cl^{\ominus}$ | 7,9 | 15,3 | dGTP |
| 2 | Hexadecyl-pyridinium-$Cl^{\ominus}$ | | | dGTP |
| 3 | Benzethonium-Cl | 33,1 | 12,0 | dATP |
| 4 | Benzethonium-Cl | | | dATP |
| 5 | 8-Keto-hexadecyl-pyridinium-Cl | 0,4 | 0,05 | dGTP |
| 6 | 8-Keto-hexadecyl-pyridinium-Cl | 2,5 | 7,5 | dGTP |
| 7 | 3,5-bis (n-hexadecyloxy-carbonyl -N-methyl-pyridinium-Cl | 2,0 | 9,2 | dGTP |
| 8 | 4-(17-tritriacontyl)-N-methyl-pyridinium-Cl | 4 | 10,1 | dGTP |
| 9 | nach Tabelle 3 Nr. 9 | 9 | 0,5 | dGTP |
| 10 | nach Tabelle 3 Nr. 10 | 0,1 | 7,9 | dATP |

Die Inhibitor-Konzentrationen sind in 50 % der vollständigen Inhibierung angegeben. Der Assay, der verwandt wurde ist der gemäß von Clements, J; D'Ambrosio,J; Brown, N.C.; J.Biol.Chem. (1975) 250, 522 und Wright, G.E.; Brown, N.C.; Biochem.Biophys. Acta (1976) 432, 37.

Pharmakodynamische Versuche :

Die Bedeutung hochreaktiver Sauerstoffmoleküle (Superoxidradikale $O_2$, Peroxide $H_2O_2$, Hydroxilradikale ·OH, Singulettsauerstoff $^1O_2$) im entzündlichen Prozess ist bekannt (s. z.B. McCord, J.M, K. Wong: Phagocytosis-produced free radicales: roles in cytotoxicity and inflammation. In: Oxygen Free Radicales and Tissue Damage, Excepter Medica, Amsterdam-Oxford-New York, 1979, 343-360; Allgemeine und spezielle Pharmakologie, Herg. W. Forth, D. Henschler, W. Rummel, Biowissenschaftlicher Verlag, 1983 ). Sie entstehen u.a. bei der Phagocytosen durch aktivierte Leukoxyten (Monozyten, Makrophagen, polymorphkernige, neutrophile Granulozyten) und können zur Abtötung körperfremder Zellen, wie Bakterien, Bazillen u.a. auch bei bestimmten Viren, wenn das Immunsystem und die für IgG bzw. dem Komplementanteil $C_3$ spezifische Rezeptoren der Phagozyten funktionstüchtig sind, dienen. Die phagozytierenden Zellen selbst werden durch ein System, bestehend aus mehreren Enzymsystemen vor Schädigung durch diese besonders aktiven Formen des Sauerstoffs intrazellulär geschützt.

Es wurde nun gefunden, daß quartäre Ammoniumbasen der allgemeinen Formeln I und II



BAD ORIGINAL

I.

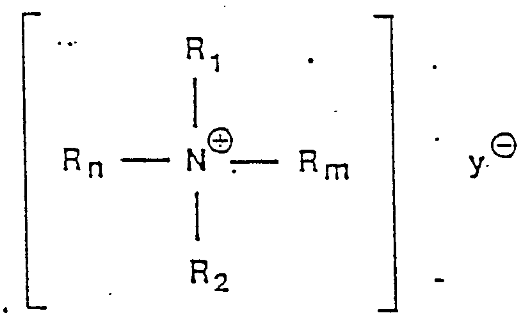

II.

$$\left[ HET \equiv N^{\oplus} - (CH_2)_x - CH_3 \right] \; y^{\ominus}$$

wobei Y⊖ ein Gegenion sowohl anorganischer, z.B. Cl⊖, Br⊖, $H_2PO_4^-$ oder organischer Natur, z.B. Fumarat, Malat, Salizylat, Acetat, Propionat, Gluconat und Alginat sein kann, der Heterocyclus sowohl ein Pyridin, Pyrimidin, Pyrazin, Imidazol, Thiazol oder Purin - aber ein π-Überschuß bzw. π-Mangel Aromatensystem - sein kann, alle in der Lase sind, bei pH ≤ 7,0 diese Sauerstoffradikale gemäß dem nachfolgenden Reaktionsmechanismus zu eliminieren:

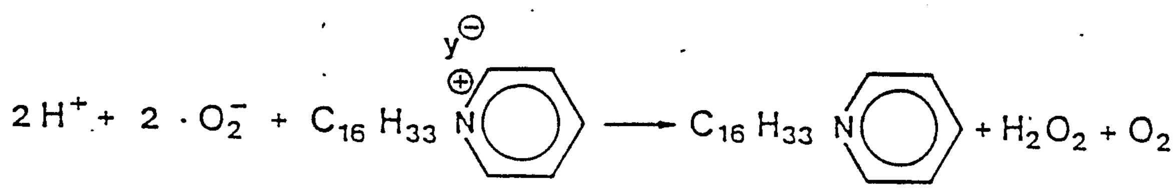

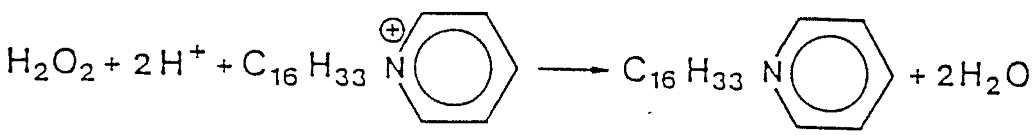

$$H_2O_2 + \cdot O_2^- \xrightarrow[pH \leq 6,0]{CP\ CL} 2\,OH^{\ominus} + O_2$$

$$\cdot O_2^- \xrightarrow{H_2O} e^{\ominus}_{H_2O} + O_2$$

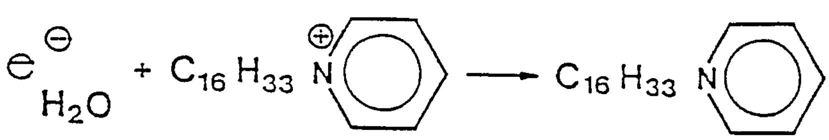

In allen Reaktionen, die im entzündlichen Gebiet zwischen pH 5,0 und 6,0 ablaufen, verlangen, was durch die verfahrensmässige Herstellung dieser Erfindung gewährleistet ist, einen pH-Bereich ≤ 7,0. Dadurch werden die gebildeten, agressiven Sauerstoffradikale gemäß der Reaktion 1-4 durch das N-Tensid,z.B. Cetylpyridiniumchlorid, als auch die entstehenden hydratisierten, kurzlebigen Elektronen, die durch Zusammenstoß $\cdot O_2^-$ -Radikale mit $H_2O$ entstehen können, abgefangen. Dadurch haben die N-Tenside in dem pH-Bereich ≤ 7,0 erfindungsgemäß eine membranprotektive Wirkung, so daß es nicht zu Entzündungsreaktionen gemäß eines Prostaglandinmechanismus kommen kann. Die hohe Einfangrate $\cdot O_2^-$ -Radikale bei diesen N-Tensiden von $k = 5 \times 10^{12} N^{-1} sec^{-1}$ und ihrer Abhängigkeit von der Ionenstärke, die allerdings durch Zusatz von Ethanol/Glycerol konstant gehalten werden kann, wird durch die elektrostatische Doppelschichtstruktur der quartären Ammoniumbasen erklärbar.

So werden erfindungsgemäß fehlgeleitete lytische Reaktionen, an denen aggressive Sauerstoffradikale beteiligt sind, als pathogene Mechanismen der entzündlichen Erkrankungen, hervorgerufen durch Mikroorganismen und Viren, verhindert. So werden auch u.a. die cytotoxische Wirkung der Folgeprodukte dieser agressiven $.O_2^-$ -Radikale durch die erfindungsgemäßen N-Tenside, wie am Beispiel von Cetylpyridiniumhalogenid gezeigt, verhindert und u.a. Depolymerisationen von Hyaluronsäuren, Proteoglykanen, Collagenfibrillen, Cytoskeletons usw. und auch bei mukösen und membranösen Geweben (äussere Oberflächen) verhindert.

Weiterhin wurde gemäß der beschriebenen verfahrensmässigen Herstellung gefunden, daß Verbindungen der Struktur I und II die Infektion von menschlichen Zellen in vitro vermindert wird, so daß die erfindungsgemäß hergestellten micellaren Lösungen von I und II einen Schutz für die Zellen bzw. deren externer Oberfläche darstellen.

Es wurde weiter gefunden, daß dieser Schutz durch Inkorporation von $Hg(CN)_2$, ZnEDTA und/oder antiviralen, antifungalen und antibakteriellen Wirkstoffen verstärkt wird:

Es wurde gefunden, daß bei Inkubation von mit Influenzavirus, Untergruppe $A_2$, infizierte Monolayer-Zellkulturen von Vero-Zellen als auch bei Herpes simplex-Virus HSV I-III in vitro mehr als 60 % der Zellen vor der Infektion durch das betreffende Virus geschützt werden.

Es wurde weiter gefunden, daß der Effekt der Protektion durch die $N^+$-Tenside gemäß der allgemeinen Formel I und II für Mono-layer Zellfunktionen in vitro durch die antiviralen Wirkstoffe nicht verstärkt wird, wohl aber werden die Hemmkonzentrationen der antiviralen Wirkstoffe von Cytarabin, Idoxuridin, Trifuorthymidin als auch Herpes simplex Virus Typ 1 oder Influenza Virus Typus $A_2$ infizierte Monolayer-Zellen um 30 % gesenkt gegenüber Applikationen, die keine quartäre Ammoniumbasen gemäß Formel I und II enthielten. Die Kombination von $N^+$-Tensid gemäß der allgemeinen Formel I und II erwies sich somit als das wirksame Virustatikum in Kombination mit micellar gebundenen antiviralen Wirkstoffen (Abb. 4).

Es wurde weiter gefunden, daß die $N^+$-Tenside gemäß der allgemeinen Formel I und II die antifungale Wirkung in Kombination mit antifungalen Wirkstoffen wie Econazol, Clotrimazol, Miconazol verstärkt (≈35 %), da die $N^+$-Base bei geeignetem Gegenion in der Lage ist, Cholesterol aus der externen Membran des Pilzes oder Hyphen unter Bildung von gemischten Micellen ("mixed micelles") zu extrahieren und dann die antifungalen Wirkstoffe, die wieder gebunden sind, in das Zellinnere des Fungus injizieren kann.

Es wurde weiter gefunden, daß die antifungale Wirkung durch Amphotericin B und eines N-Tensides der Formel II, vorzugsweise Hexadecylpyridinium-bromid, Decyl-und Hexadecyl-1-pyridinium-chlorid oder Hexadecyl-4-hydroxy-pyridinium-chlorid durch einen bislang nicht bekannten Mechanismus um das zehnfache verstärkt wird. Dies beinhaltet erfindungsgemäß, daß eine wesentlich geringere Wirkstoffkonzentration des pharmazeutischen Wirkstoffes ausreicht, um die gleichen therapeutischen Effekte zu erreichen.

Es wurde u.a. gefunden, daß die fungistatische Wirkung durch micellaren Einbau von ZnEDTA und ZnO, insbesondere auch durch $Hg(CN)_2$ in die N-Tenside der Formel I und II, insbesondere bei Hexadecylpyridiniumchlorid und Hexadecyl-4-hydroxy-pyridiniumbromid verstärkt wird, insbesondere bei Konzentrationen der anorganischen Wirkstoffe, wo sie selber noch nicht wirksam sind.

Es wurde gefunden, daß erfindungsgemäß die Micellen der N-Tenside in wässriger Phase bei pH ≤ 7,0 therapeutische Mengen von Benzoylperoxid, das schlecht wasserlöslich und alkohollöslich ist, micellar binden können.So können z.B. 1 g Benzoylperoxid in 20 ml Benzethoniumchlorid oder in 25 ml Hexadecylpyridiniumchlorid, insbesondere aber in 3-Hexadecylbenzothiazonium-bromid gelöst werden. Bei örtlicher Anwendung löst die micellare Lösung ähnliche Schäleffekte an der Haut aus wie Tretinoin. Aufgrund seiner zusätzlichen sehr guten bakteriostatischen Eigenschaften, sowohl des Benzoylperoxides als auch des N-Tensides, ist erfindungsgemäß diese Kombination bei entzündlichen Akneformen besonders geeignet, z.B. bei Acne comedonica, Acne papulo-pustulosa und Acne conglobata.

Es wurde gefunden, daß die erfindungsgemäß hergestellten Micellen in wässriger Phase der N-Tenside, in denen $Hg(CN)_2$ oder ZnO, $ZnSO_4$, ZnEDTA micellar eingeschlossen ist, in der Zellkultur irreversibel und virusspezifisch die Vermehrung von Herpes simplex Viren infolge Hemmung der viralen DNS-Polymerase, hemmen. Die nichtinfizierten Zellen bleiben weitgehend unbeeinflußt, so daß die in dieser Erfindung beschriebenen Verfahren, z.B. für Hexadecyl-pyridiniumchlorid, 3-Hexadecylbenzothiazolium-bromid (Sulfat) einschließlich der vorne genannten anorganischen Wirkstoffe, zu einem risikolosen Therapeutikum führt. Die adstringierenden Eigenschaften von $Hg(CN)_2$, ZnO, $ZnSO_4$, ZnEDTA, spielen keine Rolle, da im hydrophoben Core der Micellen keine freien Ionen vorliegen, a) da z.B. $Hg(CN)_2$ (richtiger $Hg_2(CN)_4$) undissoziiert ist, b) die anorganischen Wirksubstanzen durch ihre Lipophilie eingeschlossen sind und c) fast kein Wasser im hyrophoben Kern vorliegt.

Der kombinierte Effekt, die Bildung von gemischten Micellen ("mixed micelles") der N-Tenside gemäß der allgemeinen Formel I und II mit der durch den Virus befallenen Membran und der Phospholipid-Doppelmembran des Virus selber und die anschließende antivirale Wirkung auf die Virus DNS-Polymerase durch die vorne genannten anorganischen und organischen Wirkstoffe wie der Nukleosid analoge, 5-Ethyl-2'-desoxy-uridin und Viderabin, konnte gemäß Abb. 4a, b nachgewiesen werden.

Dieser Mechanismus konnte auch bei Rhino-und Influenza-Viren nachgewiesen werden. Ähnliche Wirkungen, jedoch bei geringeren Wirkstoffkonzentrationen wurden für 1,1':3,3'-Biscyclobutan und für 1,1':3,3'Amin substituierte Kubane gefunden.

Es wurde gefunden, daß die verstärkte antivirale Wirkung bei Phospholipid-Viren, Adeno-Viren und Herpes simplex I durch das N-Tensid und die micellar eingeschlossenen Wirkstoffe gemäß folgenden biochemischen Mechanismen ihre Wirkung synergistisch entfalten:

a) Bindung an DNS, RNS-bildende Enzymsysteme, Entfaltung der Polypeptidkette durch das N-Tensid (Denaturierung) verstärkte.

b) "template"-Bindung, z.B. Daunomycin, Adriamycin

c) Binding an Nucleosidanaloga, z.B. das vorne erwähnte ara-CTP-C5'-triphosphat das Cytosinarabinosids, Azathioprin;

d) Bindung von anorganischen Wirkstoffen, z.B. $ZnSO_4$, ZnO, $Hg(CN)_2$, Wolframsäure-antimonate, z.B. $(NH_4)_{18}$ $(NaW_{21}Sb_9O_{86})_{17}$ und $K_{18}(KW_{21}Sb_9O_{86})_{17}$, sowie Hg-substituierte Kubane des vorne genannten Typus.

Im Zusammenhang mit der antiviralen Wirkung der micellar eingeschlossenen antiviralen Wirkstoffe gemäß der verfahrensgemäßen Bearbeitung ist eine Reduktion der $ED_{50}$ um 20-25 % in vitro gegenüber dem reinen Wirkstoff zu verzeichnen, so daß die gleiche molekularbiologische Wirkung bei einer ca. 20 %igen Dosis durch den micellaren Effekt zu erzielen ist. Dies gilt insbesondere für micellar eingeschlossenes Rubaricin in Hexadecyl-pyridinium-Bromid, Hexadecyl-benzothiazolium-chlorid und Benzethonium-chlorid. DNA-Viren, Herpes-Viren sind bei diesen Beispielen am sensitivsten im Gegensatz zu Rimantadin + N-Tenside der Formel I und II, welche primär in vitro gegen RNA-Viren wirksam sind.

Es wurde weiter gefunden, daß die Antitumoraktivität von Adenosin-Desaminase Inhibitoren, die micellar gemäß Formel I und II verfahrensgemäß gelöst sind, z.B. Erythro-9-(2-hydroxy-3-nonyl)-Adenin, Deoxycoformycin, um das Zehnfache verstärkt wird. Das gleiche konnte auch für Aspartat-Transcarbamylase Inhibitoren festgestellt werden; so wurde durch micellar eingeschlossenes N-(phospono-acetyl)-aspartat die Biosynthese von Pyrimidin durch Blockierung der Karbamylierung von Aspartat um das 20-fache verstärkt.

Außerdem wurde gefunden, daß sowohl micellar eingeschlossenes $Hg(CN)_2$, $ZnSO_4$, ZnO oder ZnEDTA, wie auch 5-trifluor-methyl-2'-Desoxyuridin, welches aus Trifluor-methyl-Urazil in vitro entsteht, die Thymidin-Synthetase - ein Schlüsselenzym der DNS-Synthese - irreversibel hemmt.

Die Pyrimidin-Biosynthese wird durch Pyrazofurin, einem natürlich vorkommenden Antibiotikum, welches micellar eingeschlossen ist, um 20 % irreversibel gehemmt, bei gleichzeitiger Reduzierung der Zelltoxizität.

Es wurde weiterhin gefunden, daß die Diffusionsbarriere für Antibiotika, z.B. Tetrazyklinen, Aminoglykosiden auch für $\beta$-Lactamantibiotika (Penicillin) nach einer gewissen Zeit bei E. coli Bakterien für die micellar eingeschlossenen Wirkstoffe herabgesetzt werden. Diese Diffusionsbarriere ist konzentrationsabhängig für die vorne genannten Wirkstoffe, jedoch nicht für die verfahrensgemäß hergestellten N-Tenside. Hier handelt es sich um Faltungsprozesse an der äusseren Membran, primär um die Änderung der Struktur der Porine innerhalb der äusseren Membran von E. coli, so daß z.B. die anorganischen Wirkstoffe $Hg(CN)_2$, $ZnSO_4$, ZnEDTA in das Periplasma der äusseren Zellmembranen von grammnegativen Bakterien hinein diffundieren können.

Die Porine sind membranöse, wassergefüllte Poren, durch die hydrophile pharmazeutische Wirkstoffe in das Innere der Zelle diffundieren können. Hydrophobe pharmazeutische Wirkstoffe können diese Porine nicht passieren. Die $N^+$-Tenside, insbesondere der allgemeinen Formel $HET{\equiv}N\text{-}(CH_2)_x\text{-}CH_3y^{\ominus}$ als auch Benzetoniumabkömmlinge können diese wassergefüllten Poren passieren. Somit können micellar eingeschlossene pharmazeutische, hydrophobe (lipophile) Wirkstoffe, insbesondere anorganischer Natur, durch den hydrophilen, äußere Form der $N^+$-Tenside durch passive Diffusion ins Zellinnere gelangen.
Hier reagieren sie dann außerdem mit den Zellwand-synthetisierenden Enzymen, insbesondere mit $Hg(CN)_2$ bei Konzentrationen von 10 µg/ml, ZnEDTA bei c= 5 µg/ml.
Die Geschwindigkeit der Diffusion von micellar eingeschlossenen Wirkstoffen steigt mit steigendem hydrophoben Charakter, normalerweise ist dies genau <u>umgekehrt</u>, z.B. sinkt die Diffusionsgeschwindigkeit bei gramnegativen Bakterien mit steigendem hydrophoben Charakter. Weiterhin begünstigt eine positive Ladung die Diffusion und die Ausbildung von "mixed micelles" von diesen erfindungsgemäß herzustellenden N-Tensiden.
Die Gültigkeit dieser Befunde konnte durch Untersuchungen der Diffusions-und Auflösungsgeschwindigkeiten verschiedener radioaktiv ($C^{14}$) markierter N-Tenside an der Membran (Periplasma) konzentrationsabhängig nachgewiesen werden.

Es wurde außerdem in vitro gefunden, daß die Thymidilat-Synthetase (TS) (EC2.1.1.45) sowohl durch $Hg(CN)_2$ in wässriger Lösung als auch in micellarer Lösung eines N-Tensides der Formel I und II, in dem $Hg(CN)_2$ hydrophob gelöst ist, gehemmt wird. TS katalysiert die Umwandlung von dUMP und $CH_2$-$H_4$-Folat zu dTMP und $H_2$-Folat. Da dieses Enzym für die Synthese von dTMP essentiell ist, also für die DNS-Synthese schlechthin, stellt es somit auch ein Target für pharmazeutische Wirkstoffe gegen neoplastische Zellen dar. Es wurde nun gefunden, daß z.B. eine erfindungsgemäß hergestellte Lösung von Hexadecylpyridinium-chlorid, das $Hg(CN)_2$ hydrophob gebunden hält, gemäß der Tabelle 1 aufgeführten zytostatischen Aktivitäten gegenüber Leukämiezellen(L1210 -Zellen) hat. So konnte u.a. gefunden werden, daß TS, dUMP und $Hg(CN)_2$ als anorganischer pharmazeutischer Wirkstoff einen ternären Komplex gemäß -(A,B)

A

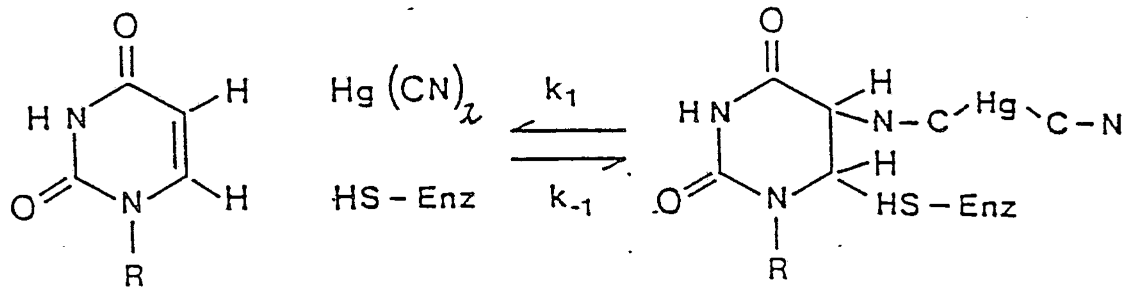

(d UMP)

R = DESOXy RiBOSE

B

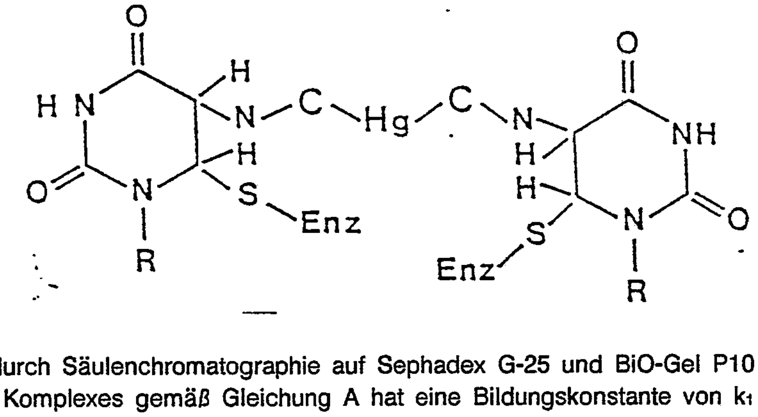

bilden, der durch Säulenchromatographie auf Sephadex G-25 und BiO-Gel P10 isoliert werden kann. Die Bildung des Komplexes gemäß Gleichung A hat eine Bildungskonstante von $k_1$ = 0,51 $h^{-1}$, im Falle von Hexadecylpyridiniumchlorid, und $k_1$ = 0,79 $h^{-1}$ bei Benzethoniumchlorid und micellar eingeschlossenem $Hg(CN)_2$. Die Dissoziationskonstanten belaufen sich auf $k_{-1}$ = 0,015 $h^{-1}$ (CPCl) und $k_{-1}$ = 0,02 $h^{-1}$, also beide sehr langsam, sowohl die Bildung als auch die Dissoziation des Komplexes. Dagegen ist die Bildung des Dimeren nach B wesentlich schneller: $k_1$ = 0,02 $h^{-1}$ und $k_{-1}$ = 0,015 $h^{-1}$ bei CPCl und $k_1$ = 0,01 $h^{-1}$ für Benzethoniumchlorid. D.h., daß micellare Lösungen von quartären Ammoniumbasen gemäß Formel I

und II bei pH ≤ 7,0, welche $Hg(CN)_2$ hydrophob gebunden halten, sind daher therapeutisch bei langsam wachsenden Tumoren, wo andere Inhibitoren bei TS unwirksam sind und die beobachtete Cytotoxizität gegenüber den normalen Zellen von anderen Antimetaboliten bei schnell wachsenden, proliferierenden Zellen kann daher verlangsamt werden.

Ribavirin, welches ein synthetisches 1,2,4-Triazolnukleosid ist, besitzt ein breites antivirales Spektrum für DNA-und RNA-Viren. Micellar eingeschlossenes Ribavirin durch kationische Tenside der Form (Het≡$N^+$-$(CH_2)_x$-$CH_3$ )Y⊖ sehr schnell die Membranbarriere passiert, schneller als der pharmazeutische Wirkstoff selber. Auch die Umwandlung von Ribavirin zu Monophosphaten, Diphosphaten und Triphospaten durch die spezifischen zellulären Enzyme wird gesteigert, so daß die Hemmung der virusinduzierten Enzyme, welche notwendig sind für die virale Nukleinsäurebiosynthese, beschleunigt wird. Während Ribavirin alleine nur einen moderaten Effekt auf die zelluläre DNA-Synthese hat und zytotoxisch im Bereiche von 200-1000 µg/ml bei normalen Zellinien ist, sinkt die Zytotoxizität in Gegenwart von kationischen Micellen, wenn micellar eingeschlossen, auf 50 µg/ml (in vitro-Teste), gemessen gegen Herpes simplex (DNA-Virus) infizierten Zellen.

Amantadin (I-Adamantanamin-HCl) besitzt besondere pharmakodynamische Wirkung gegen Influenza-Viren (Klasse A). Die Replikation der meisten Influenza-A-Stämme werden in vitro zwischen 0,2 - 0,6 µg/ml gehemmt. Micellar eingeschlossenes Amantadin in kationische Micellen, insbesondere der Form (Het≡N-$(CH_2)_x$-$CH_3$ )Y⊖ bewirken eine Reduzierung der Konzentration an pharmazeutischem Wirkstoff auf 0,05 µg/ml Amantadin gemessen nach Hayden et. al., (Plaque inhibition assay for drug susceptibility resting of influenca viruses. Antimicrob. Ag. Chermother. 1980, 17 : 865-70; Grunert et al.; Sensitivity of influenza A/New Jersey 18/76/(Hswl-N1)-virus to amantadine HCl, J. Inf. Dis. 1977, 136, 297-300). Während Amantadin gegen Influenza-Virus Typ B fast keine Aktivität besitzt, besteht eine Hemmung von micellar eingeschlossenem Amantadin in den kationischen Tensiden der Formel

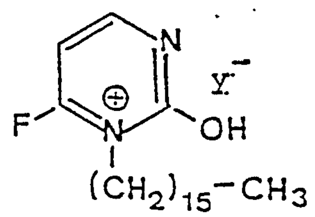

I

$Y^-$ = Fumarsäurerest

(2-Hydroxy-5-Fluor-Hexadecylpyrimidinium-fumarat)

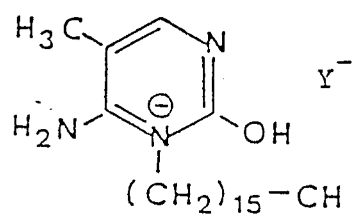

$Y^-$ = Fumarsäurerest

(2-Hydroxy,5-methyl-6-amino-hexadecylpyrimidinium-fumarat)

Bei einer Konzentration von 0,01 Gew.% von Amantadin bei Influenza-Virus Typ B entsprechend einer Konzentration von 0,5 µg/ml pharmazeutischen Wirkstoff in vitro.

Ein überraschender Effekt von micellar eingeschlossenem Amantadin in den beiden kationischen Tensiden der obigen Formeln wurde gefunden, das Influenza-Virus Typ A gegen Amantadin in vitro nicht resistent wird, während es bei Amantadin allein der Fall ist.

Rimantadin-HCl ($\alpha$-Methyl-l-adamantanmethylamin-hydrochlorid) hat die gleichen pharmakodynamischen Wirkungen in vitro wie Amantadin, jedoch ist stärker wirksam bei gleicher Dosis. Auch hier wurde überraschenderweise gefunden, daß micellar eingeschlossenes Rimantadin in ein kationische Tensid, insbesondere bei beiden obigen Formeln, der gleiche in vitro-Effekt gefunden wurde, wie beim reinen pharmazeutischen Wirkstoff, allerdings bei einer wesentlich geringeren Dosis von 0,05 $\mu$g/ml.

Unter den anorganischen pharmazeutischen Wirkstoffen enfaltet $Hg_2(CN)_4$ und micellar gebundenes $Hg(CN)_2$ in kationischen Micellen ein überraschendes antivirales, interferoninduziertes Spektrum. In Zellkulturen von Lymphozyten und Fibroblasten konnte eine Induktion von Interferon $\alpha_1$ und Interferon $\beta$ nach Inkubation mit micellar eingeschlossenen $Hg(CN)_2$ in einem kationischen Tensid der Formel

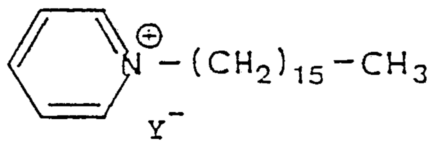

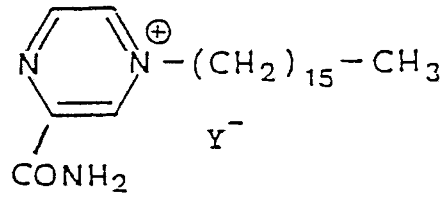

$Y^-$ = Chlorid

$Y^-$ = Salizylat

Hexadecylpyridiniumchlorid

2-Carboxamid-hexadecyl-pyrazinium-salizylat

bei einer Konzentration von $Hg(CN)_2$ von 5 $\mu$g/ml bis zu 15 $\mu$g/ml von einer 0,1 % ($^g/g$) kationischen Tensides festgestellt werden. Es wurde im Falle von Interferon $\alpha_1$ Konzentrationen von 20-50 Units/ml ermittelt, im Falle von Interfern $\beta$ 10-20 Units/ml. Durch den micellaren Einbau des Quecksilbercyanids ist die Freisetzung des Interferon $\alpha_1$ insbesondere aber durch das Interferon $\beta$ bei Fibroblastenkulturen um das 10 bis 100-fache gegenüber einfachen wässrigen, gepufferten Lösungen von Quecksilber II-canid gesteigert.

Sekundäreffekte

Die beobachteten Nebenwirkungen von Interferon $\alpha_1$, wie z.B. Kopfschmerzen, Lymphozytopenie, leichtes bis mittelschweres Krankheitsbefinden liegen bei den hier beschriebenen pharmazeutischen Zubereitungen, insbesondere gegen Influenza-und Rhinoviren, nicht vor bzw. wurden nicht beobachtet. Dies liegt vornehmlich daran, daß wesentlich geringere Einheiten/ml des Interferon $\alpha$, induziert durch den anorganischen pharmazeutischen Wirkstoff, therapeutisch zur Anwendung kommen als bei einer Therapie mit Interferon $\alpha$ alleine. So wurden auch keine toxischen Effekte, z.B. gastrointestinale Störungen, Gewichtsverlust, Alopezia, periphere Krämpfe, Paraesthesien und Knochenmarksdepressionen beobachtet, welche allerdings reversibel sind.

Die hämatologische Toxizität, welche im Falle von Interferon $\alpha_1$ dosisabhängig ist (Thershold-dosis $3 \times 10^6$ Units/ml), liegt bei diesen pharmazeutischen Zubereitungen für Quecksilbercyanid, Rimantadin, Amantadin und Ribavirin nicht vor.

b) Die pharmazeutische Zubereitung, bestehend z.B. aus Hexadecylpyridiniumchlorid oder einem Pyrimidiniumderivat und einem Hexadecylrest in Gegenwart von micellar eingeschlossenem Quecksilbercyanid bewirken in der Zellkultur eine Interferonproduktion. Es handelt sich hier um eine Interferoninduktion durch freigesetztes Quecksilbercyanid, das örtlich in hohen Konzentrationen vorkommt und mit einem relativ hohem Molekulargewicht von 4500 durch Ausbildung von polymeren Strukturen. (Paradies, Oligomere Struktur von Quecksilbercyanid in Lösung, Vortrag Deutsch-Österreichische Chemische Gesellschaft, Innsbruck, Mai (1986.) Somit gehört diese pharmazeutische Zubereitung zu den Stoffen definierter Interferoninduktoren von hohem Molekulargewicht wie z.B. synthetische doppelsträngige RNA:PolyI:PolyC als auch niedrigem Molekulargewicht wie z.B. 10-Carboxymethyl-9-acridanon. Trotz dieser Heterogenität der Interferonwirkung ist es antiviral wirksam. Diese Wirkung diente zum biologischen Nachweis dieser pharmazeutischen Zubereitung. Es kann gesagt werden, daß die Interferonbehandlung von Zellen in Zellkultur derartig verändert werden, daß eine nachfolgende Virusreplikation in diesen Zellen gehemmt wird. Dadurch unter-



BAD ORIGINAL

scheiden sich Interferone in ihrem Wirkungsmechanismus grundsätzlich von Virustatika, die den Stoffwechsel der Viren selbst hemmen. Da Interferone auf die Zellen wirken, ist es nicht verwunderlich, daß sie auch andere Wirkungen auf die Zellen ausüben können. Dies gilt nicht nur für Zellkulturen sondern hat auch Auswirkungen für den Gesamtorganismus. Es ist bekannt aus vielfältigen Untersuchungen, daß Interferon eine Vielzahl von Viren in ihrer Vermehrung hemmt. Das Ausmaß der Hemmung ist abhängig vom jeweiligen Virussystem. Somit scheint die Vermehrung fast aller Viren durch Interferon-Behandlung der Zelle hemmbar zu sein. Es gibt offensichtlich verschiedene Mechanismen, mittels deren Interferone wirksam sein können. So wird z.B. die Vermehrung von Retroviren auf die Bildung des "budding" d.h. des Ausschleusens neu gebildeter Virionen beeinflußt. Ein ähnlicher Mechanismus scheint auch für die pharmazeutische Zubereitung mit micellar eingeschlossenem $Hg(CN)_2$ zuzutreffen. So wurde im Rahmen der Erfindung beim Herpes simplex-Virus (HSV 1-3) durch die pharmazeutische Zubereitung bestehend aus Cetylpyridiniumchlorid und Quecksilbercyanid (s. Beispiel) induziertes Interferon auf die Synthese früh viralkodierter Proteine des HSV die sogenannten $\beta$-Proteine nachgewiesen. Beim SV40-Virus scheint Interferon ebenfalls auf einen sehr frühen Schritt der Vermehrung zu wirken, der noch vor der primären Transkription liegt.

Die erfindungsgemäße pharmazeutische Zubereitung, speziell micellar eingeschlossenes Quecksilbercyanid in 7-Hexadecylimidazolium-2,6-diamino-[4,5-d]-pyrimidin-chlorid ließen die Interferon bedingte Hemmung bei verschiedenen lytischen RNA-Viren beobachten. Die Inhibition erfolgt auf der Ebene der Regulation der viralen Proteine. Sie wird hervorgerufen durch Induktion bestimmter zellulärer Enzyme. Bei näherer Untersuchung wurde gefunden, daß die Enzyme der 2',5'-Oligo--adenylat-Synthetase, der 2,5-A-abhängigen Endoribonuklease und die dsRNA-abhängige Proteinkinase hemmen. Für die beiden ersteren konnte durch Korrelationsstudien und durch Charakterisierung von Zellmutanten eine Rolle in der antiviralen Aktivität gegen lytische RNS-Viren durch micellar gebundenes $Hg(CN)_2$ nachgewiesen werden.

In diesen experimentell nachgewiesenen Effekten konnte auch ein antiproliferativer Effekt dieser pharmazeutischen Zubereitung auf die Interferone in vielfältiger Weise auf die Membranen und das Zytoskelett von Zellen nachgewiesen werden. So beeinflussen sie weiterhin die Expressionen einer Reihe wichtiger Gene, z.B. die der Haupthistokompatibilitätslokus, die sogenannten Transplantationsantigene. Somit zeichnen sich auch immunregulatorische Wirkungen ab (Interleukin-1-Synthese). Dadurch ergeben sich therapeutisch und pharmakodynamisch folgende Aspekte: Die Induzierung der Interferone durch diese pharmazeutische Zubereitung führt zu verstärkter Expression der Zelloberflächenproteine, die die wichtigste Rolle bei der Immunantwort spielen. Es sind dies die sogenannten Transplantationsantigene. Weiter ist anzuführen, daß mindestens zwei wichtige zelluläre Komponenten der körpereigenen Abwehr aktiviert werden, nämlich die Makrophagen und die natürlichen Killerzellen. Außerdem, was vor allem das Interferon $\gamma$ betrifft, scheinen auch die Funktionen vom B-Lymphozyten maßgeblich durch diese pharmazeutische Zubereitung beeinflußt zu werden.

Somit ergibt sich für die erfindungsgemäße pharmazeutische Zubereitung, insbesondere bestehend bei Hexadecylpyridiniumchlorid und micellar eingeschlossenem Quecksilbercyanid oder von 7-Hexadecylimidazolium-2,6-diamino-[4,5-d]pyrimidinchlorid oder auch das Bromid, eine Induktion, wenn auch keine spezifische, von Interferon. Es kann kein Zweifel daran bestehen, daß Interferone nicht nur in vitro sondern auch in vivo nicht nur antiviral sondern auch immunregulatorisch wirksam sind. Obwohl der direkte antivirale Effekt auch in vivo bedeutsam sein kann, spielen im Organismus bei der Interferon-Wirkung, wie sie oben aufgezeichnet worden sind, weitere indirekte Mechanismen eine Rolle, z.B. die Aktivierung von Makrophagen speziell bei Influenza-Virus A und B. Die Tatsache, daß Interferon $\gamma$ Makrophagen aktivieren kann, scheint auch im Hinblick auf bakterielle und parasitäre Infektionen wichtig zu sein, da bei deren Abwehr Makrophagen eine entscheidende Rolle spielen.

Posologie und therapeutische Indikationen:

Die therapeutischen Indikationen sowie die Dosis ergeben sich durch die micellar eingeschlossenen Konzentrationen der pharmazeutischen Wirkstoffe:

-So bestehen Indikationen für aufkommende und bestehende Erkältungskrankheiten, die vornehmlich durch Influenza-und Rhinoviren verursacht werden;

-katarrhalische Entzündungen viruider Genese;

-Hautinfektionen und infektiöse Dermatosen;

-hartnäckige, antiseptische Wundbehandlung;

- Dermatomykosen;

-Mykosen;

-Prophylaxe und Therapie bakteriell bedingter Hautläsionen wie z.B. Pyodermie, Otitis media;
-mikrobielle und sekundär infizierte Ekzeme;
-Überempfindlichkeit gegen Makrolid-Antibiotika;
-Akne, insbesondere entzündliche Formen mit Papeln und Pusteln;
-bakterielle Infektionen und Sekundärinfektionen der Haut, sofern sie durch grampositive und/oder gramnegative meklocyclinsensible Keime hervorgerufen werden;
-Akne vulgaris;
-Verhütung von Nabelinfektionen;
-chirurgische und traumatische Wunden;
-lokaler Schutz vor Infektionen und mit Antibiotika empfindlichen Keimen infizierte Wunden;
-Furunkel, Karbunkel, Abszess;
-Dermatomykosen, verursacht durch Dermatophyten, Hefen, Schimmelpilze und anderen Pilzen, Pityriasis versicolor,
-Erythrasma durch Cornebakterien;
-Candidosen der Haut und Schleimhäute
-Herpes simples I-III, Herpes-Keratitis
-solare und senile Keratosen, prämaligne Veränderungen und oberflächliche Basaliome, auch in strahlengeschädigter Haut;
-Plattenepitelkarzinome der Haut und Mukosa im Kopf-und Halsbereich; dermatologische Malignome;

Je nach Indikationsstellung und pharmazeutischem Wirkstoff richtet sich die spezielle Dosis. Da die Erhaltungs-und Anfangsdosis der hier beschriebenen pharmazeutischen Wirkstoffe bekannt sind, wird je nach Applikationsart und galenischer Zubereitung z. B. Cremn, Zäpfchen, Tropfen, Tabletten, Kapseln und liposomartige Verkapselungen nur 50 % der normalen therapeutischen Gesamtdosis benötigt, je nach Konzentration des micellar eingeschlossenen pharmazeutischen Wirkstoffes.

Schilderung der Dosisreduzierung aufgrund des potenzierenden (synergistischen) Effektes:

Micellen in wässriger Lösung, auch mit eingeschlossenen lipophilen Wirkstoffen, stehen im dynamischen Gleichgewicht mit ihren monomeren Tensiden, d.h. die Micellen ändern Form, Größe und Hydratation. U.a. verlassen monomere kationische Tenside eine einzelne Micelle, um sich an einer anderen Micelle in wässriger Lösung wieder einzugliedern, so daß -auch wenn die Konzentration des Tensides weit über der KMK liegt - immer eine gewisse Konzentration von fluktuierenden Monomeren besteht. Durch Zugabe des Potenzierungsgemisches wird diese Dynamik dahingehend gestört, daß

1.) bei konstanter Temperatur und chemischen Potentials die Form, Größe und monodisperse Homogenität der isotropen Lösung erhalten bleibt, somit tritt auch kein Verlust an micellar eingeschlossenem lipophilen (hydrophoben) pharmazeutischen Wirkstoff ein.

2.) Die Konzentration an Monomeren, welche destabilisierend auf die geometrische Form der Micelle wirkt, wird zugunsten eines Einbaues in die"vollständigen" Micelle in isotroper Lösung eingeschränkt. Dies bewirkt, daß das System einschließlich der micellar eingeschlossenen hydrophoben pharmazeutischen Wirkstoffen "leckt". Dies wird vornehmlich dadurch verhindert, daß das Potenzierungsgemisch, insbesondere Glycerin und Dimethylsulfoxid, die Wasserstruktur an der externen Oberfläche der Micelle so einfrieren ("Tridymit-Struktur"), daß sie eisartige Strukturen annehmen und die Wassermoleküle sehr unbeweglich werden.

3.) Die pharmazeutische Zubereitung wirkt aufgrund des potenzierenden Effektes durch Glycerin, wie z.B. in vitro gezeigt werden konnte, weniger zytotoxisch, d.h. es schädigt vornehmlich die beschädigte (infizierte) Zelle,weniger die gesunde Zelle im Zellverband.

Die Erfindung weist insbesondere folgende Vorteile auf:

Die in dieser Erfindung hergestellten N-Tenside, einschließlich ihrer verfahrensgemäßen Einschließung der Wirkstoffe, bedingt eine erhebliche, z.T. bis zu 80%ige Reduzierung der Toxität der anorganischen Wirkstoffe, z.B. bei $Hg(CN)_2$, (auch $HgCl_2$, $Hg_2Cl_2$, $HG(NH_2)Cl$ [Präzipitat], ZnEDTA) und Zn Salzen im allgemeinen, als auch bei nephrotoxischen, ototoxischen Antibiotika, insbesondere bei Polymixinen, Erythromycin, Gentamycin, Tetrazyclin, von ca. 30 % da

1.) die Micellen, als auch ihre eingeschlossenen Wirkstoffe, nicht - wegen ihrer Größe - resorbiert werden,

2.) die miceller eingeschlossenen Wirkstoffe sich nur am Ort - meistens topisch - entfalten, so daß geringe Konzentrationen an Wirkstoff ausreichen, da zusätzlich noch der synergistische Effekt des N-Tensides besteht.

So wurde u.a. gefunden, daß der hemmende Effekt der Speichelsekretion von Atropin durch Hexadecylpyridiniumchlorid als auch durch Benzothiazolium-sulfat durch micellare Katalyse um das Zehnfache verstärkt wird, bei verfahrensgemäßer Herstellung der N-Tenside, pH $\leq$ 7,0. Die verstärkte Wirkung auf die Peripherie ist u.a. durch die micellare Auftrennung des Racemates in L(-) Hyocyamin verantwortlich (siehe Abbildung 1).

Auch stabilisiert z.B. Hexadecyl-benzthiazolium-sulfat das Atropin durch Einbeziehung der hydrophoben Molekülregionen des Atropins in den micellaren Kern.

Dieser Inhalt des Patentanspruches erstreckt sich auch auf die antiphlogistischen Eigenschaften der hier beschriebenen quartären organischen Ammoniumbasen. Das Gegenion $Y^-$ nimmt verfahrensbedingt Einfluß auf die Größe, Form und micellare Stabilität, kann aber auch selber ein pharmazeutischer Wirkstoff sein, so daß eine Arzneimittelwirkung pharmakodynamisch verstärkt werden kann. Die hier beschriebenen Tenside haben den großen Vorteil, daß sie neben intrinsischen pharmakodynamischen Eigenschaften milieuabhängig und darüber hinaus im sauren pH-Bereich stabil sind. Die verfahrensmäßigen erhältlichen Micellen als pharmazeutische Zubereitung mit eingeschlossenen lipophilen (hydrophoben) pharmazeutischen Wirkstoffen wirken als Träger für antimikrobielle, antivirale, keratolytische, antifungale und antineoplastische Wirkstoffe, können aber u.a. selber antimikrobielle, antifungal und antiviral und antiphlogistisch (topisch) wirksam sein.

Insbesondere beschreibt die vorliegende Erfindung eine pharmazeutische Zubereitung zur Herstellung von micellaren gelösten hydrophoben anorganischen Wirkstoffen wie Quecksilber-II-Cyanid, Zink, Wolfram und Antimon-Verbindungen sowie Salze der Phosphonsäure. Es wurde gefunden, daß diese anorganischen Verbindungen sowohl antivirale als auch antineoplastische Wirkungen haben.

Auch die Bildung von vesikulären Strukturen der quartären Ammoniumbasen von 4-und 3,5-substituierten Hexadecylpyridinium-$Y^-$ erfolgt spontan bei konstanter Temperatur, Druck, Ionenstärke einschließlich in Gegenwart von eingesetzten stöchiometrisch pharmazeutischen Wirkstoffen, welche sowohl vesikulär (Hohlraum) als auch micellar (doppelmembranartig) gebunden werden können.

Insbesondere bezieht sich die Erfindung auf ein verbessertes Herstellungsverfahren zur Erzeugung von multilamellaren Lipdbläschen auf der Basis von kationischen Tensiden, die dazu benutzt werden können, insbeson dere lipophile (hydrophobe) pharmazeutische Wirkstoffe einzukapseln.

Die meisten bisher bekannten Verfahren leiden entweder an einer zu geringen Einkapselwirkung oder an einer Begrenzung der Materialtypen, welche eingeschlossen werden sollen, oder auch an beiden. So sind bekanntermaßen die meisten dieser Prozesse auf den Einschluß hydrophiler Materialien und pharmazeutischer Wirkstoffe beschränkt und können nicht wirkungsvoll den Einschluß von lipophilen pharmazeutischen Wirkstoffen vornehmen. Im Gegensatz dazu sind alle derzeit verfügbaren Verfahren mit Ausnahme das von Banghan et al. (Biochim.Biophys.Acta 443:629-634, 1976) nur für die Einkapselung biologisch aktiver Substanzen in oligo-multilamellaren oder unilamellaren Liposomen geeignet.

Ein besonderer Vorteil dieser pharmazeutischen Zubereitung auf der Basis vesikulärer Strukturen von $N^+$-Tensiden ist die hydrophobe Einkapselung von pharmazeutischen Wirkstoffen. Eine besonders vorteilhafte Folge der durch Ultraschallbehandlung und Gegenionen hergestellten großkalibrigen Vesikel ist darin zu sehen, daß die Gefahr des Austritts des pharmazeutischen Wirkstoffes aus der Bläschenhaut des Ansatzes reduziert oder eliminiert wird. Deshalb kann diese Form des quartären $N^+$-Tenside auf der Basis von sechsgliedrigen Heterozyklen, insbesondere für das Einkapseln hydrophober pharmazeutischer Wirkstoffe herangezogen werden, die dazu verwendet werden können, um lokale z.B. örtlich begrenzte statt systemische Wirkungen zu erzielen.

Während die meisten der bekannten Verfahren auf die Einkapselung hydrophiler Wirkstoffe beschränkt sind, kann mit dieser Erfindung die Einkapselung von hydrophoben pharmazeutischen Wirkstoffen vorgenommen werden. Versuche haben gezeigt, daß sogar anorganische lipophile pharma zeutische Wirkstoffe wie z.B. Quecksilber-II-Cyanid mit hoher Wirksamkeit eingeschlossen werden können und ihre pharmakodynamische Wirkung durch das Potenzierungsgemisch noch verstärkt werden kann.

Dieser Nachteil wird durch die neuen N-Tenside der allgemeinen Formel II und neuer Benzethonium-Verbindungen wie auch die Vesikel auf der Basis von $N^+$-Tensiden entweder durch micellaren Einschluß der pharmazeutischen Wirkstoffe oder durch kovalente Verknüpfung der Wirkstoffe mit dem $N^+$-Tensid unter Beibehaltung der äußeren morphologischen Form der gesamten Micelle aufgehoben.

Bekannt ist die bakterizide Wirkung von Chlorhexidin bei grampositiven und gramnegativen Bakterien, jedoch resistent gegen gramnegative Bazillen. Gefunden wurde, daß micellare Lösungen von quartären Ammoniumbasen gemäß der allgemeinen Formel I und insbesondere II die 2-4 Gew.% Chlorhexidin hydrophob im micellaren Kern gebunden halten, die Resistenz gegen gramnegative Bazillen aufgehoben

und ihre therapeutische Wirksamkeit im Verhältnis zum Chlorhexidin alleine verstärken. Die beobachteten Nebenwirkungen von Chlorhexidin wie Kontaktdermatiden, Hauteffekte topischer Art, Photosensibilität der Haut, finden bei verfahrensgemäßer Herstellung der micellaren Lösungen der N-Tenside der Formel I und II nicht statt.

Es ist Gegenstand der vorliegenden Erfindung, die eingangs erwähnte Heterogenität von Form und Größe der Micellen auch in Gegenwart von Potenzierungsgemischen abzustellen. Es wird somit gewährleistet, daß eine monodisperse Form von kationischen organischen Ammoniumbasen auch in Gegen wart von pharmazeutischen Wirkstoffen und Potenzierungsgemischen bei der Herstellung erreicht wird.

Diese erfindungsgemäßen quartären organischen Ammoniumbasen beseitigen die oben geschilderten Nachteile der bislang herkömmlichen Invertseifen. So besteht außerdem großes Interesse sowohl an der therapeutischen Verwendung von quartären Ammoniumbasen, die sowohl als pharmazeutischer Wirkstoff fungieren und als Träger von Wirkstoffen unterschiedlichster Art, z.B. antimikrobieller, antiviraler, antifungaler oder antineoplastischer Natur, micellar aufnehmen können. Sie sollen daher die eingangs genannten, vor allem milieu-bedingten Nachteile nicht besitzen.

Die erfindungsgemäßen, mit pharmazeutisch kovalent verbundenen Wirkstoffe, wie z.B. Pyrimidin-und Purinabkömmlinge am $N_1$ bzw. $N_7$, auf der Basis von quartären Ammoniumbasen, haben den Vorteil

1. daß diese maskierten Antimetabolite aus der Pyrimidin-bzw. Purin-Reihe, keine intramoleculare Wechselwirkungen anionischer bzw. kationischer Natur eingehen. Sie sind neutral geladen (z.B.kein Nukleotid-dianion durch das Phosphat) und können daher ungehindert in die pro-bzw. eukaryontische Zelle diffundieren, so daß hohe intrazelluläre Antimetabolit-(z.B. 5'-Nukleotid) Konzentrationen erreicht werden;

2. daß die pharmazeutischen Wirkstoffe durch N-C-Hydrolyse mittels der vorhandenen Enzymsysteme der germinalen bzw. eukaryontischen Zellen, erst am Target oder auch topisch freigesetzt werden;

3. durch die Erhöhung der Hydrophobizität der Alkyl-bzw. Aryl-Kette bzw. Restes am $N^+$-Tensid wird die Membran-Permeabilität erhöht, so daß die pharmazeutischen Wirkstoffe quantitativ passiv in das Zytosol übertreten können. Im Gegensatz zu Di-Anionen oder Kationen, welche die Membran unter physiologischen pH-Bedingungen und Ionenstärken schwer passieren können, ist dies bei den verfahrensgemäßen $N^+$-Tensiden ungehindert;

4. die hohe Hydrophobizität bedingt auch einen hohen Verteilungskoeffizient im System $CHCl_3$-$H_2O$ bei pH 8,0;

5. durch die konzentrierte Aufnahme von hydrophoben bzw. hydrophilen pharmazeutischen Wirkstoffen wird zusätzlich zu den kovalent verankerten und die Wirkstoffkonzentration nach Penetration durch die germinale Membran, fungale Zellwand (Hemmung der Chitinsynthetase) oder virale Phospholipid-Doppelmembran durch einen Konzentrationsgradienten (extrazellulär - intrazellulär) erhöht. Dadurch resultiert eine geringe Anflutungszeit.

Im Gegensatz zu Liposomen als Träger von pharmazeutischen Wirkstoffen, wie sie z.B. in der US-Patentschrift 3,993,754 oder in der europäischen Patentschrift 0,102,324 genannt sind, haben diese hier erfindungsgemäß beschriebenen Micellen von quartären Ammoniumbasen die Vorteile,

1. daß sie wasserunlösliche Wirkstoffe micellar im sogenannten "liquid core" aufnehmen können, und dadurch sowohl topisch als auch enteral durch Öffnen der Micelle diese wasserunlöslichen Wirkstoffe kontrolliert freisetzen können, z.B. Rimantadin, Amantadin, Tromantadin, die bei Influenza-Viren bzw. Herpes Simplex-Viren sowohl der Haut als auch des Auges wirksam sind.

2. wasserlösliche Wirkstoffe können sowohl im Sternlayer als auch micellar gelöst werden, wenn sie selber hydrophobe Bereiche haben, wie z.B. Polyen-Verbindungen, Tetrazykline, Aminoglykoside und aromatische Antimetabolite, Z.B. Trifluorthymidin, Viderabin, Cytarabin, 5-Jod und 5-Fluor-desoxyuridin, 5-Ethyl-2'-desoxyuridin, Erythromycin und Nalidixinsäure.

3. Die hier erfindungsgemäß beschriebenen kationischen N-Tenside haben zwei spezifische Bindungsstellen mit hohen Bindungskonstanten ($K_3$ = 10-15$\mu$M) und großer Bindungskapazität (Kapazität 100 $\mu$g/Micelle): die eine ist bedingt durch die hydrophoben Wechselwirkungen zwischen dem "liquid core" der Micelle und dem hydrophoben Bereich des Wirkstoffs ( G = 15-20kcal/Mol) als auch die $\pi$-$\pi$-Wechselwirkungen der hier beschriebenen Wirkstoffe zwischen den N-Heterozyklen der N-Tenside und den pharmazeutischen Wirkstoffen, die zweite Bindungsstelle ist unspezifischer Art und ist an der Grenzfläche zwischen Stern-layer und hydrophobem Kern lokalisiert. Die Bindungskonstante liegt im Bereich von $K_3$ = 20 mM, die Bindungskapazität 100-200 $\mu$g/Micelle. Die unspezifische Bindungsstellen liegen fast ausschließlich im Guy-Chapman-Layer. Im Gegensatz zu Liposomen, wo die Zahl der unspezifischen Bindungsstellen wesentlich höher ist, kann die Zahl der unspezifischen Bindungsstellen durch Zugabe von Ethanol/Glycerol ausgeschaltet werden, da die Kräfte, welche in dem Guy-Chapman-Layer wirken, durch Konzentrationen von Ethanol, Glycerin bis zu 30-35 Gew.% ausgeschaltet werden, ohne die Bindungskapazität, -stärke der hydrophoben Kräfte bzw. im Stern-layer zu beeinflussen (nur Polarität und Konfiguration).

4. Die hier beschriebene Erfindung hat den Vorteil, daß die micellar eingeschlossenen pharmazeutischen Wirkstoffe nicht wieder den micellaren Verband verlassen, wie z.B. bei Liposomen, die nach den bisher bekannten Verfahren "lecken". Die "Dichtigkeit" ("sealing") der vorliegenden Erfindung von micellar eingeschlossenen Wirkstoffen ist z.B. am Beispiel von micellar gebundenen radioaktiv markiertem Trifluorthymidin, Cytarabin und Idoxuridin nachzuweisen. So wurde u.a. gefunden, daß Idoxuridin erst nach 200 Tagen 5 Gew.% seiner ursprünglichen micellar eingeschlossenen Konzentration (2000µg) im Falle von Hexadecyl-pyridinium-oder Benzethonium-chlorid Micellen verliert. Die entsprechenden Werte für radioaktiv markiertes Trifluorthymidin und Cytarabin liegen bei 210 und 300 Tagen (20°).

5. Nach dem Verfahren der vorliegenden Erfindung lassen sich diese Micellen mit den eingeschlossenen anorganischen und organischen Wirkstoffen bei pH = 7,0 auf einfache Weise ohne großen apparativen Aufwand in wässriger Phase herstellen, welche kleine, einfache Micellen mit einem Durchmesser von ca. 50-100 Å und große Micellen mit einem Durchmesser von 600-10.000 Å -je nach Gegenion - enthalten. Außerdem werden durch ein Gemisch von Glycerol/Ethanol im Verhältnis von 2 Gew.% : 15 Gew.% gegenüber Wasser beide Micellen verschiedener Größenordnung sowohl in ihrer Form (Kugel, Hemizylinder, Stab, Diskus) als auch in ihrer Kompaktheit durch Senkung der KMK, wie auch durch Reduktion der Freien Energie der gesamten Micelle in der wäßrigen Phase infolge Verdünnung der Elektronendichte an der externen Oberfläche, stabilisiert. Mittels geeigneter Trennmethoden, z.B. HPLC, Ultrafiltration, Gelfiltration und/oder präparativer Zentrifugation kann man kleine von großen Micellen präparativ trennen.

6. Die Stabilität, Haltbarkeit und Lagerfähigkeit dieser so hergestellten Micellen aus quartären organischen Ammoniumbasen, pH = 7,0, gegenüber Temperatur, Dichtigkeit ("sealing and leaking") und Lagerfähigkeit ist durch die Einlagerung der pharmazeutischen Wirkstoffe in hydrophoben Kern der Micellen im Ver hältnis zu den Micellen ohne Wirkstoffe bei gleichen $Y^-$ erhöht. Im Gegensatz zu den Liposomen tritt hier kein Schmelzen bei höherer Temperatur (>40°C) ein, sondern bei verfahrensgemäßer Herstellung ändern sich die hydrodynamischen Verhältnisse erst ab >60°C. Da bei zunehmender Temperatur diese so hergestellten Micellen von quartären Ammoniumbasen eher eine Reduzierung im hydrodynamischen Radius eingehen, daher kompakter werden, sind diese Art Micellen thermodynamisch stabiler als künstliche Liposomen oder Liposomen + quartäre Ammoniumbasen. Diese Vorgänge sind leicht im Routine-Verfahren durch inelastische Lichtstreuung bei der Herstellung zu prüfen.

7. Die Hydrophobizität bzw. die Penetration der $H_2O$-Moleküle in diese so hergestellten Micellen und deren Beeinflussung durch anorganische pharmazeutische Wirkstoffe, z.B. $Hg(CN)_2$, ZnEDTA, $ZnSO_4$, ZnO, Wolframsäure-antimonate, $K_{18}(KW_{21}Sb_9O_{86})_{17}$, als auch der organischen Substanzen ließ sich durch NMR-Spektroskopie nachweisen:

Am Beispiel des 8-Ketohexadecylpyridinium-chlorid (8-KHPCl) kann man die erfindungsgemäße Anwendung der Aufnahme von pharmazeutischen Wirkstoffen demonstrieren. Folgerichtig wurde nunmehr gefunden, daß eine chemische Verschiebung von 146,6 ppm für eine 0,1 molare micellare Lösung in Wasser auftritt, die jedoch durch z.B. $Hg(CN)_2$ nach 147,2 ppm verschoben wird. Micellen in wäßriger Lösung, die 0,05 molar an 8-KHPCl und 0,2 molar an CPCl (Cetylpyridiniumchlorid) sind, ergaben allerdings eine chemische Verschiebung von 147,2 ppm für das $^{13}C$-Carbonyl-Signal von 8-KHPCl. Vergleicht man diese beiden Zahlen mit den Verschiebungen von 8-KHPCl in Methanol (145,7 ppm) und Acetonitril (144,0 ppm) wird deutlich, daß die CO-Gruppe in dieser Micelle eine weitgehend wäßrige Umgebung einnimmt. $Hg(CN)_2$ spielt hierbei eine doppelte Rolle, die damit auch die therapeutische Breite in vitro bestimmt: der hydrophobe Charakter des $Hg(CN)_2$ bewirkt eine hohe Löslichkeit im hydrophoben Kern von z.B. Hexadecylpyridiniumchlorid als Monmer und bedingt eine chemische Verschiebung von $\delta_{c8}$ 27,5 ppm $^{13}C$ der $CH_2$-Kette auf 32,5 ppm, während in 8-KHPCl-Micellen Quecksilber II-cyanid in der Nähe der Ketogruppe ($C_9$) als $Hg_2(CN)_4$ in $H_2O$ (siehe oben) gelöst ist und durch diese $H_2O$-Löslichkeit ist die Konzentration von $H_2$-$(CN)_4$ limitiert.

Abbildung 5 zeigt die Abhängigkeit der Extinktion der micellar eingeschlossenen anorganischen Wirkstoffe und des N-Phosphono-acetyl-L-aspartats in Hexadecylpyridiniumchlorid.

<u>Legenden zu den Abbildungen, soweit sie noch nicht in der Beschreibung enthalten sind.</u>

Ab b. 12:

1) Abhängigkeit des Stokes' Radius (hydrodynamischer Radius) von N-Cetyl-4-methyl-imidazoliumchlorid mit mizellar eingeschlossenem Rimantadin gemessen durch inelastische Laser-Lichtstreuung von der Temperatur

2) Abhängigkeit des Stokes' Radius von N-Hexadecyl-5-carboxamid-chlorid mit mizellar eingeschlossenen 5-Fluor-cytosin von der Temperatur

3) Abhängigkeit des Stokes' Radius von 2,-4-Dihydroxy-5-methyl-hexadecyl-pyridinium-chlorid mit mizellar eingeschlossenem Gentamycin von der Temperatur

Abb. 8:

1) Abhängigkeit des Stokes's Radius von Benzyldimethyl [2-[2-(p-1,1,3,3,tetramethylbutyl-p,p'-dimethyl-phenoxy) ethoxy]ethyl]ammonium-chlorid mit mizellar eingeschlossenem Viderabin von der Temperatur

2) Abhängigkeit des Stokes' Radius von Benzyldimethyl[2[2-(p-1,1,3,3,tetramethylbutyl-p,p'-dimethyl-phenoxy) ethoxy]ethyl]ammonium-chlorid mit 5-Tri-Fluor-thymedin von der Temperatur

Abb. 7:

1) Abhängigkeit des Stokes's Radius von 8-Ketohexadecylpyridinium-chlorid mit mizellar eingeschlossenem Z-Miconazol von der Temperatur

2) Abhängigkeit des Stokes' Radius von 8-Ketohexadecylpyridinium-chlorid mit mizellar eingeschlossenem Z-Miconazol + $Hg(CN)_2$ von der Temperatur

Abb. 11:

1) Abhängigkeit des Stokes' Radius von 3,5-bis [(n-hexadecyloxy) carbonyl] -N-methyl-pyridiniumchlorid mit lamellar eingeschlossenem Amantidin von der Temperatur; nicht ultraschallbehandelt

2) Abhängigkeit des Stokes' Radius von 3,5-bis [(n-hexadecyloxy) carbonyl] -N-methyl-pyridiniumchlorid mit lamellar eingeschlossenem Amantidin von der Temperatur; ultraschallbehandelt

3) Abhängigkeit des Stokes' Radius von 3,5-bis (n-hexadecyloxy) carbonyl -N-methyl-pyridiniumchlorid mit lamellar eingeschlossenem Rimantadin von der Temperatur; ultraschallbehandelt

Abb. 13:

1) Abhängigkeit des Stokes' Radius von N-Hexadecyl-pyridiniumchlorid und mizellar eingeschlossenem $Hg(CN)_2$ von der Temperatur; nicht ultraschallbehandelt

2) Abhängigkeit des Stokes' Radius von N-Hexadecyl-pyridiniumchlorid und mizellar eingeschlossenem $Hg(CN)_2$ von der Temperatur; ultraschallbehandelt

**Ansprüche**

1. Pharmazeutische Zubereitung,
dadurch gekennzeichnet,
daß sie aufgebaut ist aus einer Micelle, bestehend aus einem kationischen Tensid mit einem einwertigen Anion und einem hydrophoben pharmazeutischen Wirkstoff, dispergiert in einem Lösungsmittel, dessen pH-Wert kleiner ≦ 7 ist, wobei die kritische Micellbildungskonzentration (KMK) im Bereich von $1{,}0 \, . \, 10^{-7}$ bis $1{,}5 \, . \, 10^{-4}$ Mol/Liter liegt.

2. Pharmazeutische Zubereitung,
nach Anspruch 1
dadurch gekennzeichnet,
daß sie aufgebaut ist aus einer Micelle, bestehend aus einem kationischen Tensid mit einem einwertigen Anion in einer Menge von 0,01 bis 0,1 Gewichts.% bezogen auf die gesamte pharmazeutische Zubereitung, und einem hydrophoben pharmazeutischen Wirkstoff in einer Menge von 0,001 bis 0,5 Gew.% bezogen auf die gesamte pharmazeutische Zubereitung, dispergiert in einem Lösungsmittel, dessen pH-Wert ≦ 7,0 ist, in einer Menge von 99,40 bis 99,989 Gew.% bezogen auf die gesamte pharmazeutische Zubereitung, wobei die kritische Micellbildungskonzentration (KMK) im Bereich von $1{,}0 \, . \, 10^{-7}$ Mol/l bis $1{,}5 \, . \, 10^{-4}$ Mol/l liegt.

3. Pharmazeutische Zubereitung, nach Anspruch 1 oder 2, dadurch gekennzeichnet, daß das kationische Tensid eine Verbindung der allgemeinen Formel

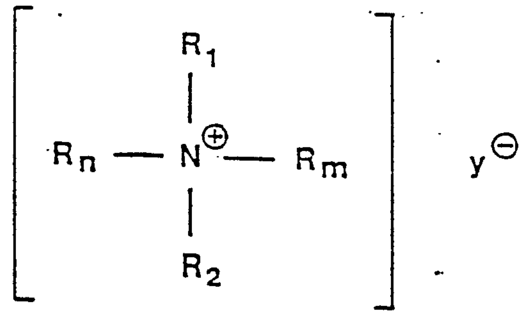

ist, wobei
$R_1$ = ein Alkylrest mit 1 - 12 C-Atomen oder ein Aralkylrest,
$R_2$ = ein Alkylrest mit 1 - 12 C-Atomen oder ein Aralkylrest,
$R_n$ = ein geradkettiger oder verzweigter Alkylrest, substituiert oder nicht substituiert, mit 1 - 22 C-Atomen, vorzugsweise 10 - 20 C-Atomen oder ein Alkenylrest mit 8 - 20 C-Atomen, vorzugs weise 8 - 10 C-Atomen oder ein 5-oder 6-gliedriger aromatischer Heterozyklus mit einem oder 2 Stickstoffatomen, und wahlweise eine Schwefelatom oder einem Sauerstoffatom und
$R_m$ = ein geradkettiger oder verzweigter Alkylrest, substituiert oder nicht substituiert, mit 1 - 22 C-Atomen, vorzugsweise 10 - 20 C-Atomen oder ein Alkenylrest mit 8 - 20 C-Atomen, vorzugsweise 8 - 10 C-Atomen oder ein 5-oder 6-gliedriger aromatischer Heterozyklus mit einem oder 2 Stickstoffatomen, und wahlweise einem Schwefelatom oder einem Sauerstoffatom oder ein Chinoliniumrest und
$y^-$ = ein einwertiges Anion ist.

4. Pharmazeutische Zubereitung nach einem oder mehreren der vorhergehenden Ansprüche, dadurch gekennzeichnet, daß $R_1$ ein Alkylrest mit 6 C-Atomen ist.

5. Pharmazeutische Zubereitung nach einem oder mehreren der vorhergehenden Ansprüche, dadurch gekennzeichnet, daß $R_2$ ein Alkylrest mit 6 C-Atomen ist.

6. Pharmazeutische Zubereitung nach einem oder mehreren der vorhergehenden Ansprüche, dadurch gekennzeichnet, daß $R_n$ ein geradkettiger oder verzweigter Alkylrest mit 12 bis 16 C-Atomen ist.

7. Pharmazeutische Zubereitung nach einem oder mehreren der vorhergehenden Ansprüche, dadurch gekennzeichnet, daß $R_m$ ein geradkettiger oder verzweigter Alkylrest mit 12 bis 16 C-Atomen ist.

8. Pharmazeutische Zubereitung nach einem oder mehreren der vorhergehenden Ansprüche, dadurch gekennzeichnet, daß $R_n$ ein geradkettiger oder verzweigter Alkylrest mit 10 C-Atomen ist.

9. Pharmazeutische Zubereitung nach einem oder mehreren der vorhergehenden Ansprüche, dadurch gekennzeichnet, daß $R_m$ ein geradkettiger oder verzweigter Alkylrest mit 10 C-Atomen ist.

10. Pharmazeutische Zubereitung nach einem oder mehreren der vorhergehenden Ansprüche, dadurch gekennzeichnet, daß $R_n$ ein geradkettiger Alkenylrest mit 10 C-Atomen ist.

11. Pharmazeutische Zubereitung nach einem oder mehreren der vorhandenen Ansprüche, dadurch gekennzeichnet, daß $R_n = R_1 = R_2$ ein Methylrest ist.

12. Pharmazeutische Zubereitung nach einem oder mehreren der vorhergehenden Ansprüche, dadurch gekennzeichnet, daß $R_n$ der n-Hexadecyl (Cetyl)-Rest ist.

13. Pharmazeutische Zubereitung nach einem oder mehreren der vorhergehenden Ansprüche, dadurch gekennzeichnet, daß $R_m$ ein n-Hexadecyl (Cetyl)-Rest ist.

14. Pharmazeutische Zubereitung nach einem oder mehreren der vorhergehenden Ansprüche, dadurch gekennzeichnet, daß $R_n$ 2-oder 4-Methyl oder 2-oder 4-Ethylpyridinium ist.

15. Pharmazeutische Zubereitung nach einem oder mehreren der vorhergehenden Ansprüche, dadurch gekennzeichnet, daß $R_n$ 2-Methyl-oder 2-Ethylimidazolinium ist.

16. Pharmazeutische Zubereitung nach einem oder mehreren der vorhergehenden Ansprüche, dadurch gekennzeichnet, daß $R_n$ 2-Methyl-8-chlorchinolinium ist.

17. Pharmazeutische Zubereitung nach einem oder mehreren der vorhergehenden Ansprüche, dadurch gekennzeichnet, daß $R_m$ 2-oder 4-Methyl oder 2-oder 4-Ethylpyridinium ist.

18. Pharmazeutische Zubereitung nach einem oder mehreren der vorhergehenden Ansprüche, dadurch gekennzeichnet, daß $R_m$ 2-Methyl-oder 2-Ethylimidazolinium ist.

19. Pharmazeutische Zubereitung nach einem oder mehreren der vorhergehenden Ansprüche, dadurch gekennzeichnet, daß $R_m$ 2-Methyl-8-chlorchinolinium ist.

20. Pharmazeutische Zubereitung nach einem oder mehreren der vorhergehenden Ansprüche, dadurch gekennzeichnet, daß das einwertige Anion Chlorid, Bromid, Jodid, Formiat, Acetat, Propionat, Hydrogensulfat, Malat, Fumarat, Salizylat, Alginat, Glukonat oder Ethylsulfat ist.

21. Pharmazeutische Zubereitung nach einem oder mehreren der vorhergehenden Ansprüche, dadurch gekennzeichnet, daß das kationische Tensid die Formel

$$\left[ \mathrm{HET} \equiv \mathrm{N}^{\oplus} - (\mathrm{CH_2})_x - \mathrm{CH_3} \right] \; y^{\ominus}$$

besitzt, wobei
Het≡ $N^+$-ein substituierter oder nichtsubstituierter Pyridiniumrest oder
ein substituierter oder nicht substituierter Pyrimidiniumrest oder
ein substituierter Pyrazin-(1,4-Diazinium)rest oder
ein Imidazoliumrest (4,5-d)pyrimidin Rest substituiert oder nicht substituiert, oder
ein substituierter oder nicht substituierter Imidazolium-Rest oder
ein substituierter oder nicht substituierter Pyrazoliumrest, oder
ein substituierter oder nicht substituierter Thiazoliumrest, oder
ein substituierter oder nicht substituierter Benz-thiazoliumrest, oder
ein substituierter oder nicht substituierter Benz-imidazoliumrest,
x = 8 bis 20 und
$y^-$ = die Anionen gemäß dem Patentanspruch 20
bedeuten.

22. Pharmazeutische Zubereitung nach einem oder mehreren der vorhergehenden Ansprüche, dadurch gekennzeichnet, daß das kationische Tensid ein N-Alkyl-pyridinium der Formel

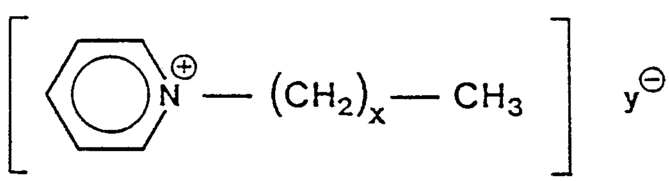

ist, wobei $y^-$ die Anionen gemäß dem Patentanspruch 20 bedeuten.

23. Pharmazeutische Zubereitung nach einem oder mehreren der vorhergehenden Ansprüche, dadurch gekennzeichnet,
daß das kationische Tensid ein Hexadecylpyridinium der Formel

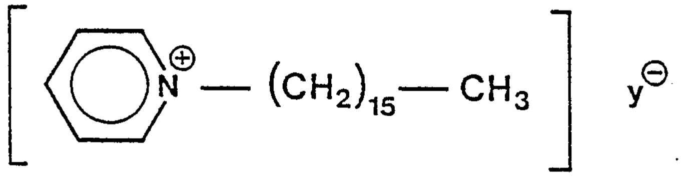

ist, wobei $y^-$ die Anionen gemäß dem Patentanspruch 20 bedeuten.

24. Pharmazeutische Zubereitung nach einem oder mehreren der vorhergehenden Ansprüche, dadurch gekennzeichnet,
daß das kationische Tensid ein N-Alkyl-4 hydroxypyridinium der Formel

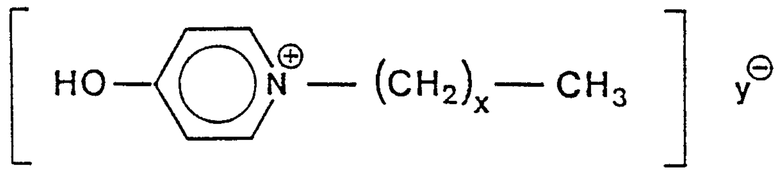

ist, wobei $y^-$ die Anionen gemäß dem Patentanspruch 20 bedeuten.

25. Pharmazeutische Zubereitung nach einem oder mehreren der vorhergehenden Ansprüche, dadurch gekennzeichnet,
daß das kationische Tensid ein Hexadecyl-4-hydroxypyridinium der Formel

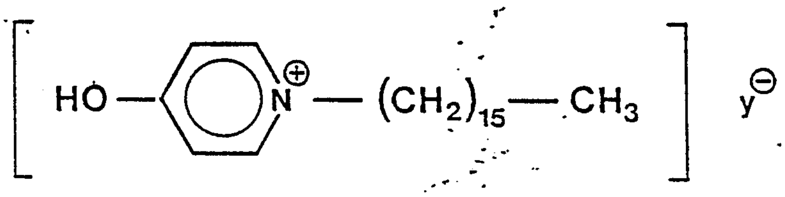

ist, wobei $y^-$ die Anionen gemäß dem Patentanspruch 20 bedeuten.

26. Pharmazeutische Zubereitung nach einem oder mehreren der vorhergehenden Ansprüche, dadurch gekennzeichnet,
daß die kationischen Tenside 2,5,6 substituierte $N_1$-Alkyl-pyrimidinium-Verbindungen der Formel

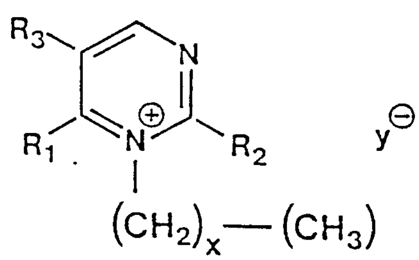

$R_1 = R_2 = R_3 = H$

$R_1 = NH_2$; $R_2 = OH$; $R_3 = H$

$R_1 = NH_2$; $R_2 = OH$; $R_3 = H$

$R_1 = OH$; $R_2 = OH$; $R_3 = CH_3$

$R_1 = OH$; $R_2 = OH$; $R_3 = H$

$R_1 = F$; $R_2 = OH$; $R_3 = H$

$R_1 = OH$; $R_2 = OH$; $R_3 = F$

sind, wobei $y^-$ die Anionen gemäß dem Patentanspruch 20 bedeuten.

27. Pharmazeutische Zubereitung nach einem oder mehreren der vorhergehenden Ansprüche, dadurch gekennzeichnet,
daß das kationische Tensid ein 2,5,6 substituiertes $N_1$-Hexadecylpyrimidinium der Formel

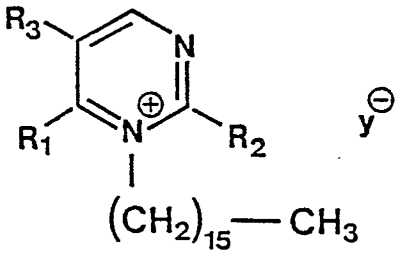

$R_1 = R_2 = R_3 = H$

$R_1 = NH_2$ ; $R_2 = OH$ ; $R_3 = H$

$R_1 = NH_2$ ; $R_2 = OH$ ; $R_3 = H$

$R_1 = OH$ ; $R_2 = OH$ ; $R_3 = CH_3$

$R_1 = OH$ ; $R_2 = OH$ ; $R_3 = H$

$R_1 = F$ ; $R_2 = OH$ ; $R_3 = H$

$R_1 = OH$ ; $R_2 = OH$ ; $R_3 = F$

ist, wobei y⁻ die Anionen gemäß dem Patentanspruch 20 bedeuten.

28. Pharmazeutische Zubereitung nach einem oder mehreren der vorhergehenden Ansprüche, dadurch gekennzeichnet, daß das kationische Tensid ein 4-n-Alkyl-pyrazinium-2-carboxamid der Formel

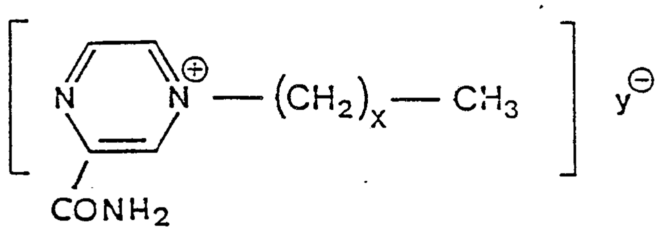

ist wobei y⁻ die Anionen gemäß dem Patentanspruch 20 bedeuten.

29. Pharmazeutische Zubereitung nach einem oder mehreren der vorhergenden Ansprüche, dadurch gekennzeichnet, daß das kationische Tensid ein 4-Hexadecylpyrazinium-2-carboxamid der Formel

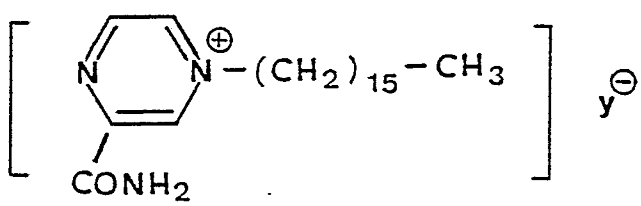

ist, wobei y⁻ die Anionen gemäß dem Patentanspruch 20 bedeuten.

30. Pharmazeutische Zubereitung nach einem oder mehreren der vorhergehenden Ansprüche, dadurch gekennzeichnet, daß das kationische Tensid ein 7-n-Alkyl-imidazolium [4,5-d]-pyrimidin der Formel

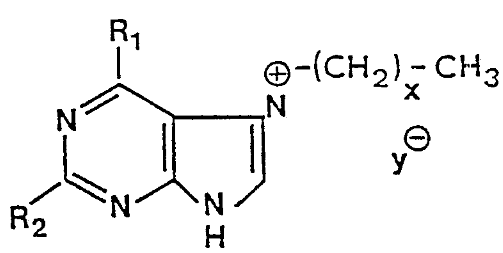

$R_1 = OH$ ; $R_2 = OH$

$R_1 = H$ ; $R_2 = H$

$R_1 = F$ ; $R_2 = NH_2$

$R_1 = F$ ; $R_2 = OH$

$R_1 = NH_2$ ; $R_2 = H$

$R_1 = NH_2$ ; $R_2 = NH_2$

ist, wobei $y^-$ die Anionen gemäß dem Patentanspruch 20 bedeuten.

31. Pharmazeutische Zubereitung nach einem oder mehreren der vorhergehenden Ansprüche, dadurch gekennzeichnet,
daß das kationische Tensid ein 7-Hexadecylimidazolium [4,5-d]pyrimidin der Formel

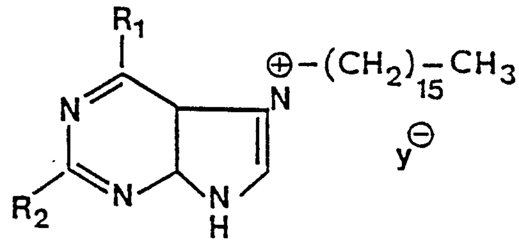

$R_1 = OH$ ; $R_2 = OH$
$R_1 = H$ ; $R_2 = H$
$R_1 = F$ ; $R_2 = NH_2$
$R_1 = F$ ; $R_2 = OH$
$R_1 = NH_2$ ; $R_2 = H$
$R_1 = NH_2$ ; $R_2 = NH_2$

ist, wobei $y^-$ die Anionen gemäß dem Patentanspruch 20 bedeuten.

32. Pharmazeutische Zubereitung nach einem oder mehreren der vorhergehenden Ansprüche, dadurch gekennzeichnet,
daß die kationischen Tenside 3-n-Alkyl-5 6-substituierte Benzimidazolium-Verbindungen der Formel

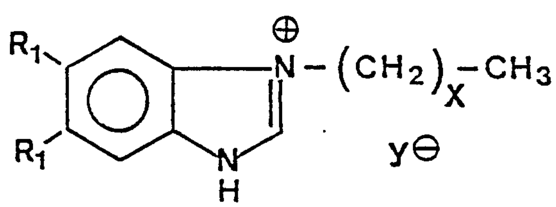

$R_1 = OH$

sind, wobei $y^-$ die Anionen gemäß dem Patentanspruch 20 bedeuten.

33. Pharmazeutische Zubereitung nach einem oder mehreren der vorhergehenden Ansprüche, dadurch gekennzeichnet,
daß die kationischen Tenside 3-Hexadecyl-5,6-substituierte Benzimidazolium-Verbindungen der Formel

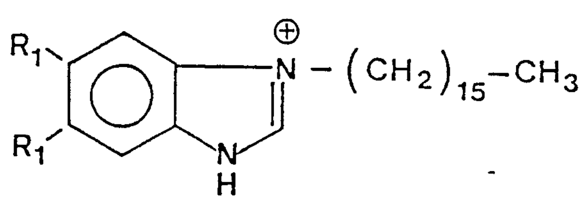

$R_1 = OH$

besitzt, wobei $y^-$ die Anionen gemäß dem Patentanspruch 20 bedeuten.

34. Pharmazeutische Zubereitung nach einem oder mehreren der vorhergehenden Ansprüche, dadurch gekennzeichnet,
daß die kationischen Tenside 4-substituierte n-Alkyl-2-pyrazolium-Verbindungen der Formel

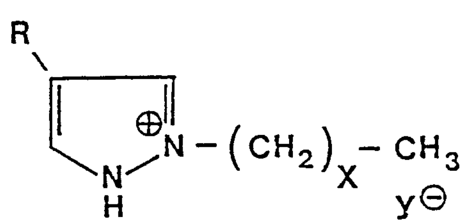

$R = H$ ; $CH_3$; $OH$

sind, wobei y⁻ die Anionen gemäß dem Patentanspruch 20 bedeuten.

35. Pharmazeutische Zubereitung nach einem oder mehreren der vorhergehenden Ansprüche, dadurch gekennzeichnet,
daß die kationischen Tenside 4-substituierte 2-Hexadecylpyrazolium-Verbindungen der Formel

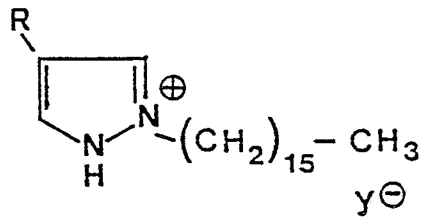

R = H ; $CH_3$; OH

besitzt, wobei y⁻ Anionen gemäß dem Patentanspruch 20 bedeuten.

36. Pharmazeutische Zubereitung nach einem oder mehreren der vorhergehenden Ansprüche, dadurch gekennzeichnet,
daß die kationischen Tenside 1-n-Alkyl-4-substituierte Imidazolium-Verbindungen der Formel

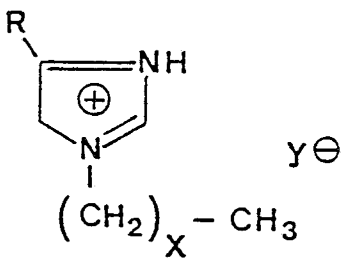

R = H ; $CH_3$ ;

besitzt, wobei y⁻ die Anionen gemäß dem Patentanspruch 20 bedeuten.

37. Pharmazeutische Zubereitung nach einem oder mehreren der vorhergehenden Ansprüche, dadurch gekennzeichnet,
daß die kationischen Tenside 1-Hexadecyl-4-substituierte Imidazolium-Verbindungen der Formel

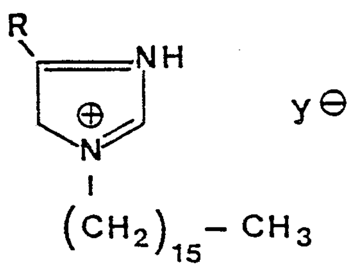

R = H ; $CH_3$ ;

besitzt, wobei y⁻ die Anionen gemäß dem Patentanspruch 20 bedeuten.

38. Pharmazeutische Zubereitung nach einem oder mehreren der vorhergehenden Ansprüche, dadurch gekennzeichnet,
daß die kationischen Tenside 3-n-Alkyl-5,6-substituierte Thiazolium-Verbindungen der Formel

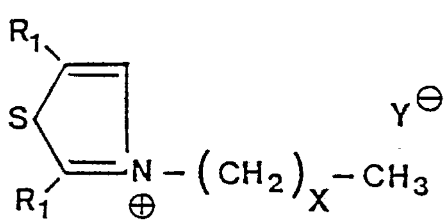

$R_1$ = H ;

$R_1$ = $CH_3$ ;

besitzt, wobei y⁻ die Anionen gemäß dem Patentanspruch 20 bedeuten.

39. Pharmazeutische Zubereitung nach einem oder mehreren der vorhergehenden Ansprüche, dadurch gekennzeichnet,
daß die kationischen Tenside 3-Hexadecyl-5,6-substituierte Thiazolium-Verbindungen der Formel

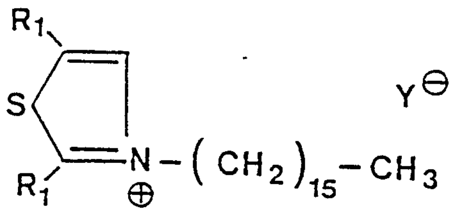

$R_1 = H$;

$R_1 = CH_3$;

besitzt, wobei $y^-$ die Anionen gemäß dem Patentanspruch 20 bedeuten.

40. Pharmazeutische Zubereitung nach einem oder mehreren der vorhergehenden Ansprüche, durch gekennzeichnet,
daß die kationischen Tenside 3-n-Alkyl-5,6-substituierte-Benzthiazolium-Verbindungen der Formel

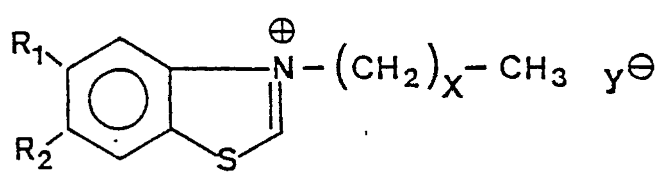

$R_1 = R_2 = H$

$R_1 = CH_3$

$R_1 = R_2 = OH$

$R_1 = R_2 = CH_3$

sind, wobei $y^-$ die Anionen gemäß dem Patentanspruch 20 bedeuten.

41. Pharmazeutische Zubereitung nach einem oder mehreren der vorhergehenden Ansprüche, dadurch gekennzeichnet,
daß die kationischen Tenside 4-[1,1bis n-Alkyl (Niederalkyl)] N-Hexadecylpyridinium-Verbindungen der Formel

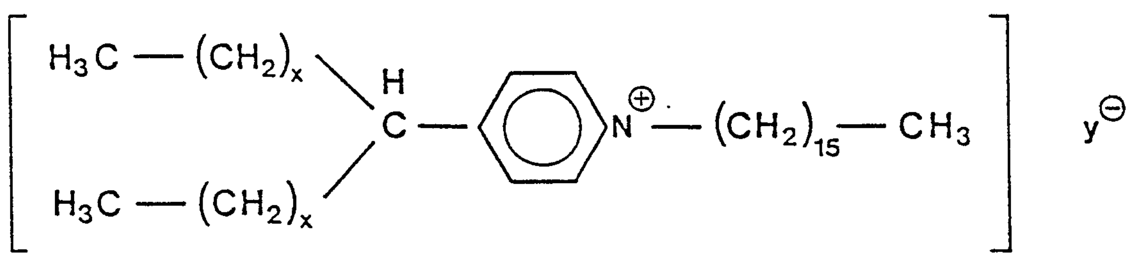

sind, wobei $y^-$ die Anionen gemäß dem Patentanspruch 20 bedeuten.

42. Pharmazeutische Zubereitung nach einem oder mehreren der vorhergehenden Ansprüche, dadurch gekennzeichnet,
daß die kationischen Tenside 3,5-bis [(n-Alkyloxy)carbonyl] N-Hexadecylpyridinium-Verbindungen der Formel

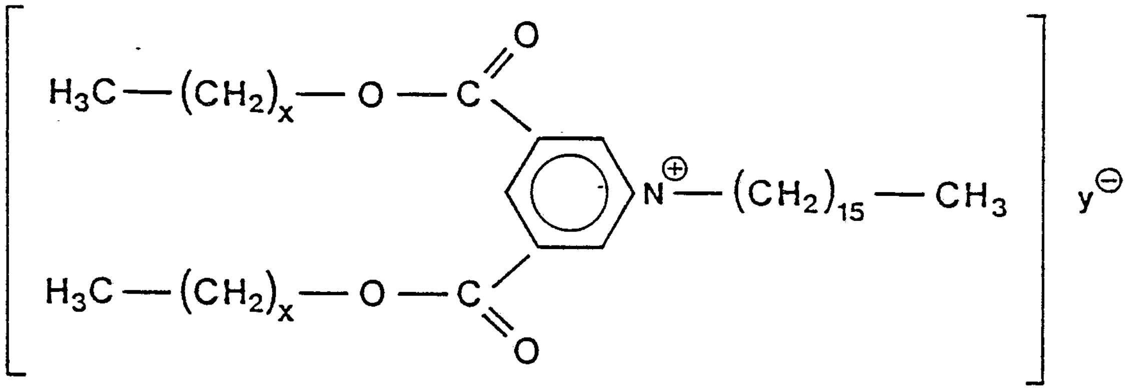

sind, wobei die y⁻ die Anionen gemäß dem Anspruch 20 bedeuten.

43. Pharmazeutische Zubereitung nach einem oder mehreren der vorhergehenden Ansprüche, dadurch gekennzeichnet,
daß das kationische Tensid ein 4-(17-tritriacontyl)-N-methyl-pyridiniumchlorid der Formel

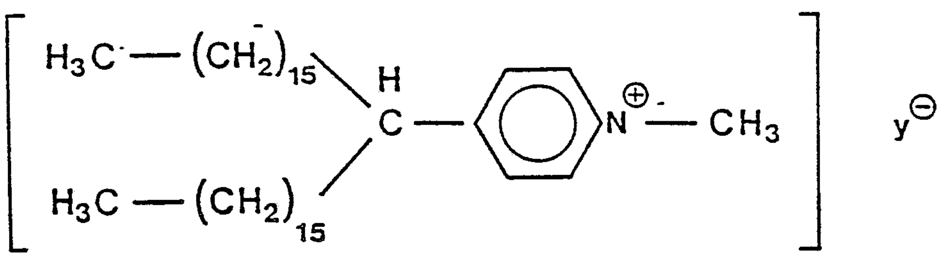

ist.

44. Pharmazeutische Zubereitung nach einem oder mehreren der vorhergehenden Ansprüche, dadurch gekennzeichnet,
daß das kationische Tensid ein 3,5-bis[(n-hexadecyloxy)carbonyl)]-N-methyl-pyridiniumchlorid der Formel

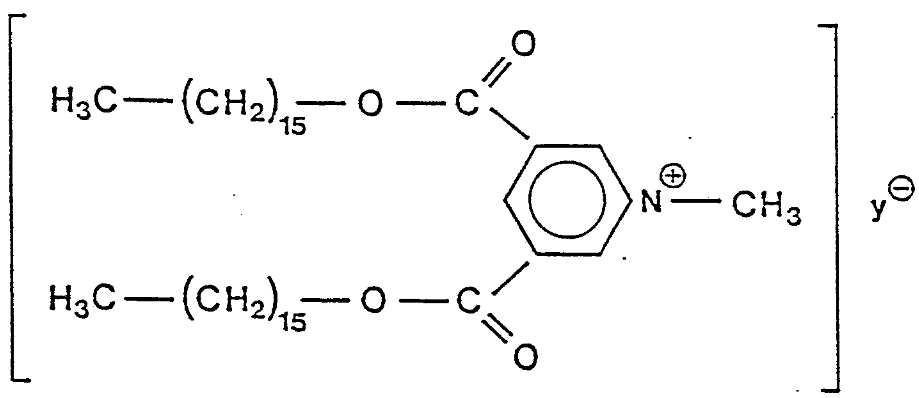

ist.

45. Pharmazeutische Zubereitung nach einem oder mehreren der vorhergehenden Ansprüche, dadurch gekennzeichnet,
daß das kationische Tensid die Formel

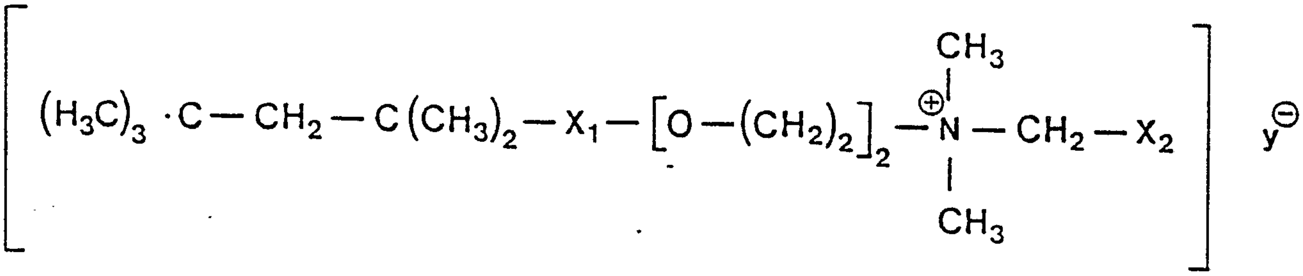

besitzt, wobei

$x_1$ = ein nichtsubstituierter oder in der 4-Stellung oder in der 3,5-Stellung oder in der 1,2,4,5-Stellung substituierter Phenylrest,

$x_2$ = ein nichtsubstituierter oder in der 4-Stellung oder in der 3,5-Stellung oder in der 1,2,4,5-Stellung substituierter Phenylrest und

$y^-$ = die Anionen gemäß dem Patentanspruchen 20 bedeuten.

46. Pharmazeutische Zubereitung nach einem oder mehreren der vorhergehenden Ansprüche, dadurch gekennzeichnet, daß die kritische Micellbildungskonzentration (KMK) im Bereich von 1,0 bis 8,5 . $10^{-7}$ Mol/l liegt.

47. Pharmazeutische Zubereitung nach einem oder mehreren der vorhergehenden Ansprüche, dadurch gekennzeichnet, daß das kationische Tensid mit einem einwertigen Anion in einer Menge von 0,o5 bis 0,1 Gew.% bezogen auf die gesamte pharmazeutische Zubereitung enthalten ist.

48. Pharmazeutische Zubereitung nach einem oder mehreren der vorhergehenden Ansprüche, dadurch gekennzeichnet, daß das kationische Tensid mit einem einwertigen Anion in einer Menge von 0,08 bis 0,1 Gew.% bezogen auf die gesamte pharmazeutische Zubereitung enthalten ist.

49. Pharmazeutische Zubereitung nach einem oder mehreren der vorhergehenden Ansprüche, dadurch gekennzeichnet, daß der hydrophobe pharmazeutische Wirkstoff in einer Menge von 0,06 bis 0,5 Gew.% bezogen auf die gesamte pharmazeutische Zubereitung enthalten ist.

50. Pharmazeutische Zubereitung nach einem oder mehreren der vorhergehenden Ansprüche, dadurch gekennzeichnet, daß der hydrophobe pharmazeutische Wirkstoff in einer Menge von 0,001 bis 0,005 Gew.% bezogen auf die gesamte pharmazeutische Zubereitung enthalten ist.

51. Pharmazeutische Zubereitung nach einem oder mehreren der vorhergehenden Ansprüche, dadurch gekennzeichnet, daß das Lösungsmittel Wasser ist.

52. Pharmazeutische Zubereitung nach einem oder mehreren der vorhergehenden Ansprüche, dadurch gekennzeichnet, daß das Lösungsmittel Wasser + Glycerin ist.

53. Pharmazeutische Zubereitung nach einem oder mehreren der vorhergehenden Ansprüche, dadurch gekennzeichnet, daß das Lösungsmittel Wasser + Glycerin + Ethanol ist.

54. Pharmazeutische Zubereitung nach einem oder mehreren der vorhergehenden Ansprüche, dadurch gekennzeichnet, daß das einwertige Anion ein monobasischer oder dibasischer Fettsäurerest ist.

55. Pharmazeutische Zubereitung nach einem oder mehreren der vorhergehenden Ansprüche, dadurch gekennzeichnet, daß das einwertige Anion Acetat, Propionat, Fumarat, Maleat, Succinat, Aspartat oder Glutamat ist.

56. Pharmazeutische Zubereitung nach einem oder mehreren der vorhergehenden Ansprüche, dadurch gekennzeichnet, daß das einwertige Anion ein Zuckerrest ist.

57. Pharmazeutische Zubereitung nach einem oder mehreren der vorhergehenden Ansprüche, dadurch gekennzeichnet, daß das einwertige Anion Glukonat, Galacturonat oder Alginat ist.

58. Pharmazeutische Zubereitung nach einem oder mehreren der vorhergehenden Ansprüche, dadurch gekennzeichnet, daß das einwertige Anion Chlorid, Bromid, Jodid, oder Hydrogensulfat ist.

59. Pharmazeutische Zubereitung nach einem oder mehreren der vorhergehenden Ansprüche, dadurch gekennzeichnet, daß der hydrophobe pharmazeutische Wirkstoff ein antimikrobieller Wirkstoff ist.

60. Pharmazeutische Zubereitung nach einem oder mehreren der vorhergehenden Ansprüche, dadurch gekennzeichnet, daß der hydrophobe pharmazeutische Wirkstoff ein antifungaler Wirkstoff ist.

61. Pharmazeutische Zubereitung nach einem oder mehreren der vorhergehenden Ansprüche, dadurch gekennzeichnet, daß der hydrophobe pharmazeutische Wirkstoff ein antiproliferativer Wirkstoff ist.

62. Pharmazeutische Zubereitung nach einem oder mehreren der vorhergehenden Ansprüche, dadurch gekennzeichnet, daß der hydroprobe pharmazeutische Wirkstoff ein antivi raler Wirkstoff ist.

63. Pharmazeutische Zubereitung nach einem oder mehreren der vorhergehenden Ansprüche, dadurch gekennzeichnet, daß der hydrophobe pharmazeutische Wirkstoff eine anorganische Verbindung der Elemente Zink oder Quecksilber oder Wolfram und/oder Antimon ist.

64. Pharmazeutische Zubereitung nach einem oder mehreren der vorhergehenden, Ansprüche, dadurch gekennzeichnet, daß die anorganische Verbindung $Z_nSO_4$ ist.

65. Pharmazeutische Zubereitung nach einem oder mehreren der vorhergehenden Ansprüche, dadurch gekennzeichnet, daß die anorganische Verbindung $Z_n$ O ist.

66. Pharmazeutische Zubereitung nach einem oder mehreren der vorhergehenden Ansprüche, dadurch gekennzeichnet, daß die anorganische Verbindung $Hg(CH)_2$ ist.

67. Pharmazeutische Zubereitung nach einem oder mehreren der vorhergehenden Ansprüche, dadurch gekennzeichnet, daß die anorganische Verbindung $(NH_4)_{18}$ $(NaW_{21}Sb_9O_{86})_{17}$ ist.

68. Pharmazeutische Zubereitung nach einem oder mehreren der vorhergehenden Ansprüche, dadurch gekennzeichnet, daß die anorganische Verbindung ein Alkali-oder Erdalkalisalz der Phosphonsäure $ROP(O)Me_2$ ist.

69. Pharmazeutische Zubereitung nach einem oder mehreren der vorhergehenden Ansprüche, dadurch gekennzeichnet, daß die anorganische Verbindung ein N-Phosphonoacetyl-1-aspartat ist.

70. Pharmazeutische Zubereitung nach einem oder mehreren der vorhergehenden Ansprüche, dadurch gekennzeichnet, daß der hydrophobe pharmazeutische Wirkstoff ein Antibiotikum ist.

71. Pharmazeutische Zubereitung nach einem oder mehreren der vorhergehenden Ansprüche, dadurch gekennzeichnet, daß das Antibiotikum Daunomycin und/oder Adriamycin und/oder Canamycin und/oder Gentamycin und/oder Neomcyin und/oder Polymyxine und/oder Tobramycin und/oder Amikacin und/oder Tetracyclin und/oder Chloramphenicol und/oder Erythromcyin und/oder Clindamycin und/oder Rifampicin und/oder Bacitrazin und/oder Thyrotrozin ist.

72. Pharmazeutische Zubereitung nach einem oder mehreren der vorhergehenden Ansprüche, dadurch gekennzeichnet, daß der hydrophobe pharmazeutische Wirkstoff ein antiviraler Wirkstoff ist.

73. Pharmazeutische Zubereitung nach einem oder mehreren der vorhergehenden Ansprüche, dadurch gekennzeichnet, daß der antivirale Wirkstoff Idoxuridin und/oder 5-Ethyl-2'-desoxyuridin und/oder Trifluorthymidin und/oder Ribavirin und/oder Amantadin und/oder Rimantadin und/oder Vidarabin und/oder 2,6-di-amino-kuban und/oder 1,1':3,3'-Bis-cyclobutan und/oder Dehydrokuban und/oder 2,6-Di-amino des Kubans ist.

74. Pharmazeutische Zubereitung nach einem oder mehreren der vorhergehenden Ansprüche, dadurch gekennzeichnet, daß der hydrophobe pharmazeutische Wirkstoff ein antifungaler Wirkstoff ist.

75. Pharmazeutische Zubereitung nach einem oder mehreren der vorhergehenden Ansprüche, dadurch gekennzeichnet, daß der antifungale Wirkstoff 5-Fluorcytosin und/oder Clotrimazol und/oder Econazol und/oder Miconazol und/oder Oxyconazol (Z-Form) und/oder Amphotericin B und/oder Nystatin und/oder $Z_n$ O.EDTA und/oder $Hg_2(CN)_4$ und/oder Polyoxinen ist.

76. Pharmazeutische Zubereitung nach einem oder mehreren der vorhergehenden Ansprüche, dadurch gekennzeichnet, daß der hydrophobe pharmazeutische Wirkstoff ein antineoplastischer Wirkstoff ist.

77. Pharmazeutische Zubereitung nach einem oder mehreren der vorhergehenden Ansprüche, dadurch gekennzeichnet, daß der antineoplastische Wirkstoff 5-Fluorcytosin und/oder $Hg(CH)_2$ und/oder $Hg(CH)_2$.(Ascorbat)$_4$ oder Hg-$(CH)_2$.(Acetylacetonat)$_4$ und/oder Azauridin und/oder Cytarabin und/oder Azaribin und/oder 6-Merkaptopurin und/oder Desoxycoformycin und/oder Azathioprin und/oder Thioguanin und/oder Vinblastin und/oder Vincristin und/oder Daunorubicin und/oder Doxorubicin ist.

78. Pharmazeutische Zubereitung nach einem oder mehreren der vorhergehenden Ansprüche, dadurch gekennzeichnet, daß das Lösungsmittel Wasser und/oder Ethanol und/oder Glycerin ist.

79. Pharmazeutische Zubereitung nach einem oder mehreren der vorhergehenden Ansprüche, dadurch gekennzeichnet, daß das Lösungsmittel Wasser und/oder Ethanol und/oder Dimethylsulfoxid (DMSO) ist.

80. Pharmazeutische Zubereitung nach einem oder mehreren der vorhergehenden Ansprüche, dadurch gekennzeichnet, daß x in der allgemeinen Formel des kationischen Tensids in Anspruch 21 den numerischen Wert 14 hat.

81. Pharmazeutische Zubereitung nach einem oder mehreren der vorhergehenden Ansprüche, dadurch gekennzeichnet, daß zur ihrer Herstellung die kationischen Tenside der allgemeinen Formel

$$[HET{\equiv}N^+ -(CH_2)_{14}-CH_3]\ Y^-$$

verwendet werden, wobei HET ≡ $N^+$-und $Y^-$ die in Anspruch 21 angegebenen Bedeutungen haben.

82. Pharmazeutische Zubereitung nach einem oder mehreren der vorhergehenden Ansprüche, dadurch gekennzeichnet, daß der pH-Wert des Lösungsmittels gleich 5 ist.

83. Verfahren zur Herstellung der pharmazeutischen Zubereitung nach einem der Ansprüche 1 - 82, dadurch gekennzeichnet, daß zunächst in einem Reaktionsgefäß das Lösungsmittel vorgelegt wird, dann das kationische Tensid unter Rühren bei Zimmertemperature zugegeben wird, dann zur entstandenen isotropen micellaren Lösung der hydrophobe pharmazeutische Wirkstoff unter Rühren bei Zimmertemperatur zugegeben wird und bis zu dessen vollständiger Lösung weitergerührt wird.

84. Verfahren zur Herstellung der N-alkylierten quaternären stickstoffhaltigen Heterozyklen nach einem oder mehreren der vorhergehenden Ansprüche, dadurch gekennzeichnet, daß der zu alkylierende Heterozyklus in einem geeigneten Lösungsmittel gelöst wird, anschließend unter stetem Rühren die stöchiometrische Menge n-Alkyl-halogenid zugegeben wird, anschließend unter stetem Rühren am Rückfluß über eine geraume Zeit erhitzt wird, wonach das Endprodukt nach dem Abkühlen ausfällt.

85. Verfahren nach einem oder mehreren der vorhergehenden Ansprüche, dadurch gekennzeichnet, daß der zu alkylierende unsubstituierte Heterozyklus in einem geeigneten Lösungsmittel gelöst wird, die stöchiometrische Menge n-Alkyl-halogenid zugegeben wird, anschließend unter Druck bei 100°C für eine Reaktionsdauer von 8 Stunden die Reaktion vollendet wird, wonach das Endprodukt nach dem Abkühlen ausfällt.

86. Verfahren zur Herstellung nach einem oder mehreren der vorhergehenden Ansprüche, dadurch gekennzeichnet, daß die zu alkylierenden Verbindungen des Pyrimidins in einem Lösungsmittel gelöst werden, die

stöchiometrische Menge von n-Alkyl-magnesium-halogeniden in einem geeigneten Lösungsmittel unter stetem Rühren zugetropft wird, auf 40 - 80°C erwärmt wird und weiter gerührt wird für 12 Stunden, und die Reaktion durch Zugabe von 50 gew.%-iger Bromwassersäure beendet wird.

87. Verfahren zur Herstellung nach einem oder mehreren der vorhergehenden Ansprüche, dadurch gekennzeichnet, daß das Lösungsmittel 1,2-Dimethoxy-ethan und/oder n-Heptan ist.

88. Verfahren zur Herstellung nach einem oder mehreren der vorhergehenden Ansprüche, dadurch gekennzeichnet, daß die zu alkylierenden Verbindungen der 7-n-Alkyl-imidazolinium [4,5-d] pyrimidine in einem geeigneten Lösungsmittel gelöst werden, unter gleichzeitiger Zugabe von Triethyloxonium-borfluorid ($Et_3OBF_4$) und das entsprechende n-Alkyl-halogenid, das ebenfalls in einem geeigneten Lösungsmittel gelöst ist, unter stetem Rühren zugetropft wird, und die Reaktion unter stetem Rühren bei 65 - 80°C nach 16 Stunden beendet wird, wonach das Reaktionsprodukt nach dem Abkühlen ausfällt.

89. Verfahren zur Herstellung nach einem oder mehreren der vorhergehenden Ansprüche, dadurch gekennzeichnet, daß die Lösungsmittel gemäß Anspruch 88 Azeton oder Di-iso-butyl-keton oder iso-Butyl-ethyl-keton sind.

90. Verfahren zur Herstellung nach einem oder mehreren der vorhergehenden Ansprüche, dadurch gekennzeichnet, daß entweder das Reaktionsgefäß ein Reaktionskolben mit Rührwerk, Zutropfvorrichtung und Heizungseinrichtung ist oder das Reaktionsgefäß ein Edelstahl-Autoklav mit Druck-und Temperaturanzeige ist.

91. Verwendung der stickstoffhaltigen Heterozyklen nach einem oder mehreren der vorhergehenden Ansprüche zur Herstellung von micellaren oder vesikulären Strukturen in polaren oder unpolaren Lösungsmitteln, insbesondere mit extrem niedriger KMK.

92. Verwendung der stickstoffhaltigen Heterozyklen nach einem oder mehreren der vorhergehenden Ansprüche zur Aufnahme von hydrophoben pharmazeutischen Wirkstoffen bei pH 6 - 8 in polaren Lösungsmitteln oder unpolaren Lösungsmitteln.

93. Verwendung der stickstoffhaltigen Heterozyklen nach einem oder mehreren der vorhergehenden Ansprüche als eigenständige pharmazeutische Wirkstoffe bei pH 6 - 8 in polaren Lösungsmitteln.

94. Verwendung der stickstoffhaltigen Heterozyklen nach einem oder mehreren der vorhergehenden Ansprüche als spektroskopische Marker (Reporter-Gruppen). in immunologischen und klinisch-biochemischen Analyseverfahren sowie zu kolloid-chemischen Meßverfahren zur Bestimmung der KMK.

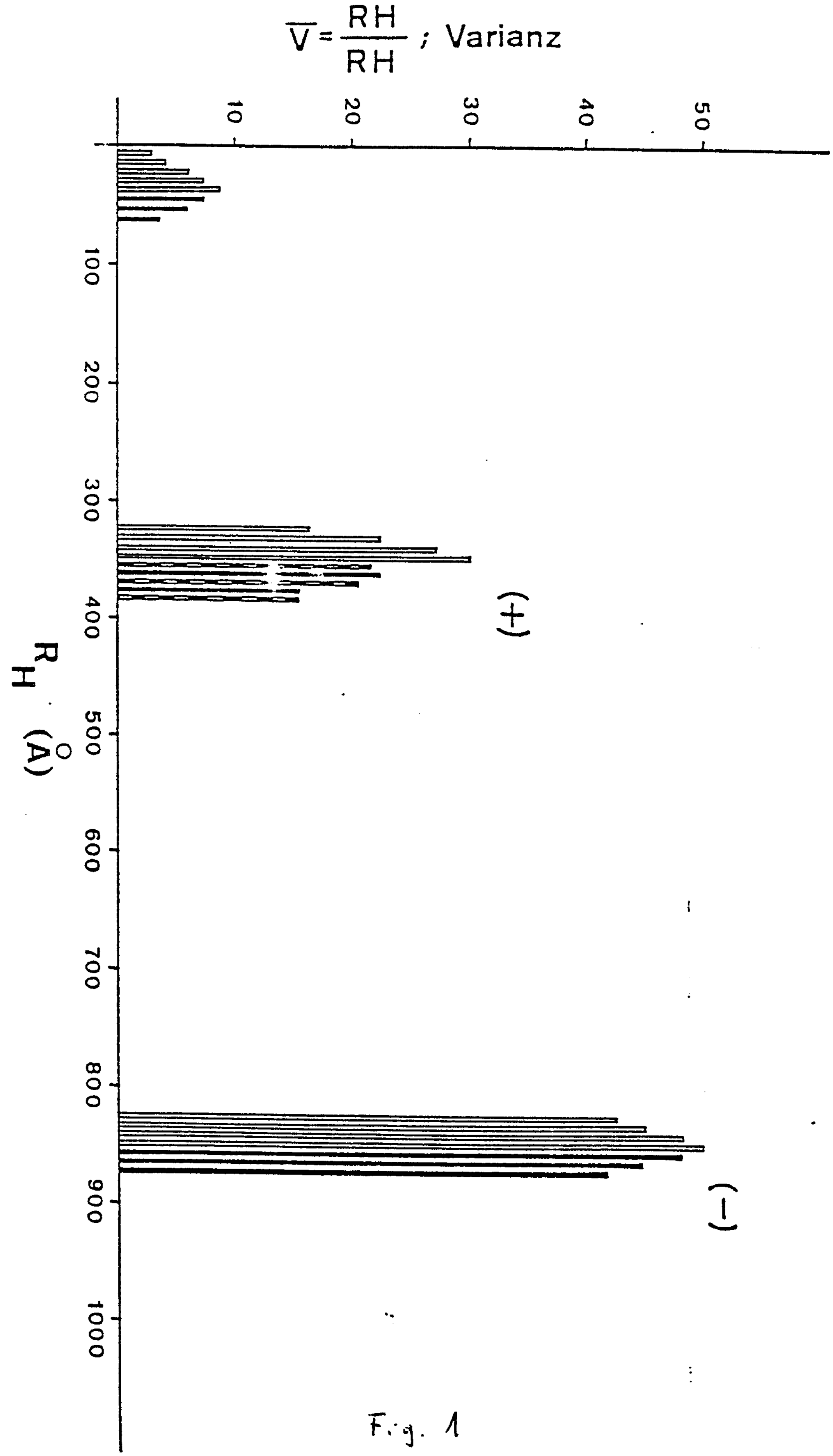
$\overline{V} = \frac{RH}{RH}$ ; Varianz
10
20
30
40
50
100
200
300
400
500
600
700
800
900
1000
(+)
(−)
$R_H$ (Å)

Fig. 1

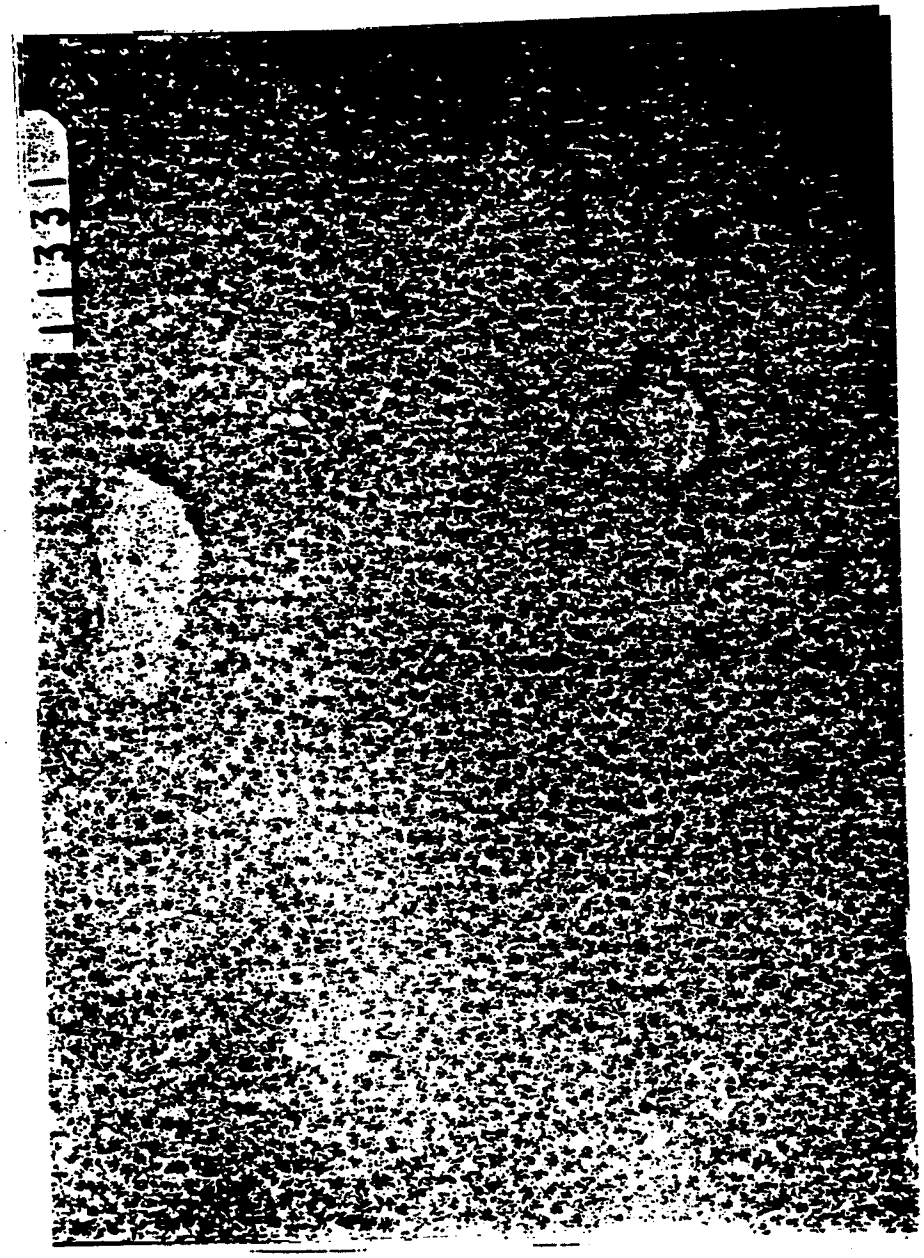

Abb. 2

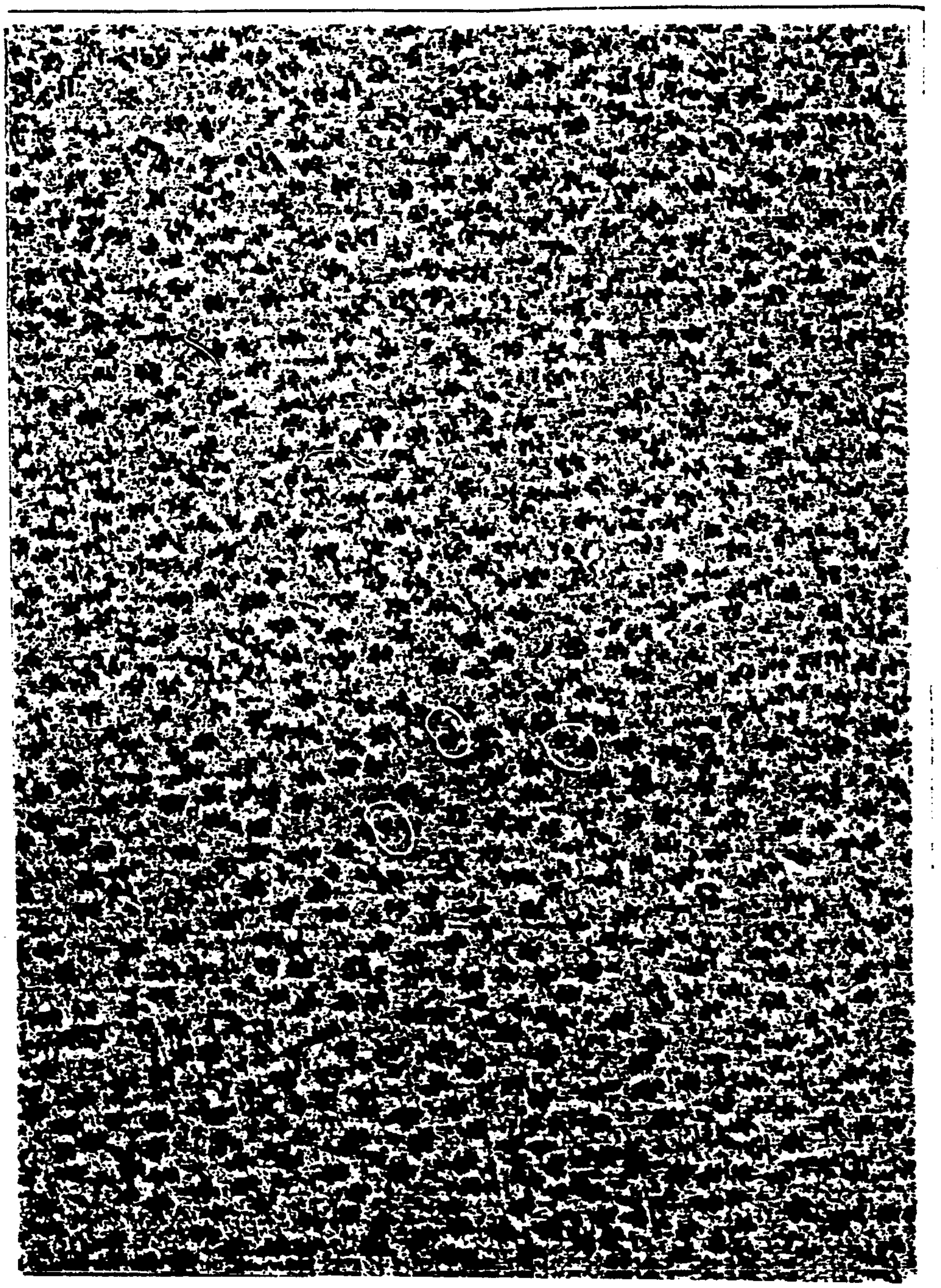

Abb. 3

Abb. 4

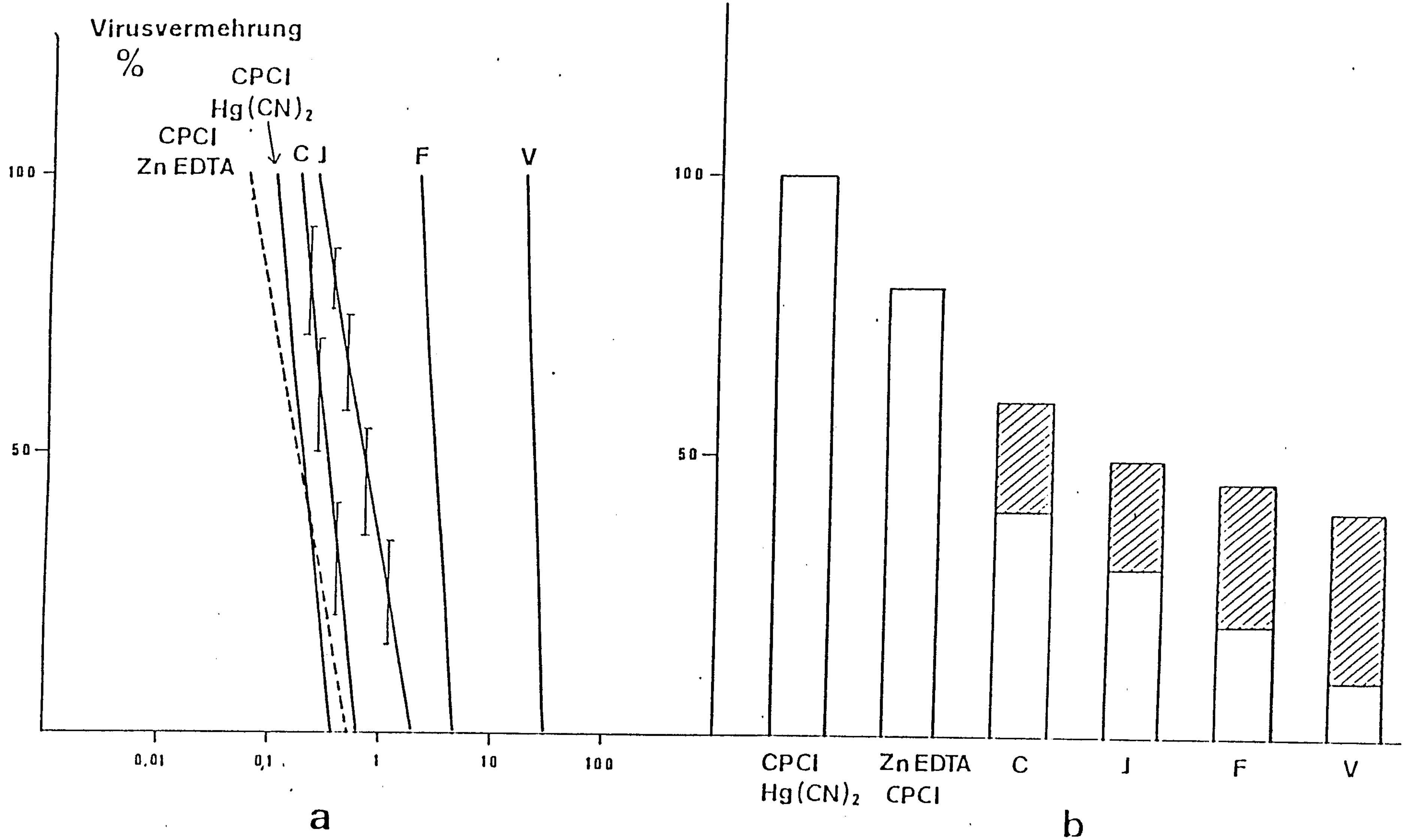

Virusvermehrung
%
CPCl
Hg(CN)₂
CPCl
Zn EDTA
C J
F
V
100
50
0,01
0,1
1
10
100
a
CPCl Hg(CN)₂
Zn EDTA CPCl
C
J
F
V
b

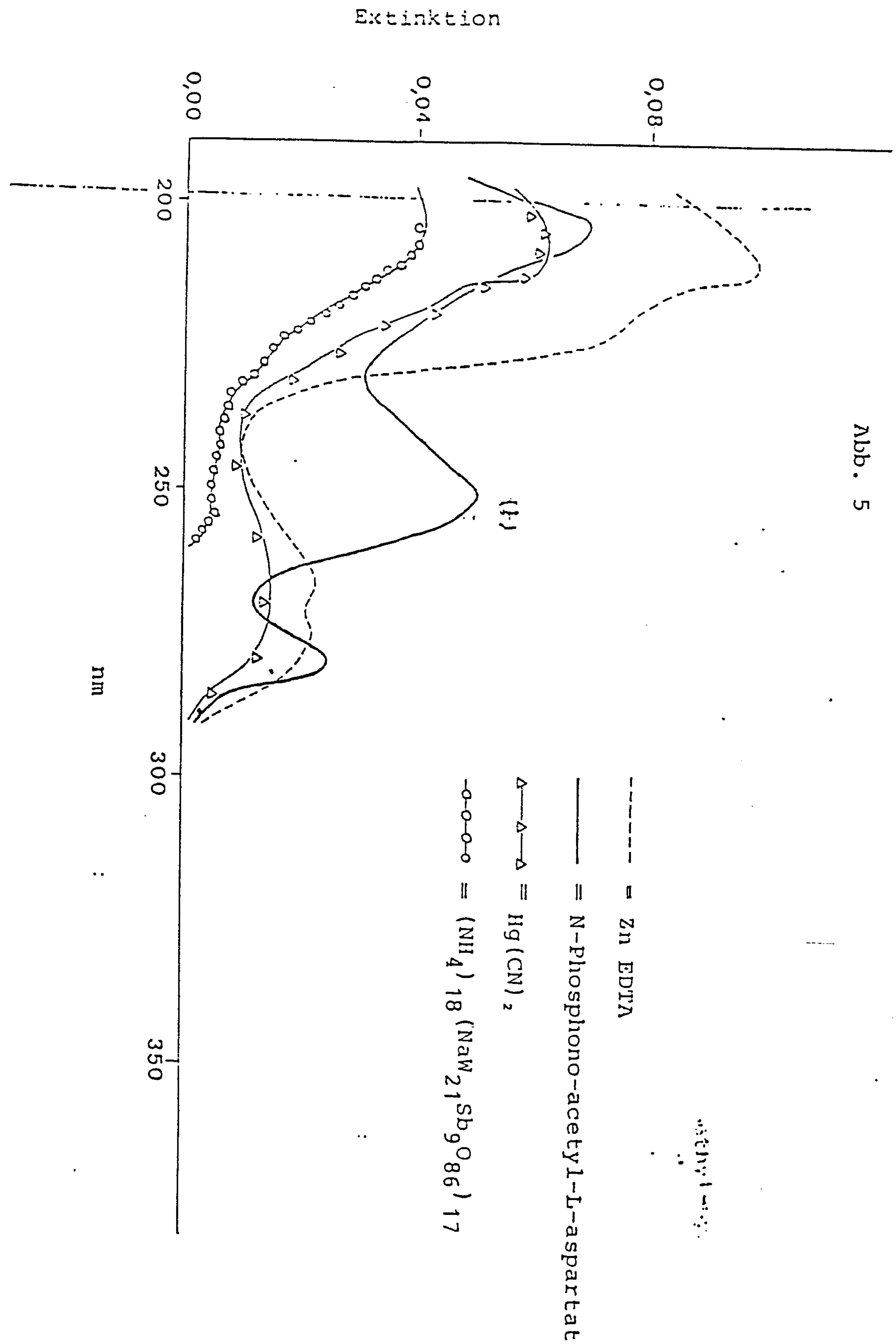
Extinktion
0,00
0,04
0,08
200
250
300
350
nm
Abb. 5
= Zn EDTA
= N-Phosphono-acetyl-L-aspartat
= Hg(CN)₂
= (NH$_4$)$_{18}$(NaW$_{21}$Sb$_9$O$_{86}$)$_{17}$

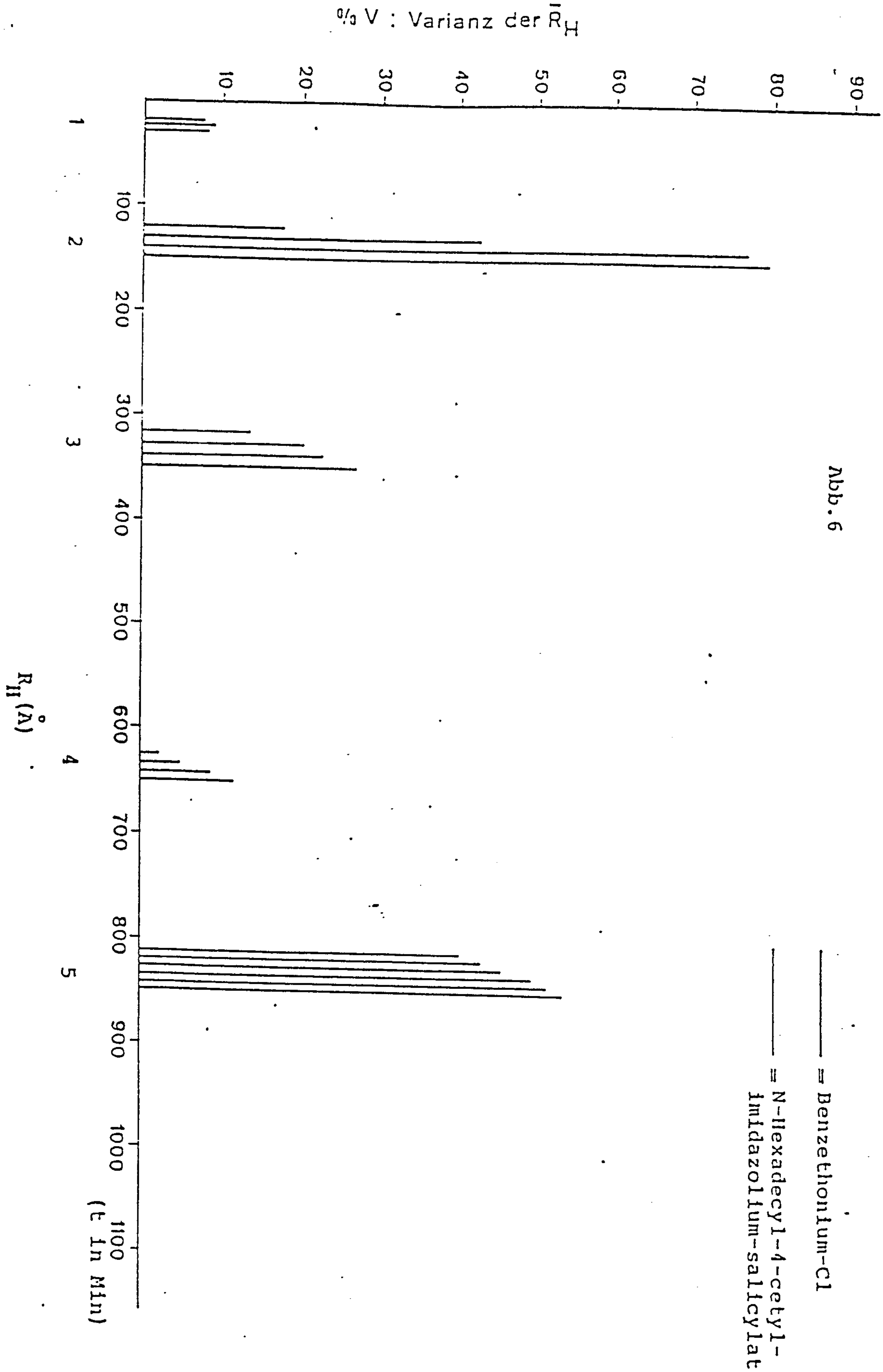
% V : Varianz der $\bar{R}_H$
10
20
30
40
50
60
70
80
90
1
2
3
4
5
100
200
300
400
500
600
700
800
900
1000
1100
(t in Min)
$R_H$ (Å)
Abb. 6
= Benzethonium-Cl
= N-Hexadecyl-4-cetyl-imidazolium-salicylat

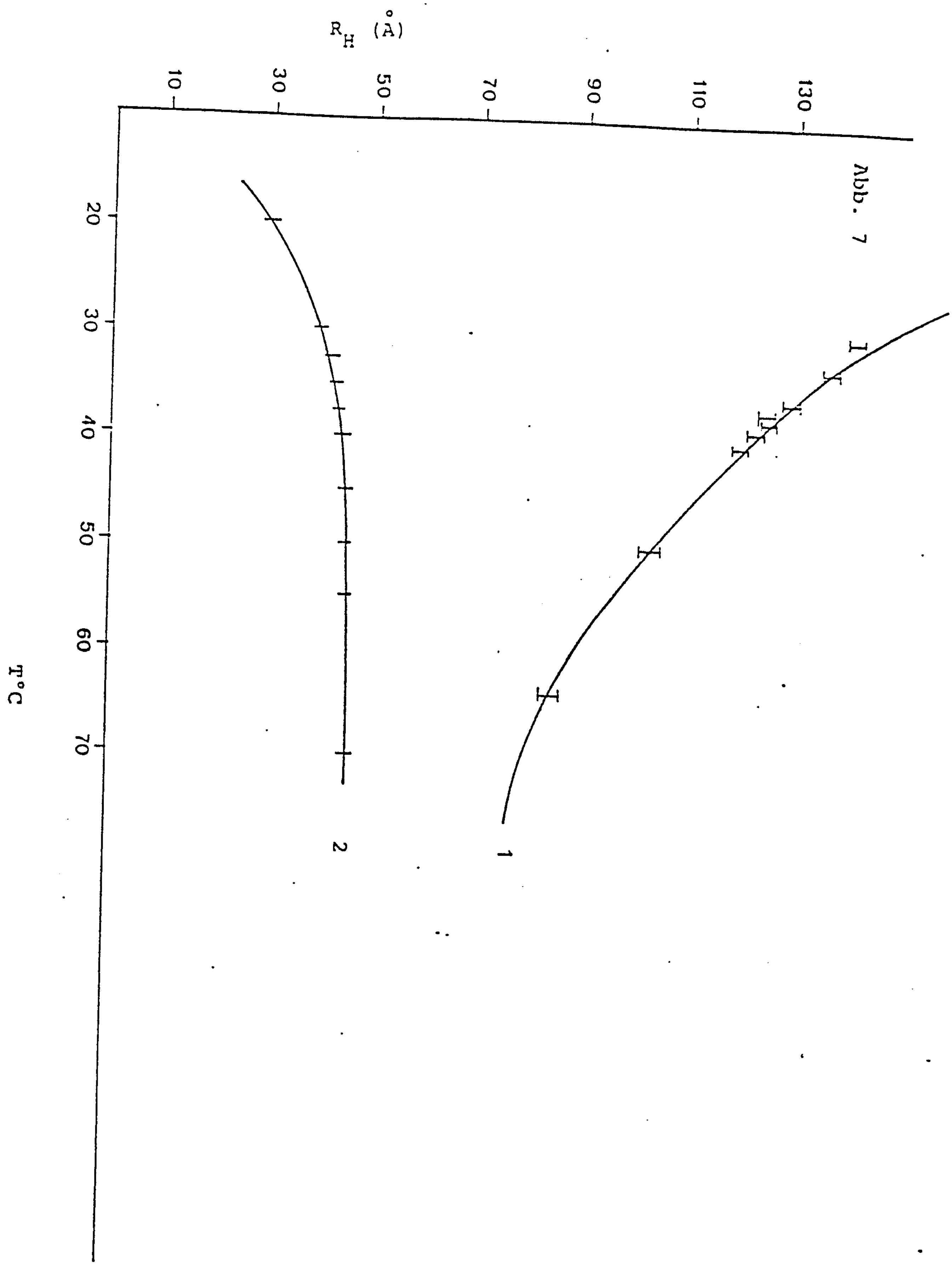


Abb. 7

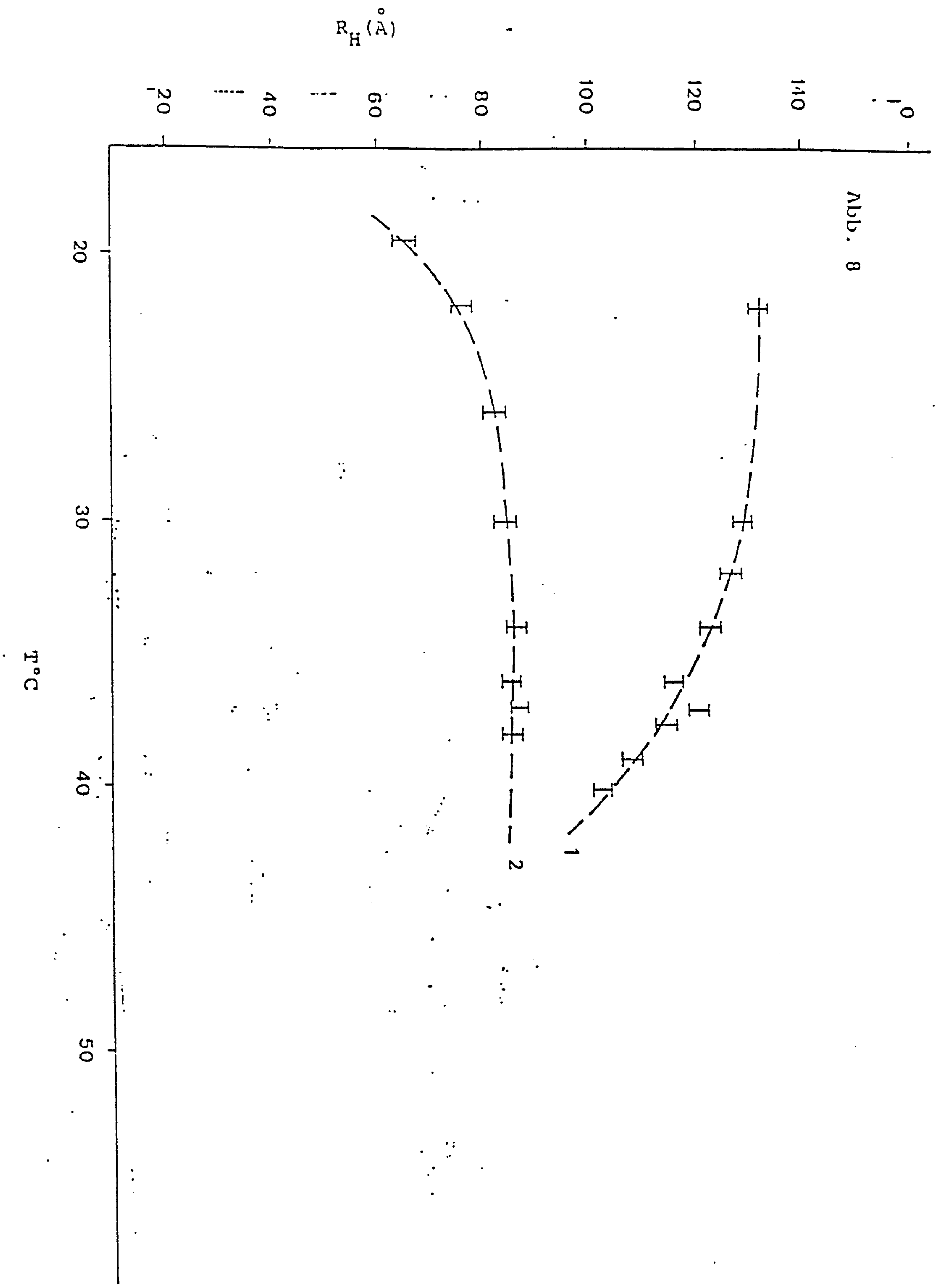


Abb. 8

Abb. 9

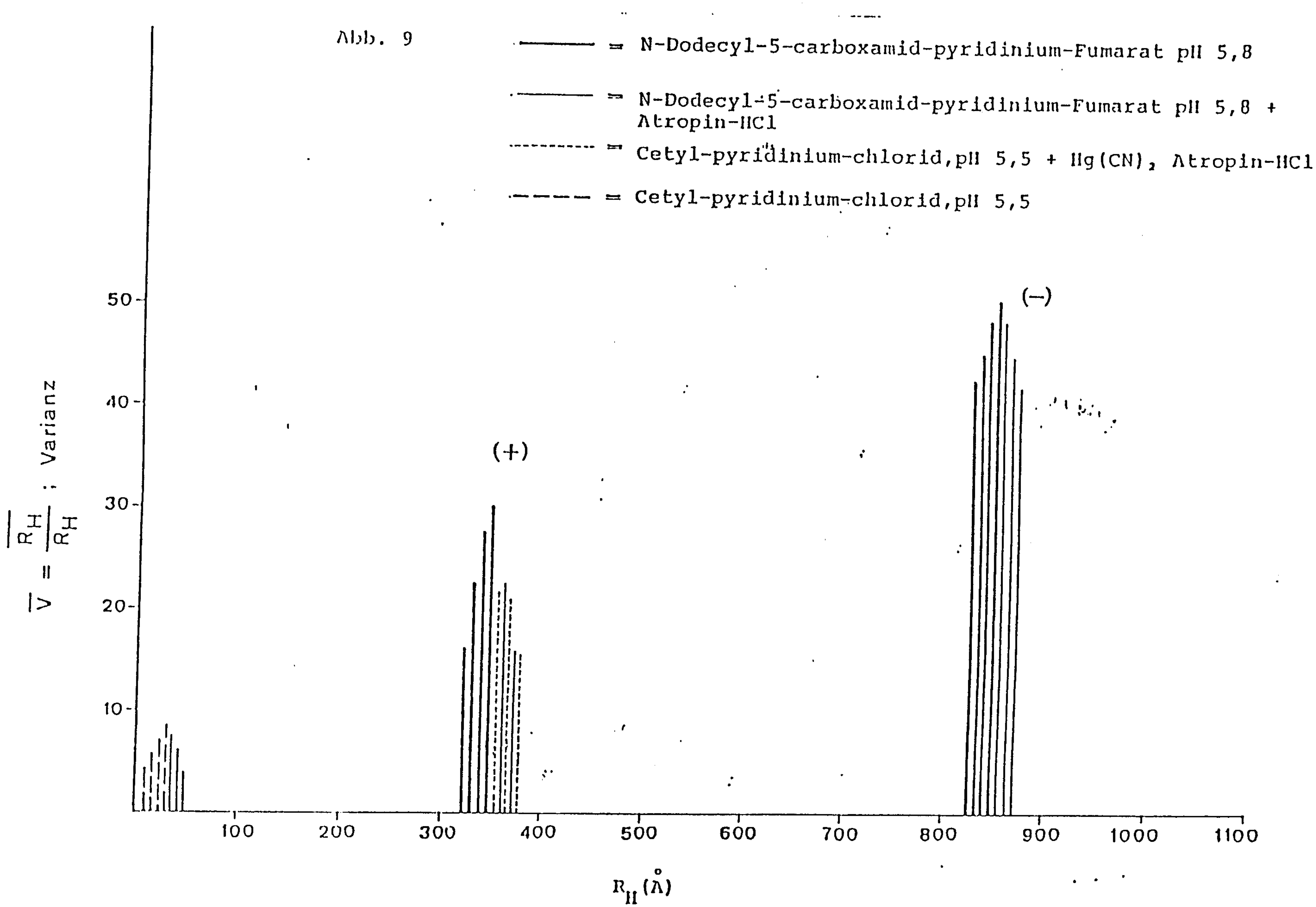

—— = N-Dodecyl-5-carboxamid-pyridinium-Fumarat pH 5,8
—— = N-Dodecyl-5-carboxamid-pyridinium-Fumarat pH 5,8 + Atropin-HCl
------ = Cetyl-pyridinium-chlorid, pH 5,5 + Hg(CN)₂ Atropin-HCl
– – – = Cetyl-pyridinium-chlorid, pH 5,5
(+)
(−)
V = R_H / R_H ; Varianz
10
20
30
40
50
100
200
300
400
500
600
700
800
900
1000
1100
R_H (Å)

Abb. 10

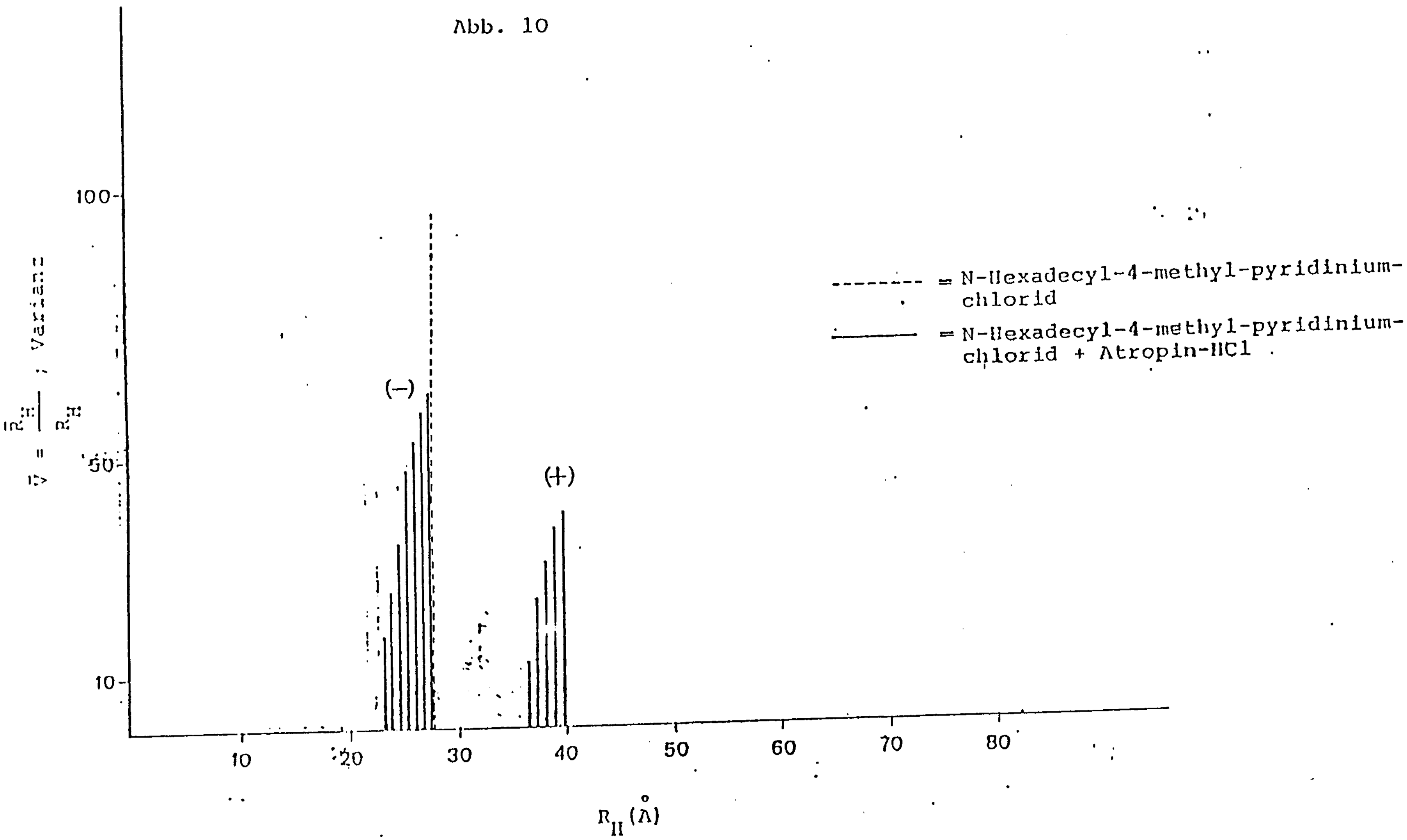


BAD ORIGINAL

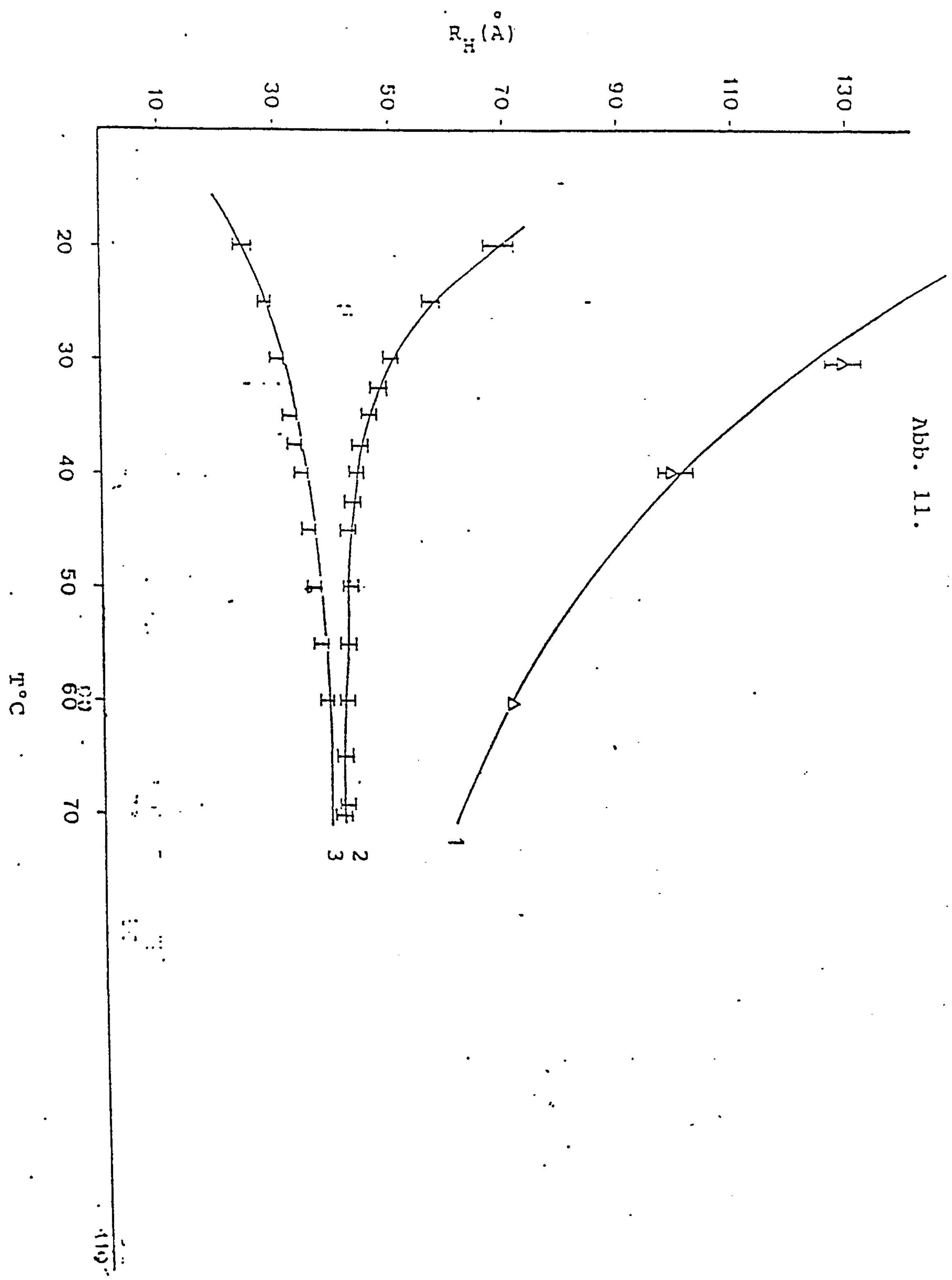


Abb. 11.

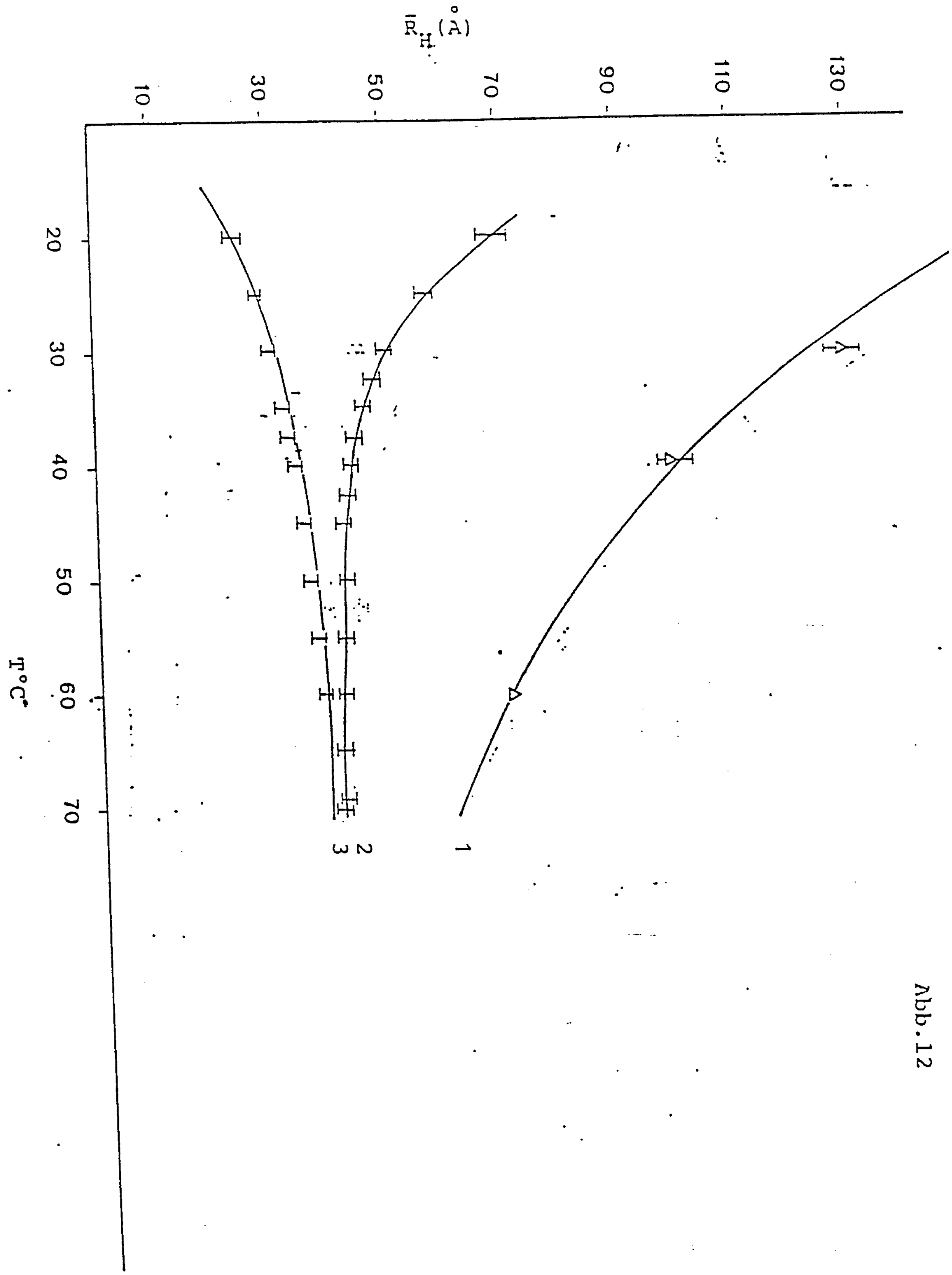


Abb.12

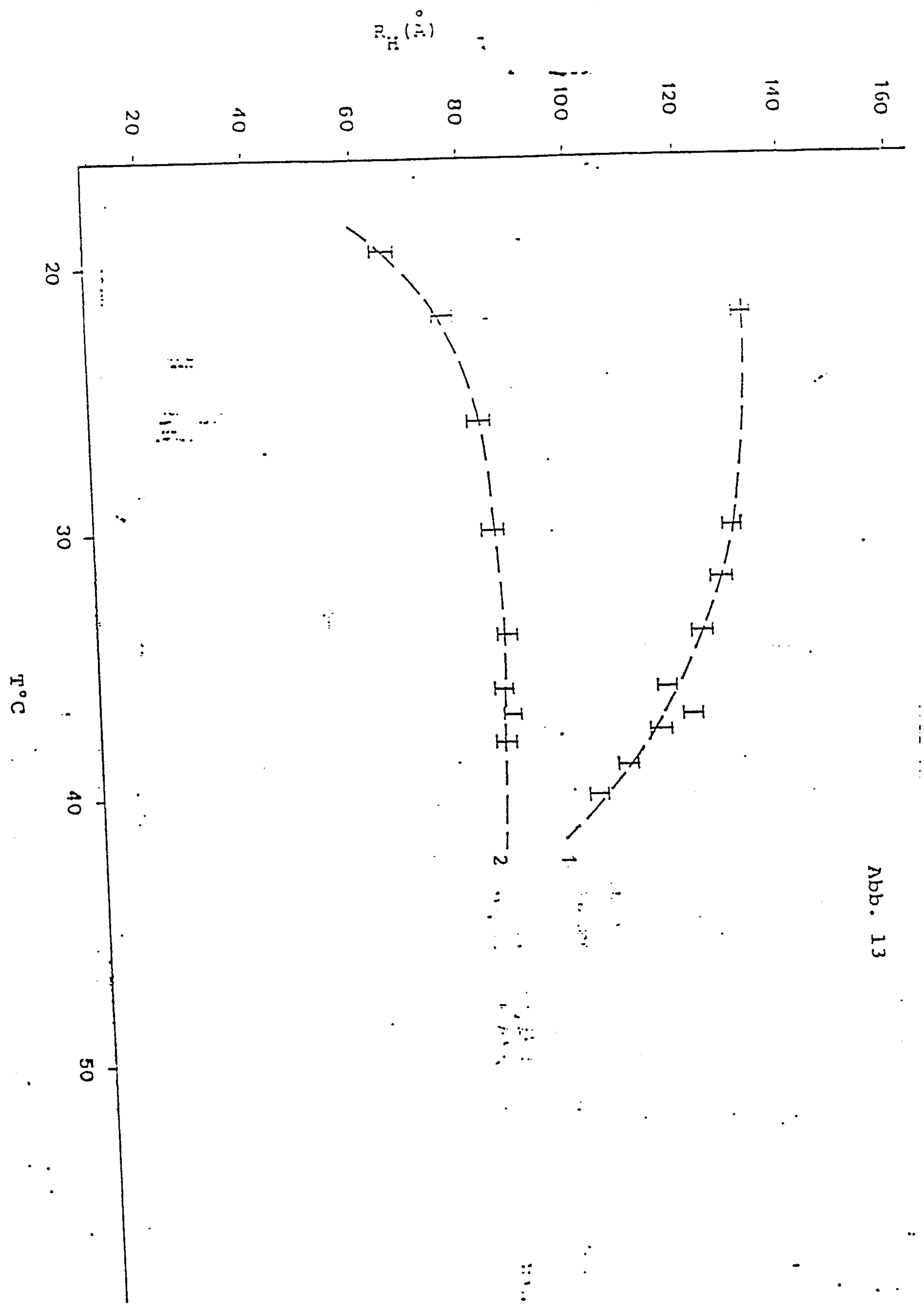


Abb. 13

$$\overline{V} = \frac{\overline{R_H}}{R_H} \cdot 100 \text{ ; Varianz}$$

Abb. 14

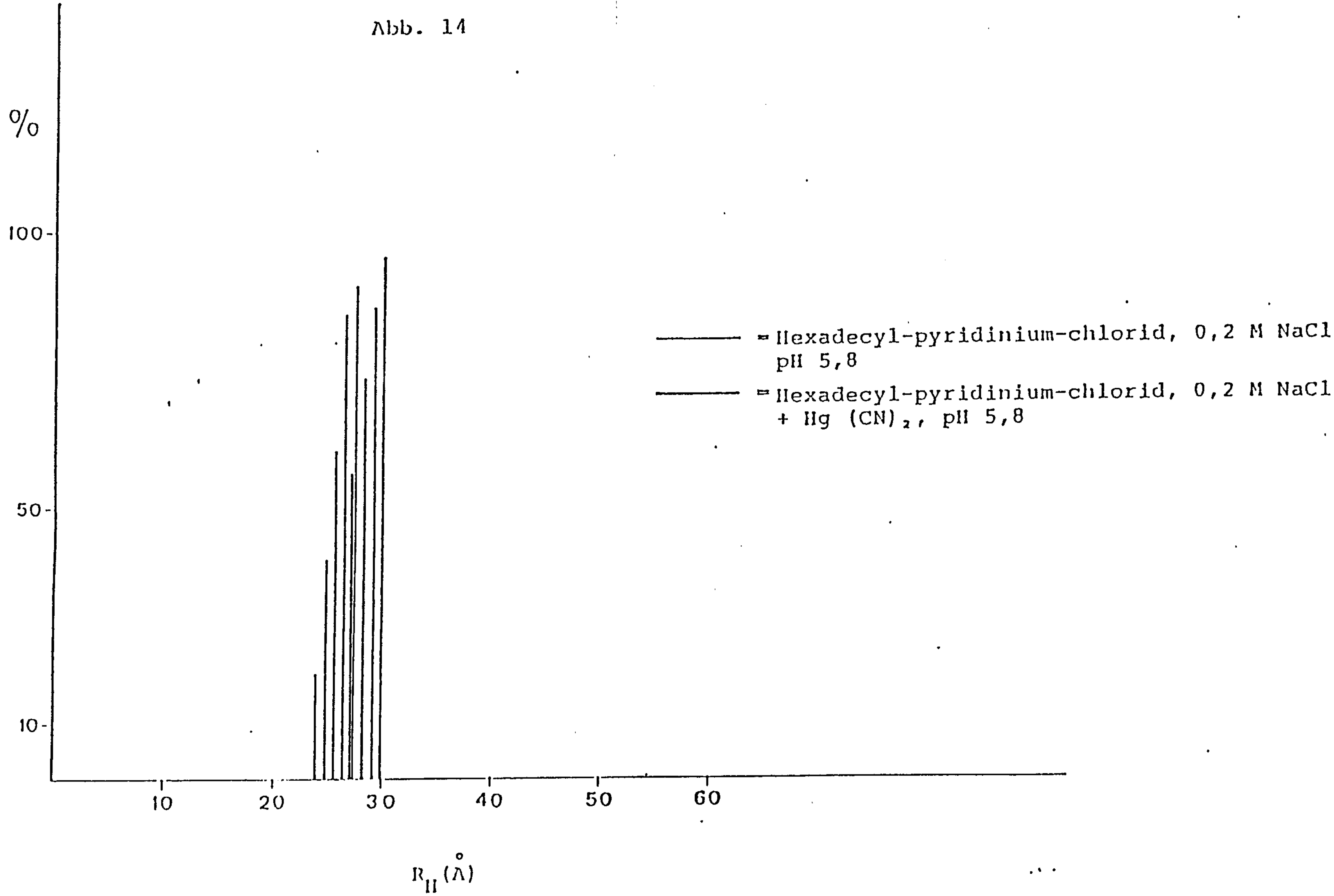

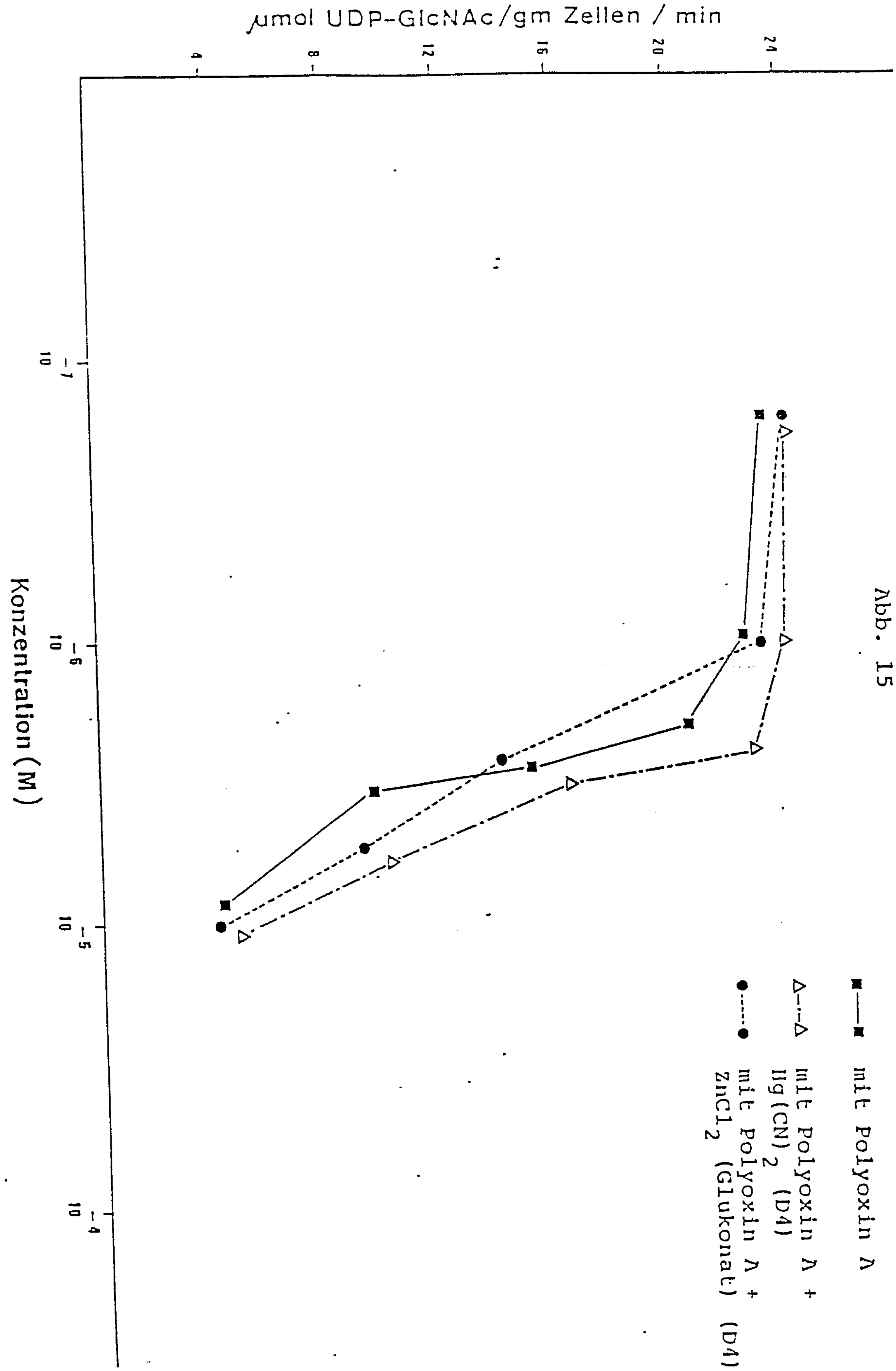
µmol UDP-GlcNAc/gm Zellen / min
4
8
12
16
20
24
$10^{-7}$
$10^{-6}$
$10^{-5}$
$10^{-4}$
Konzentration (M)
mit Polyoxin A
mit Polyoxin A + Hg(CN)$_2$ (D4)
mit Polyoxin A + ZnCl$_2$ (Glukonat) (D4)

Abb. 15

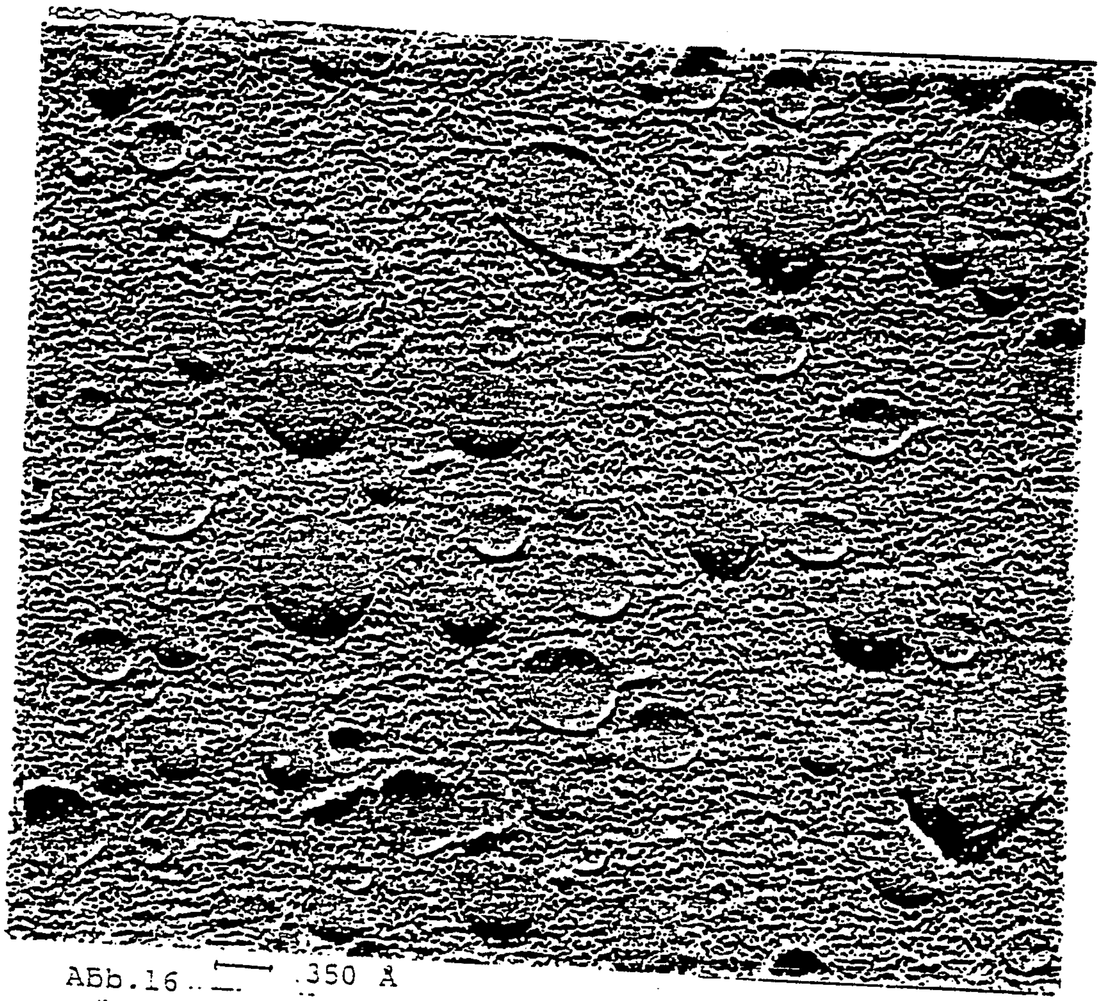

ABb.16 ⟷ 350 Å

"Freeze fracture" elektronenmikroskopische Aufnahme von Vesikeln des $N^+$-Tensides 3,5-bis (n-hexadecyloxy)carbonyl - N-methyl-pyridiniumchlorid einschließlich der eingeschlossenen Polyoxin A gemäß Arbeitsvorschrift.
Es zeigt die verschiedenen Größenordnungen der Vesikel, wenn nicht nach Größenordnung getrennt wird.

BAD ORIGINAL